# EUROPÄISCHE PATENTSCHRIFT

(11) **EP 2 054 412 B1**
(45) Veröffentlichungstag und Bekanntmachung des Hinweises auf die Patenterteilung: **02.12.2009**
(21) Anmeldenummer: 07765222.0
(22) Anmeldetag: 18.07.2007
(51) Int. Cl.: C07D 471/04

(54) **IMINO-IMIDAZO-PYRIDINDERIVATE MIT ANTITHROMBOTISCHER AKTIVITÄT**
IMINO-IMIDAZO-PYRIDINE DERIVATIVES HAVING ANTITHROMBOTIC ACTIVITY
DÉRIVÉ DE IMINO-IMIDAZO-PYRIDINE PRÉSENTANT UNE ACTIVITÉ ANTITHROMBOTIQUE

(30) Priorität: 02.08.2006 DE 102006036023
(43) Veröffentlichungstag der Anmeldung: 06.05.2009
(73) Patentinhaber: Sanofi-Aventis, 75013 Paris (FR)
(72) Erfinder: HEINELT, Uwe, 65926 Frankfurt am Main (DE); HOFMEISTER, Armin, 65926 Frankfurt am Main (DE); CZECH, Joerg, 65926 Frankfurt am Main (DE)
(74) Vertreter: Then, Johann
(86) Internationale Anmeldenummer: PCT/EP2007/006360
(87) Internationale Veröffentlichungsnummer: WO 2008/014888

(56) Entgegenhaltungen:
- EP-A- 1 391 451
- EP-A- 1 391 456

## Beschreibung

Die Erfindung betrifft neue Verbindungen der Formel I mit antithrombotischer Aktivität, die insbesondere den Protease-aktivierten Rezeptor 1 (PAR1) inhibieren, Verfahren zu ihrer Herstellung und Verwendung derselben als Arzneimittel.

Der Protease-aktivierte Rezeptor 1 (PAR1) ist ein Thrombin Rezeptor, der zur Klasse der G Protein-gekoppelten Rezeptoren (GPCR) gehört. Das Gen für PAR1 liegt auf Chromosom 5q13, besteht aus zwei Exonen und deckt eine Region von etwa 27 kb ab. PAR1 wird unter anderem in Endothelzellen, glatten Muskelzellen, Fibroblasten, Neuronen und humanen Blutplättchen exprimiert. In Blutplättchen ist PAR1 ein wichtiger Rezeptor der Signalübertragung welcher an der Initiation der Aggregation von Blutplättchen beteiligt ist. Die Aktivierung von PARs erfolgt über die proteolytische Abspaltung eines Teils des N-Terminus der PARs, wodurch eine neue N-terminale Sequenz freigelegt wird, die dann den Rezeptor aktiviert (Pharmacol Rev 54:203-217, 2002).

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der vaskulären Konstriktion: Hierdurch wird der Blutverlust in das geschädigte Areal vermindert.
2. Die nächste Phase ist die der Plättchenaktivierung durch Thrombin. Die Plättchen aggregieren an der Stelle des Gefäßwandschadens und bilden ein noch lockeres Plättchengerinnsel. Das Protein Fibrinogen ist hauptsächlich für die Stimulierung der Plättchenaggregation verantwortlich. Plättchen binden auch an freigelegtes Kollagen der geschädigten Gefäßwand.
3. Das anfänglich noch lockere Plättchenaggregat wird durch Fibrin vernetzt. Wenn der Thrombus lediglich Plättchen und Fibrin enthält, handelt es sich um einen weißen Thrombus: Sind zusätzlich rote Blutkörperchen vorhanden, handelt es sich um einen roten Thrombus.
4. Nach Wundheilung wird der Thrombus durch die Einwirkung des Proteins Plasmin aufgelöst.

Zwei alternative Wege führen zur Bildung eines Fibringerinnsels, der intrinsische und der extrinsische Weg. Diese Wege werden durch unterschiedliche Mechanismen eingeleitet, in späterer Phase konvergieren sie jedoch zu einer gemeinsamen Wegstrecke der Gerinnungskaskade. Die Bildung eines roten Thrombus oder eines Gerinnsels auf dem Boden einer Gefäßwandabnormität ohne Wunde ist das Resultat des intrinsischen Weges. Die Fibringerinnselbildung als Antwort auf einen Gewebsschaden oder eine Verletzung ist das Resultat des extrensischen Weges. Beide Wege sind beinhalten eine größere Anzahl von Proteinen, die als Gerinnungsfaktoren bekannt sind.

Der intrinsische Weg erfordert die Gerinnungsfaktoren VIII, IX, X, XI und XII sowie Präkallekrein, hochmolekulares Kininogen, Calciumionen und Phospholipide aus Plättchen. Jedes dieser Proteine führt zur Aktivierung des Faktors X. Der intrinsische Weg wird eingeleitet, wenn Präkallekrein, hochmolekulares Kininogen Faktor XI und XII an eine negativ geladene Oberfläche binden. Dieser Moment wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallekrein in Kallekrein, das wiederum den Faktor XII aktiviert. Faktor XIIa hydrolysiert weiteres Präkallekrein zu Kallekrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwsenheit von Ca²⁺-Ionen aktiviert der Faktor XIa den Faktor IX. Faktor IX ist ein Proenzym, das Vitamin-K abhängige, c-Karboxiglutamat (GLA)-Reste enthält. Die Serinproteaseaktivität kommt nach Bindung von Ca²⁺-Ionen an diese GLA-Reste zum Tragen. Mehrere der Serinproteasen der Blutgerinnungskaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Kinasekomplexes aus von Ca²⁺-Ionen und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Kinasekomplexes möglich. Faktor VIII hat in diesem Vorgang die Funktion eines Rezeptors für die Faktoren IXa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor VIIIa schließlich durch Thrombin weiter gespalten und inaktiviert. Diese duale Aktivität des Thrombins in Bezug zum Faktor VIII führt zu einer Selbstbegrenzung der Kinasekomplexbildung und damit zu einer Eingrenzung der Blutgerinnung.

Bei der Aktivierung von humanen Blutplättchen durch Thrombin spielen PAR1 und PAR4 eine zentrale Rolle; die Aktivierung dieser Rezeptoren führt in Blutplättchen zu morphologischen Veränderungen, Freisetzung von ADP und Aggregation der Blutplättchen (Nature 413:26-27, 2001).

Inhibitoren von PAR 1 werden beispielsweise in den Europäischen Patentanmeldungen EP1391451 oder EP1391452, den amerikanischen Patentanmeldungen US 6,063,847 und US 2004/0152736 sowie der Internationalen Anmeldung WO 03/089428 beschrieben.

Die erfindungsgemäßen Verbindungen der Formel I eignen sich für die prophylaktische als auch für die therapeutische Anwendung am Menschen, die an Erkrankungen leiden, die mit Thrombosen, Embolien, Hyperkoagulabilität oder fibrotischen Veränderungen einhergehen. Beispiele solcher Erkrankungen sind Thrombose, tiefe Venenthrombose, Lungenembolien, Gehirninfarkt, Herzinfarkt, Bluthochdruck, Entzündliche Erkrankungen, Rheuma, Asthma, Glomerulonephritis oder Osteoporose. Die erfindungsgemäßen Verbindungen der Formel I können zur Sekundär-Prävention eingesetzt werden und eignen sich sowohl für eine akute als auch für eine Langzeittherapie.

Die Erfindung betrifft daher eine Verbindung der Formel I 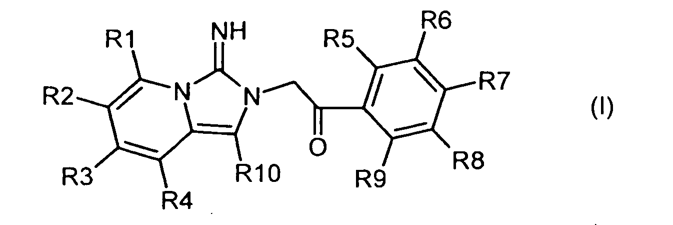 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1, R2, R3 und R4 gleich oder verschieden sind und unabhängig voneinander für
1) -(C₁-C₆)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist,
2) -O-(C₁-C₈)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist, wobei -(C₆-C₁₄)-Aryl und Het unsubstituiert oder zusätzlich ein-, zwei- oder dreifach durch R15 substituiert ist,
3) -(C₀-C₄)-Alkylen-C(O)-R11, wobei R11 für
   3)1) Wasserstoffatom,
   3)2) -(C₁-C₆)-Alkyl,
   3)3) -(C₀-C₄)-Alkylen-(C₆-C₁₄)-Aryl,
   3)4) -(C₀-C₄)-Alkylen-Het oder
   3)5) -(C₀-C₄)-Alkylen-(C₃-C₆)-Cycloalkyl, steht,
4) -(C₀-C₄)-Alkylen-C(O)-O-R11, wobei R11 wie oben definiert ist,
5) -(C₀-C₄)-Alkylen-N(R12)-R13, wobei R12 und R13 gleich oder verschieden sind und unabhängig voneinander für
   5)1) Wasserstoffatom,
   5)2) -(C₁-C₆)-Alkyl,
   5)3) -(C₁-C₃)-Fluoralkyl,
   5)4) -(C₀-C₄)-Alkylen-(C₆-C₁₄)-Aryl,
   5)5) -(C₀-C₄)-Alkylen-Het oder
   5)6) -(C₀-C₄)-Alkylen-(C₃-C₆)-Cycloalkyl, stehen,
6) -(C₀-C₄)-Alkylen-C(O)-N(R12)-R13, wobei R12 und R13 gleich oder verschieden sind und unabhängig voneinander wie oben definiert sind,
7) -(C₀-C₄)-Alkylen-N(R12)-C(O)-R13, wobei R12 und R13 gleich oder verschieden sind und unabhängig voneinander wie oben definiert sind,
8) -(C₁-C₃)-Fluoralkyl,
9) -O-(C₁-C₃)-Fluoralkyl,
10) -SO₂-CH₃ ,
11) -SO₂-CF₃ ,
12) -NO₂,
13) -CN,
14) -OH,
15) =O,
16) Wasserstoffatom oder
17) Halogen, stehen
R10 und R15 gleich oder verschieden sind und unabhängig voneinander für
1) Wasserstoffatom,
2) -(C₁-C₄)-Alkyl,
3) -O-(C₁-C₄)-Alkyl,
4) -(C₁-C₃)-Fluoralkyl,
5) -O-(C₁-C₃)-Fluoralkyl,
6) -(C₀-C₄)-Alkylen-N(R16) (R17), worin R16 und R17 unabhängig voneinander Wasserstoffatom oder -(C₁-C₆)-Alkyl bedeuten,
7) -(C₀-C₄)-Alkylen-(C₆-C₁₄)-Aryl,
8) -(C₀-C₄)-Alkylen-(C₃-C₆)-Cycloalkyl,
9) -(C₀-C₄)-Alkylen-Het,
10) -OH,
11) =O,
12) -NO₂,
13) -CN,
14) Halogen,
15) -SO₂-(C₁-C₄)-Alkyl oder
16) -SO₂-(C₁-C₃)-Fluoralkyl, stehen,
R5, R6, R7, R8 und R9 gleich oder verschieden sind und unabhängig voneinander für
1) Wasserstoffatom,
2) -(C₀-C₄)-Alkylen-(C₆-C₁₄)-Aryl, wobei Aryl unsubstituiert oder ein-, zwei-oder dreifach unabhängig voneinander durch -(C₁-C₄)-Alkyl,
   -(C₆-C₁₄)-Aryl, Het, -(C₃-C₆)-Cycloalkyl, Halogen, -NH₂, -OH oder Methoxy substituiert ist,
3) -(C₀-C₄)-Alkylen-(C₃-C₆)-Cycloalkyl,
4) -(C₀-C₄)-Alkylen-Het, wobei Het unsubstituiert oder ein-, zwei- oder dreifach unabhängig voneinander durch -(C₁-C₄)-Alkyl, -(C₆-C₁₄)-Aryl, Het, -(C₃-C₆)-Cycloalkyl, -C(O)-O-R16, -C(O)-N(R16)(R17), worin R16 und R17 wie oben definiert sind, Halogen, -NH₂, -OH oder Methoxy substituiert ist,
5) -SF₅,
6) -(C₁-C₆)-Alkyl, wobei Alkyl unsubstituiert oder ein-, zwei- oder dreifach unabhängig voneinander durch Halogen, -NH₂, -OH oder Methoxy substituiert ist,
7) -O-(C₁-C₈)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist,
8) -(C₀-C₄)-Alkylen-C(O)-R11, wobei R11 wie oben definiert ist,
9) -(C₀-C₄)-Alkylen-C(O)-O-R11, wobei R11 wie oben definiert ist,
10) -(C₀-C₄)-Alkylen-N(R12)-R13, wobei R12 und R13 gleich oder verschieden sind und unabhängig voneinander wie oben definiert sind,
11) -(C₀-C₄)-Alkylen-C(O)-N(R12)-R13, wobei R12 und R13 gleich oder verschieden sind und unabhängig voneinander wie oben definiert sind,
12) -(C₀-C₄)-Alkylen-N(R12)-C(O)-R13, wobei R12 und R13 gleich oder verschieden sind und unabhängig voneinander wie oben definiert sind,
13) -(C₁-C₃)-Fluoralkyl,
14) -O-(C₁-C₃)-Fluoralkyl,
15) -SO₂-CH₃,
16) -SO₂-CF₃,
17) -NO₂,
18) -CN,
19) -OH oder
20) Halogen, stehen oder
R5 und R6, R6 und R7, R7 und R8 oder R8 und R9 bilden zusammen mit den Ringatomen, an die sie gebunden sind, einen vier- bis achtgliedrigen Heterocyclus, der zusammen mit dem Phenylring, an den der Heterocyclus anneliert ist, ein bicyclisches System bildet, wobei der heterocyclische Teil unsubstituiert oder ein-, zwei- oder dreifach unabhängig voneinander durch - (C₁-C₄)-Alkyl, -(C₆-C₁₄)-Aryl, -(C₃-C₆)-Cycloalkyl, Halogen, -NH₂, -OH oder Methoxy substituiert ist.

2) Die Erfindung betrifft ferner eine Verbindung der Formel I und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1, R2, R3 und R4 gleich oder verschieden sind und unabhängig voneinander für
1) -(C₁-C₆)-Alkyl, wobei Alkyl unsubstituiert oder ein-, zwei- oder dreifach unabhängig voneinander durch -(C₃-C₆)-Cycloalkyl, Halogen, -NH₂, -OH, Methoxy, -(C₆-C₁₄)-Aryl, oder Het, substituiert ist,
   wobei Aryl ausgewählt ist aus der Gruppe Phenyl, Naphthyl, Anthryl und Fluorenyl und worin Aryl unsubstituiert oder ein-, zwei- oder dreifach durch R15 substituiert ist,
   wobei Het ausgewählt ist aus der Gruppe Acridinyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Isothiazolidinyl, 2-lsothiazolinyl, Isothiazolyl, Isoxazolyl, Isoxazolidinyl, 2-lsoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrol, Thienopyridin, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl und Xanthenyl und worin Het unsubstituiert oder zusätzlich ein-, zwei-oder dreifach durch R15 substituiert ist,
2) -O-(C₁-C₆)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist, wobei Het und Aryl wie oben definiert sind,
3) -(C₀-C₄)-Alkylen-C(O)-R11, wobei R11 für
   3)1) Wasserstoffatom,
   3)2) -(C₁-C₆)-Alkyl,
   3)3) -(C₀-C₄)-Alkylen-(C₆-C₁₄)-Aryl, wobei Aryl wie oben definiert ist,
   3)4) -(C₀-C₄)-Alkylen-Het, wobei Het wie oben definiert ist, oder
   3)5) -(C₀-C₄)-Alkylen-(C₃-C₆)-Cycloalkyl, steht,
4) -(C₀-C₄)-Alkylen-C(O)-O-R11, wobei R11 wie oben definiert ist,
5) -(C₀-C₄)-Alkylen-N(R12)-R13, wobei R12 und R13 gleich oder verschieden sind und unabhängig voneinander für
   5)1) Wasserstoffatom,
   5)2) -(C₁-C₆)-Alkyl,
   5)3) -(C₁-C₃)-Fluoralkyl,
   5)4) -(C₀-C₄)-Alkylen-(C₆-C₁₄)-Aryl, wobei Aryl wie oben definiert ist,
   5)5) -(C₀-C₄)-Alkylen-Het, wobei Het wie oben definiert ist, oder
   5)6) -(C₀-C₄)-Alkylen-(C₃-C₆)-Cycloalkyl, stehen,
6) -(C₀-C₄)-Alkylen-C(O)-N(R12)-R13, wobei R12 und R13 gleich oder verschieden sind und unabhängig voneinander wie oben definiert sind,
7) -(C₀-C₄)-Alkylen-N(R12)-C(O)-R13, wobei R12 und R13 gleich oder verschieden sind und unabhängig voneinander wie oben definiert sind,
8) -(C₁-C₃)-Fluoralkyl,
9) -O-(C₁-C₃)-Fluoralkyl,
10) -SO₂-CH₃,
11) -SO₂-CF₃,
12) -NO₂,
13) -CN,
14) -OH,
15) =O,
16) Wasserstoffatom oder
17) Halogen, stehen
R10 und R15 gleich oder verschieden sind und unabhängig voneinander für
1) Wasserstoffatom,
2) -(C₁-C₄)-Alkyl,
3) -O-(C₁-C₄)-Alkyl,
4) -(C₁-C₃)-Fluoralkyl,
5) -O-(C₁-C₃)-Fluoralkyl,
6) -(C₀-C₄)-Alkylen-N(R16) (R17), worin R16 und R17 unabhängig voneinander Wasserstoffatom oder -(C₁-C₆)-Alkyl bedeuten,
7) -(C₀-C₄)-Alkylen-(C₆-C₁₄)-Aryl, wobei Aryl wie oben definiert ist,
8) -(C₀-C₄)-Alkylen-(C₃-C₆)-Cycloalkyl,
9) -(C₀-C₄)-Alkylen-Het, wobei Het wie oben definiert ist,
10) -OH,
11) =O,
12) -NO₂,
13) -CN,
14) Halogen,
15) -SO₂-(C₁-C₄)-Alkyl oder
16) -SO₂-(C₁-C₃)-Fluoralkyl, stehen,
R5, R6, R7, R8 und R9 gleich oder verschieden sind und unabhängig voneinander für
1) Wasserstoffatom,
2) -(C₀-C₄)-Alkylen-(C₆-C₁₄)-Aryl, wobei Aryl unsubstituiert oder ein-, zwei-oder dreifach unabhängig voneinander durch -(C₁-C₄)-Alkyl, -(C₆-C₁₄)-Aryl, Het, -(C₃-C₆)-Cycloalkyl, Halogen, -NH₂, -OH oder Methoxy substituiert ist, wobei Aryl und Het wie oben definiert sind,
3) -(C₀-C₄)-Alkylen-(C₃-C₆)-Cycloalkyl,
4) -(C₀-C₄)-Alkylen-Het, wobei Het unsubstituiert oder ein-, zwei- oder dreifach unabhängig voneinander durch -(C₁-C₄)-Alkyl, -(C₆-C₁₄)-Aryl, Het, -(C₃-C₆)-Cycloalkyl, -C(O)-O-R16, -C(O)-N(R16)(R17), worin R16 und R17 wie oben definiert sind, Halogen, -NH₂, -OH oder Methoxy substituiert ist, wobei Aryl und Het wie oben definiert sind,
5) -SF₅,
6) -(C₁-C₆)-Alkyl, wobei Alkyl unsubstituiert oder ein-, zwei- oder dreifach unabhängig voneinander durch Halogen, -NH₂, -OH oder Methoxy substituiert ist,
7) -O-(C₁-C₈)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist, wobei Aryl und Het wie oben definiert sind,
8) -(C₀-C₄)-Alkylen-C(O)-R11, wobei R11 wie oben definiert ist,
9) -(C₀-C₄)-Alkylen-C(O)-O-R11, wobei R11 wie oben definiert ist,
10) -(C₀-C₄)-Alkylen-N(R12)-R13, wobei R12 und R13 gleich oder verschieden sind und unabhängig voneinander wie oben definiert sind,
11) -(C₀-C₄)-Alkylen-C(O)-N(R12)-R13, wobei R12 und R13 gleich oder verschieden sind und unabhängig voneinander wie oben definiert sind,
12) -(C₀-C₄)-Alkylen-N(R12)-C(O)-R13, wobei R12 und R13 gleich oder verschieden sind und unabhängig voneinander wie oben definiert sind,
13) -(C₁-C₃)-Fluoralkyl,
14) -O-(C₁-C₃)-Fluoralkyl,
15) -SO₂-CH₃,
16) -SO₂-CF₃,
17) -NO₂,
18) -CN,
19) -OH oder
20) Halogen, stehen oder
R5 und R6, R6 und R7, R7 und R8 oder R8 und R9 bilden zusammen mit den Ringatomen, an die sie gebunden sind, einen vier- bis achtgliedrigen Heterocyclus, der zusammen mit dem Phenylring, an den der Heterocyclus anneliert ist, ein bicyclisches System bildet, ausgewählt aus der Gruppe Benzimidazol, Benzisothiazol, Benzisoxazol, Benzo[1,3]dioxol, Benzofuranyl, Benzothiazol, Benzisoxazol, Benzothiofuran, Benzothiophen, Benzo[1,3]oxathiol, Benzoxazol, Benzthiazol, Benztriazolyl, Chinazolin, Chinazolon, Chinolin, 4H-Chinolizin, Chinoxalin, Chroman, Chromen, Cinnolin, 2,3-Dihydro-benzo[1,4]dioxin, 2,3-Dihydro-benzofuranyl, 1,3-Dihydro-isobenzofuran, 3,4-Dihydro-2H-benzo[1,4]oxazin, 2,3-Dihydro-benzooxazol, 2,3-Dihydro-benzothiazol, 1,3-Dihydro-benzo[c]thiophen, 2,3-Dihydro-benzo[b]thiophen, lndazol, Indol, Indolin, lsobenzofuran, Isochinolin, Isochroman, Isoindazol, Isoindol, Isoindolin, 7-Oxa-bicyclo[4.2.0]octa-1,3,5-trien, Phthalazin, 2,3,4,5-Tetrahydro-1H-benzo[b]azepin, 6,7,8,9-Tetrahydro-5-oxa-9-aza-benzocyclohepten, 3,4,5,6-Tetrahydro-2H-benzo[b][1,4]oxazozin, Tetrahydrochinolin, 1,2,3,4-Tetrahydro-chinoxalin oder Tetrahydroisochinolin, wobei der heterocyclische Teil unsubstituiert oder ein-, zwei- oder dreifach unabhängig voneinander durch -(C₁-C₄)-Alkyl, -(C₆-C₁₄)-Aryl, -(C₃-C₆)-Cycloalkyl, Halogen, -NH₂, -OH oder Methoxy substituiert ist.

3) Die Erfindung betrifft ferner eine Verbindung der Formel I und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1, R2, R3 und R4 gleich oder verschieden sind und unabhängig voneinander für
1) Wasserstoffatom,
2) -(C₁-C₄)-Alkyl,
3) -O-(C₁-C₄)-Alkyl,
4) -(C₀-C₄)-Alkylen-C(O)-N(R12)-R13, wobei R12 und R13 gleich und verschieden sind und unabhängig voneinander Wasserstoffatom oder -(C₁-C₄)-Alkyl bedeuten,
5) -(C₁-C₃)-Fluoralkyl,
6) -(C₀-C₄)-Alkylen-C(O)-O-(C₁-C₄)-Alkyl oder
7) =O,
8) Halogen stehen,
R10 für 1) Wasserstoffatom,
2) -(C₁-C₄)-Alkyl,
3) -(C₀-C₄)-Alkylen-(C₃-C₆)-Cycloalkyl oder
4) -(C₀-C₄)-Alkylen-Phenyl, steht,
R5, R6, R7, R8 und R9 gleich oder verschieden sind und unabhängig voneinander für
1) Wasserstoffatom,
2) -(C₁-C₃)-Fluoralkyl,
3) Halogen,
4) -O-(C₁-C₄)-Alkyl,
5) -OH,
6) -(C₁-C₄)-Alkyl,
7) -SF₅,
8) -(C₀-C₄)-Alkylen-NH-C(O)-(C₁-C₃)-Fluoralkyl,
9) -(C₀-C₄)-Alkylen-N(R12)-R13, wobei R12 und R13 gleich und verschieden sind und unabhängig voneinander Wasserstoffatom oder -(C₁-C₄)-Alkyl bedeuten, oder
10) -(C₀-C₄)-Alkylen-Het, wobei Het ausgewählt ist aus der Gruppe Morpholinyl oder Pyrrolidinyl und unsubstituiert oder ein- oder zweifach unabhängig voneinander durch -(C₁-C₄)-Alkyl, =O oder -NH₂ substituiert ist, stehen oder
R5 und R6, R6 und R7, R7 und R8 oder R8 und R9 bilden zusammen mit den Ringatomen, an die sie gebunden sind, einen vier- bis achtgliedrigen Heterocyclus, der zusammen mit dem Phenylring, an den der Heterocyclus anneliert ist, ein bicyclisches System bildet, ausgewählt aus der Gruppe 2,3-Dihydro-benzo[1,4]dioxin, Benzo[1,3]dioxol, 3,4-Dihydro-2H-benzo[1,4]oxazin, 2,3,4,5-Tetrahydro-1H-benzo[b]azepin, Tetrahydrochinolin, Tetrahydroisochinolin, 1,2,3,4-Tetrahydro-chinoxalin oder 6,7,8,9-Tetrahydro-5-oxa-9-aza-benzocyclohepten, wobei der heterocyclische Teil unsubstituiert oder ein- oder zweifach durch -(C₁-C₄)-Alkyl oder Halogen substituiert ist.

Ein weiterer Gegenstand der Erfindung sind Verbindungen der Formel I aus der Reihe 1-(3,5-Di-tert-butyl-4-hydroxy-phenyl)-2-(3-imino-imidazo[1,5-a]pyridin-2-yl)-ethanon als Trifluoressigsäuresalz,
2-(3-Imino-imidazo[1,5-a]pyridin-2-yl)-1-(3-pentafluorsulfanyl-phenyl)-ethanon als Trifluoressigsäuresalz,
2-(1-Cyclopropyl-3-imino-imidazo[1,5-a]pyridin-2-yl)-1-(3,5-di-tert-butyl-4-hydroxyphenyl)-ethanon als Trifluoressigsäuresalz,
1-(3,5-Di-tert-butyl-4-hydroxy-phenyl)-2-(3-imino-1-phenyl-imidazo[1,5-a]pyridin-2-yl)-ethanon als Trifluoressigsäuresalz,
1-(3,5-Di-tert-butyl-4-hydroxy-phenyl)-2-(3-imino-8-methyl-imidazo[1,5-a]pyridin-2-yl)-ethanon als Trifluoressigsäuresalz,
1-(3,5-Di-tert-butyl-4-hydroxy-phenyl)-2-(3-imino-8-methyl-imidazo[1,5-a]pyridin-2-yl)-ethanon als Hydrobromidsalz,
1-(3,5-Di-tert-butyl-4-hydroxy-phenyl)-2-(3-imino-7-trifluoromethyl-imidazo[1,5-a]pyridin-2-yl)-ethanon als Trifluoressigsäuresalz,
1-(3,5-Di-tert-butyl-4-hydroxy-phenyl)-2-(3-imino-7-trifluoromethyl-imidazo[1,5-a]pyridin-2-yl)-ethanon als Hydrobromidsalz,
2-[2-(3,5-Di-tert-butyl-4-hydroxy-phenyl)-2-oxo-ethyl]-3-imino-2,3-dihydro-imidazo[1,5-a]pyridine-6-carbonsäuremethylester als Trifluoressigsäuresalz,
2-[2-(3-tert-Butyl-4-methoxy-5-morpholin-4-yl-phenyl)-2-oxo-ethyl]-3-imino-2,3-dihydro-imidazo[1,5-a]pyridin-6-carbonsäuremethylester als Trifluoressigsäuresalz,
1-(3-tert-Butyl-4-methoxy-5-morpholin-4-yl-phenyl)-2-(3-imino-imidazo[1,5-a]pyridin-2-yl)-ethanon als Trifluoressigsäuresalz,
1-(3-tert-Butyl-4-methoxy-5-morpholin-4-yl-phenyl)-2-(3-imino-8-methyl-imidazo[1,5-a]pyridin-2-yl)-ethanon als Trifluoressigsäuresalz, 1-(3-Dimethylamino-5-pentafluorsulfanyl-phenyl)-2-(3-imino-imidazo[1,5-a]pyridin-2-yl)-ethanon als Trifluoressigsäuresalz,
2-[2-(8-tert-Butyl-4-methyl-3,4-dihydro-2H-benzo[1,4]oxazin-6-yl)-2-oxo-ethyl]-7-ethoxy-3-imino-2,3-dihydro-imidazo[1,5-a]pyridin-6-carbonsäure-methylamid als Trifluoressigsäuresalz,
2-[2-(3-tert-Butyl-4-methoxy-5-morpholin-4-yl-phenyl)-2-oxo-ethyl]-7-chlor-3-imino-2,3-dihydro-imidazo[1,5-a]pyridin-6-carbonsäure-methylamid als Trifluoressigsäure-salz, 2,2,2-Trifluor-N-{3-[2-(3-imino-imidazo[1,5-a]pyridin-2-yl)-acetyl]-5- pentafluor-sulfanyl-phenyl}-acetamid als Trifluoressigsäuresalz,
1-(3-Brom-4-methoxy-5-trifluormethyl-phenyl)-2-(3-imino-imidazo[1,5-a]pyridin-2-yl)-ethanon als Trifluoressigsäuresalz,
2-(3-Imino-imidazo[1,5-a]pyridin-2-yl)-1-(4-methoxy-3-morpholin-4-yl-5-trifluormethylphenyl)-ethanon als Trifluoressigsäuresalz,
6-Ethoxy-3-imino-2-[2-(3-methylamino-5-pentafluorsulfanyl-phenyl)-2-oxo-ethyl]-2,3-dihydro-1H-imidazo[1,5-a]-pyridin-5-on als Trifluoressigsäuresalz,
2-[2-(3-tert-Butyl-4-methoxy-5-morpholin-4-yl-phenyl)-2-oxo-ethyl]-6-ethoxy-3-imino-2,3-dihydro-1H-imidazo[1,5-a]pyridin-5-on als Trifluoressigsäuresalz, 2-[2-(3-tert-Butyl-4-methoxy-5-morpholin-4-yl-phenyl)-2-oxo-ethyl]-7-ethoxy-3-imino-2,3-dihydro-imidazo[1,5-a]pyridin-6-carbonsäure-methylamid als Trifluoressigsäure-salz oder
2-[2-(3-tert-Butyl-4-methoxy-5-morpholin-4-yl-phenyl)-2-oxo-ethyl]-3-imino-5-methoxy-2,3-dihydro-imidazo[1,5-a]pyridin-7-carbonsäure-ethylester als Trifluoressigsäuresalz.

Unter dem Begriff "(C₁-C₄)-Alkyl" oder "(C₁-C₆)-Alkyl" werden Kohlenwasserstoffreste verstanden, deren Kohlenstoffkette geradkettig oder verzweigt ist und 1 bis 4 Kohlenstoffatome oder 1 bis 6 Kohlenstoffatome enthält, beispielsweise Methyl, Ethyl, Propyl, Isopropyl, Butyl, Isobutyl, sec-Butyl, tertiär-Butyl, Pentyl, Isopentyl, Neopentyl, 1-Ethylpropyl, Hexyl, 2,3-Dimethylbutyl oder Neohexyl.
Unter dem Begriff "-(C₀-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, 1-Methylmethylen, Propylen, 1-Methylethylen, Butylen, 1-Propylmethylen, 1-Ethyl-1-methylmethylen, 1,2-Dimethylethylen, 1,1-Dimethylmethylen, 1-Ethylethylen, 1-Methylpropylen, 2-Methylpropylen. "-C₀-Alkylen" ist eine kovalente Bindung.
Unter dem Begriff "-O-(C₁-C₈)-Alkyl" werden Alkoxyreste verstanden, deren Kohlenstoffkette geradkettig oder verzweigt ist und 1 bis 8 Kohlenstoffatome enthält, beispielsweise Methoxy, Ethoxy, Propoxy, lso-Propoxy, Butoxy, Iso-Butoxy oder tertiär-Butoxy.
Unter dem Begriff "(C₃-C₆)-Cycloalkyl" werden Reste verstanden wie Verbindungen, die sich von 3- bis 6-gliedrige Monocyclen wie Cyclopropan, Cyclobutan, Cyclopentan oder Cyclohexan herleiten.
Unter dem Begriff "-(C₆-C₁₄)-Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.
Unter dem Begriff "Het" werden Ringsysteme verstanden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-lsothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrol, Thienopyridin, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl oderXanthenyl. R5 und R6, R6 und R7, R7 und R8 oder R8 und R9 bilden zusammen mit den Ringatomen, an die sie gebunden sind, einen vier- bis achtgliedrigen Heterocyclus, der zusammen mit dem Phenylring, an den der Heterocyclus anneliert ist, ein bicyclisches System bildet, werden Verbindungen verstanden, die aus zwei miteinander verbundenen Ringsystemen bestehen, worin ein Ring ein Phenylrest darstellt und der andere Ring einen teilweise gesättigten oder aromatisches Ringsystem bildet, das je nach Ringgröße ein, zwei oder drei gleiche oder verschiedene Heteroatome aus der Reihe Sauerstoff, Stickstoff oder Schwefel enthält. Beispiele für diese Ringsysteme sind Reste wie Benzimidazol, Benzisothiazol, Benzisoxazol, Benzo[1,3]dioxol, Benzofuranyl, Benzothiazol, Benzisoxazol, Benzothiofuran, Benzothiophen, Benzo[1,3]oxathiol, Benzoxazol, Benzthiazol, Benztriazolyl, Chinazolin, Chinazofon, Chinolin, 4H-Chinolizin, Chinoxalin, Chroman, Chromen, Cinnolin, 2,3-Dihydro-benzo[1,4]dioxin, 2,3-Dihydro-benzofuranyl, 1,3-Dihydro-isobenzofuran, 3,4-Dihydro-2H-benzo[1,4]oxazin, 2,3-Dihydro-benzooxazol, 2,3-Dihydro-benzothiazol, 1,3-Dihydro-benzo[c]thiophen, 2,3-Dihydro-benzo[b]thiophen, Indazol, Indol, Indolin, Isobenzofuran, Isochinolin, Isochroman, Isoindazol, Isoindol, Isoindolin, 7-Oxa-bicyclo[4.2.0]octa-1,3,5-trien, Phthalazin, 2,3,4,5-Tetrahydro-1H-benzo[b]azepin, 6,7,8,9-Tetrahydro-5-oxa-9-aza-benzocyclohepten, 3,4,5,6-Tetrahydro-2H-benzo[b][1,4]oxazozin, Tetrahydrochinolin, 1,2,3,4-Tetrahydro-chinoxalin oder Tetrahydroisochinolin.
Unter dem Begriff "-(C₁-C₃)-Fluoralkyl" wird ein partiell oder vollständig fluorierter Alkylrest verstanden, der sich beispielsweise von folgenden Resten ableitet -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂ oder -CF₂-CF₂-CH₂F.
Unter dem Begriff "Halogen" wird Fluor, Chlor, Brom oder Jod verstanden, bevorzugt sind Fluor, Chlor oder Brom, insbesondere Fluor oder Chlor.
Unter dem Begriff "=O" werden Reste wie Carbonyl (-C(O)-) oder Nitroso (-N=O) verstanden.

Funktionelle Gruppen der verwendeten Intermediate, beispielsweise Amino- oder Carboxylgruppen in der Verbindung der Formel I, können dabei durch geeignete Schutzgruppen maskiert werden. Geeignete Schutzgruppen für Aminofunktionen sind beispielweise die t-Butoxycarbonyl-, die Benzyloxycarbonyl- oder die Phtalolylgruppe sowie die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Greene, T. W., Wuts, P.G.M., Protective Groups in Organic Synthesis (1999), 3rd Ed., Wiley-Interscience). Der Begriff Schutzgruppe kann auch entsprechende polymergebundene Schutzgruppen umfassen.
Die erfindungsgemäßen Verbindungen können nach wohlbekannten Verfahren oder nach hier beschriebenen Verfahren hergestellt werden.

Umfasst sind auch mögliche tautomere Formen der angegebenen Strukturen wie beispielsweise der Formel III/IIIt oder I/It (wenn R1 in Formel I beispielsweise OH ist):

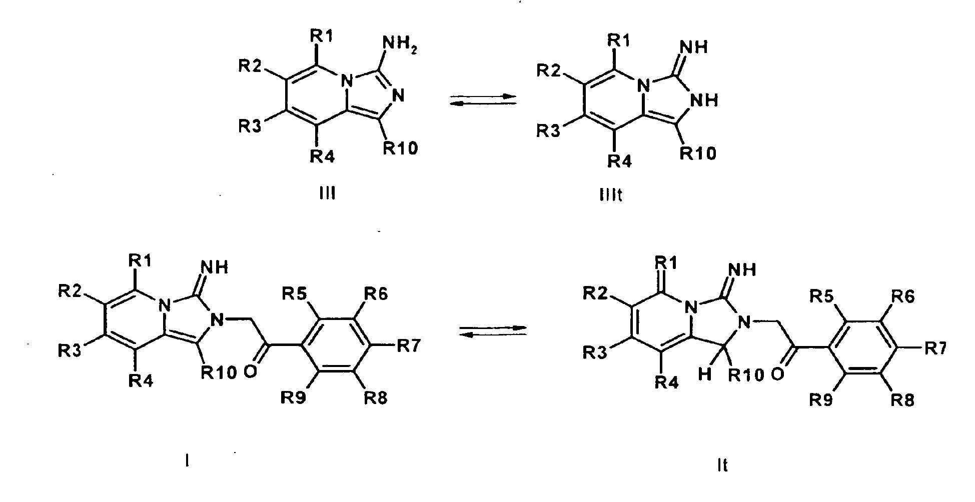

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel II 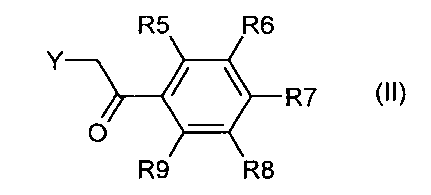 wobei die Reste R5, R6, R7, R8 und R9 wie in Formel I definiert sind und Y für Chlorid, Bromid, Mesylat oder Tosylat steht mit einer Verbindung der Formel III 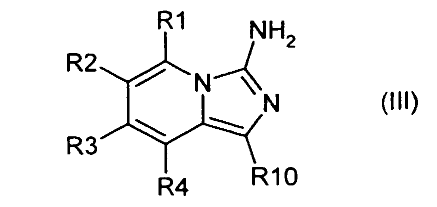 in Gegenwart einer Base und eines Lösungsmittels zu einer Verbindung der Formel I umsetzt, oder
b) eine Verbindung der Formel VII 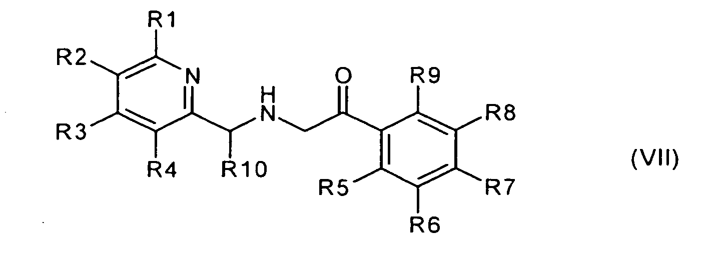 wobei die Reste R1 bis R10 wie in Formel I definiert sind mit einer Verbindung Z-CN, wobei Z Tosylat oder Bromid bedeutet, in Gegenwart einer Base zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

Die Erfindung betrifft ferner ein Verfahren zur Herstellung der Verbindung der Formel I nach Schema 1.

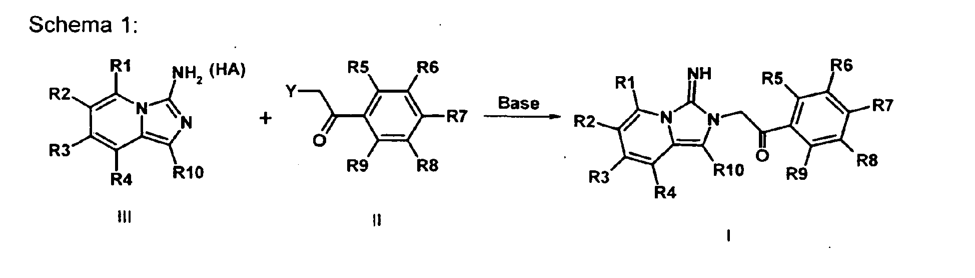

Die Edukte II und III, wobei III gegebenenfalls (ggf.) in Form eines Salzes vorliegt, werden dabei bei RT oder leicht erhöhter Temperatur von 40 °C bis 60 °C vorteilhafterweise in Gegenwart einer Base, bevorzugt Hünig-Base, in einem Lösungsmittel, bevorzugt Dimethylformamid (DMF), zu der Verbindung der Formel I umgesetzt. Die Reste R1 bis R10, sind wie in Formel definiert, Y entspricht einer guten Fluchtgruppe wie Chlorid, Bromid, Mesylat oder Tosylat, bevorzugt Bromid oder Mesylat.
Verbindungen der Formel II sind käuflich oder lassen sich nach literaturbekannten Verfahren gewinnen. Ein Zugang zu Pentafluorsulfanyl-Derivaten der Formel II ist unten beschrieben.
Verbindungen der Formel III lassen sich entsprechend Schema 2 darstellen (siehe auch DE 2211796).

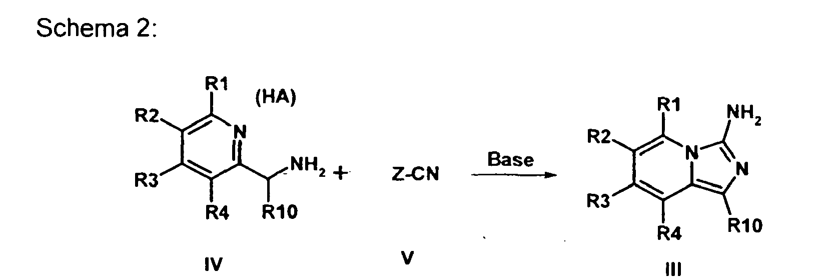

Dabei werden Verbindungen der Formel IV - ggf. in Form ihrer Salze (HA) - bevorzugt in Gegenwart einer Base mit einer Cyan-Quelle V zu den gewünschten lmidazopyridinen cyclisiert. Als Säuren HA kommen bevorzugt HBr, HCl, Trifluoressigsäure (TFA) und Schwefelsäure in Frage. Z steht für eine gute Fluchtgruppe, bevorzugt Tosylat oder Bromid.
Verbindungen vom Typ der Formel IV sind käuflich oder können entsprechend Schema 3a gewonnen werden.

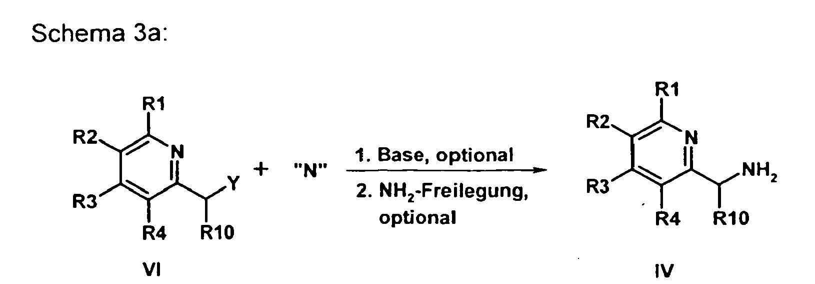

Dabei werden Pyridine vom Typ VI in Gegenwart eines Stickstoff-Nucleophils "N" und ggf. einer Folgereaktion zur Freilegung der -NH₂-Gruppe in die Pyridylmethylamine IV übergeführt. Als Stickstoff-Nukleophile kommen dabei Ammonik, das direkt ohne weitere Freilegung zu den Verbindungen vom Typ IV führt, Azide, wie Natriumazid, das zur Etablierung der Aminofunktion nachträglich reduziert werden muss, wozu Triphenylphosphin (Bioorg. Med. Chem. Lett. 2925, 2002) oder Edelmetallkatalysatoren wie Palladium oder Platin in Gegenwart von Wasserstoff in Frage kommen (J. Med. Chem. 5005, 2002), Phthalimid, das nachträglich zur Freilegung der Aminofunktion mit Hydrazin behandelt werden muss (J. Med. Chem. 1315, 2004), oder Urotropin, das zur Freilegung der Aminofunktion mit Säure, bevorzugt Salzsäure, behandelt werden muss (Synthesis 2145, 2003), in Frage. Die Reste R1 bis R4, R10 und Y sind wie oben definiert, wobei Y hier auch -OH sein kann, das in situ zu einer guten Fluchtgruppe aktiviert wird, die dann nachfolgend von einem der oben erwähnten Stickstoff-Nucleophile substituiert wird (Chem. Pharm. Bul 1493, 1989; Bioorg. Med. Chem. Lett. 2463, 2004).

Ein weiterer Zugang zu Aminen von Typ IV ist in Schema 3b dargestellt.

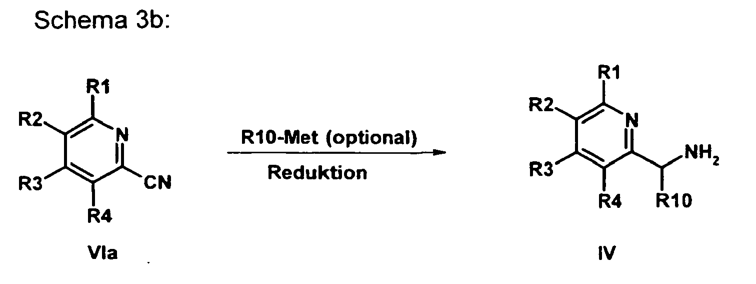

Ausgehend von 2-Cyanopyridinen vom Typ VIa wird die Nitrilfunktion mit Reduktionsmitteln wie Wasserstoff in Gegenwart von Metallkatalysatoren wie Palladium oder Raney-Nickel zu den Aminen vom Typ IV reduziert. Wird die Nitrilfunktion vor der Reduktion mit metallorganischen Reagenzien wie Grignard- oder Organolithium-Verbindungen umgesetzt, lässt sich auch auf diesem Weg der Substituent R10 einführen. Die dadurch intermediär erhaltenen Imine lassen sich durch Natriumborhydrid, Natriumtriacetoxyborhydrid oder Natriumcyanoborhydrid zu den Aminen IV reduzieren. Die Reste R1 bis R4 und R10 sind wie oben definiert. Met steht für -Li oder -MgBr.

Alternativ lassen sich Verbindungen der Formel (I) wie in Schema 4 dargestellt herstellen.

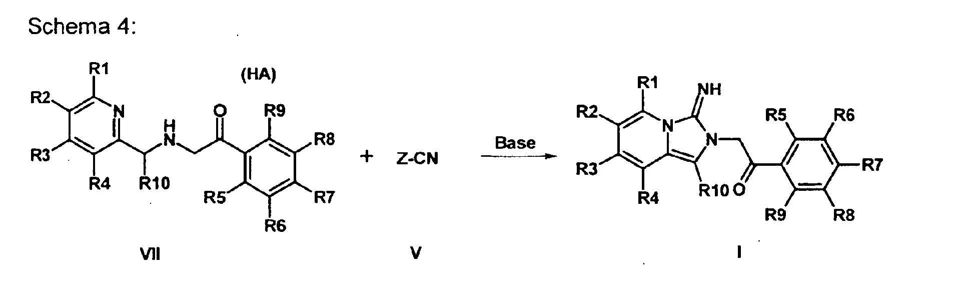

Dabei werden Verbindungen der Formel VII-ggf. in Form ihrer Salze (HA)- in einem Lösungsmittel wie Wasser, Methanol, Ethanol, Essigsäure, Acetonitril, Toluol oder geeigneten Gemischen dieser Lösungsmittel, bevorzugt Toluol, in Gegenwart einer Base, bevorzugt Hünig-Base, mit einer Cyan-Quelle V, bevorzugt Bromcyan zu den gewünschten Imidazopyridinen cyclisiert.

Verbindungen der Formel VII werden entsprechend Schema 5 erhalten, indem Amine der Formel III mit Acetophenon-Derivaten vom Typ der Formel II umgesetzt werden. Dies geschieht bevorzugt in Lösungsmitteln wie DMF, Tetrahydrofuran (THF) oder Acetonitril, bevorzugt in THF. Als Basen kommen Hünig-Base, Lithiumhexamethyldisilazan oder Kaliumcarbonat in Frage, bevorzugt Lithiumhexamethyldisilazan. Die Reste sind dabei wie oben definiert.

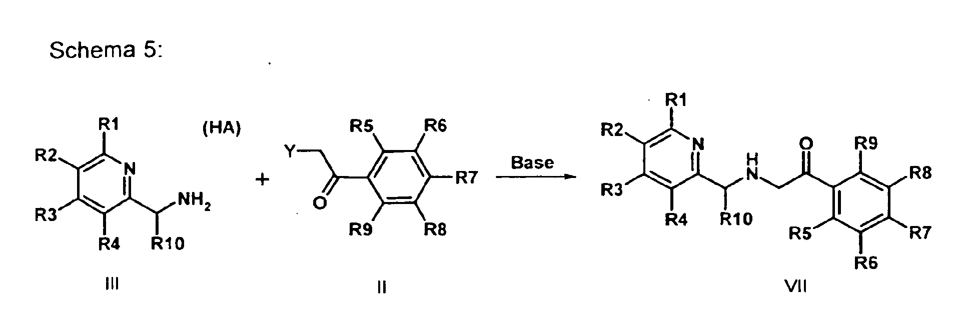

Alternativ lassen sich Verbindungen der Formel VII nach Schema 6 erhalten, indem Amine der Formel IX mit Pyridyl-Derivaten vom Typ der Formel VIII umgesetzt werden. Die Umsetzung erfolgt in Lösungsmitteln wie DMF, THF, Acetonitril oder Ethanol, bevorzugt THF. Als Basen kommen Hünig-Base, Lithiumhexamethyldi-silazan oder Kaliumcarbonat in Frage, bevorzugt Lithiumhexamethyldisilazan. Die Reste sind dabei wie oben definiert.

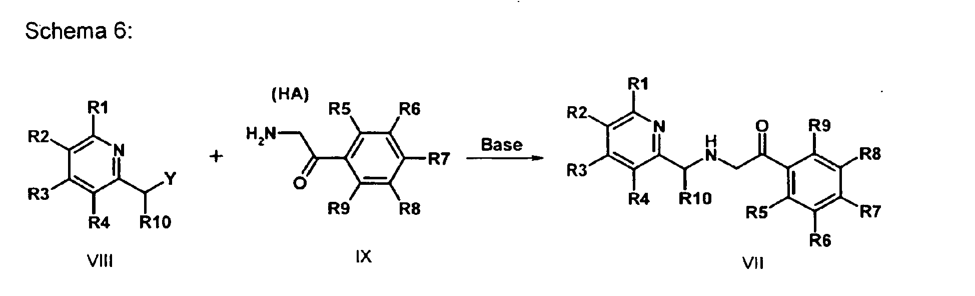

Ist einer der Reste R5, R6, R7, R8 oder R9 ein Pentafluorsulfanyl (-SF₅), so lassen sich diese Verbindungen vom Typ der Formel IIa (Y=Br) wie in Schema 7 beschrieben darstellen.

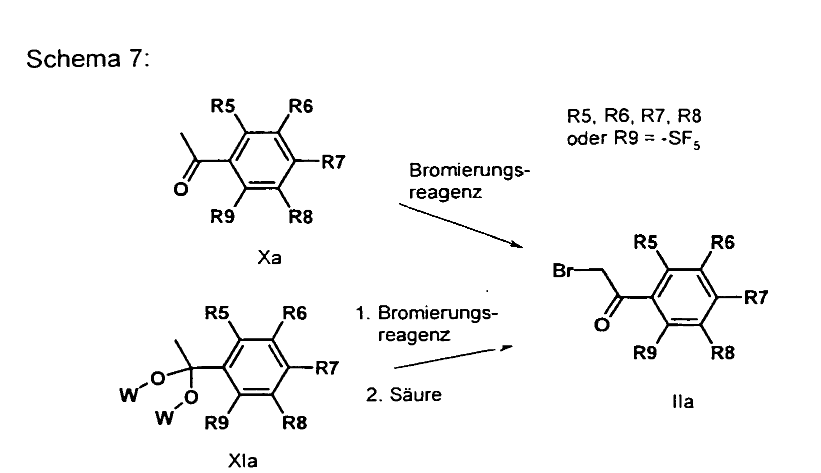

Dabei können die Acetophenon-Derivate der Formel Xa entweder direkt beispielsweise mit Br₂, N-Brom-succinimid (NBS) oder Phenyl-trimethyl-tribromid, bevorzugt in Eissessig, Methanol oder Methanol/THF-Gemischen, zu Verbindungen der Formel IIa bromiert werden oder aber es werden die entsprechenden Ketale der Verbindung der Formel XIa der Acetophenon-Derivate X mit beispielsweise obigen Bromierungsreagenzien, vorzugsweise Phenyl-trimethyl-tribromid, bromiert. Anschließend werden zur Gewinnung der Verbindungen der Formel IIa die Ketale in Gegenwart von Säure, beispielsweise Salzsäure, Bromwasserstoffsäure, Schwefelsäure, Trifluoressigsäure, bevorzugt Schwefelsäure, gespalten.

Die Ketale der Formel XI, XIa und XI' lassen sich ausgehend von den Ketonen der Formel X durch dem Fachmann bekannte Ketalisierungsreaktionen gewinnen. Bevorzugt wird die Umsetzung zu den Verbindungen der Formel XI in Methanol mit Methyl-ortho-formiat in Gegenwart von DL-10-Camphersulfonsäure (Schema 8) durchgeführt.

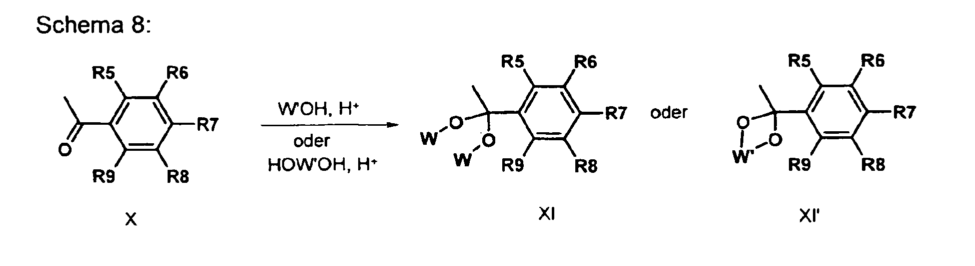

Die Reste R5 bis R9 sind dabei wie oben definiert. Der Rest W entspricht einer -(C1-C4)-Alkylgruppe. Der Rest W' entspricht Ethylen, Propylen oder Butylen oder bildet zusammen mit der Gruppe -O-C-O- einen 1,3- Dioxo-Ring der Ringgröße 5,6 oder 7. Ketale dieses Typs werden durch Umsetzung mit Alkylenglykolen wie Ethylenglykol in Gegenwart von Säuren wie Schwefelsäure oder para-Toluolsulfonsäure und/oder wasserentziehenden Mitteln erhalten. Im einfachsten Fall arbeitet man in Toluol in Gegenwart katalytischer Mengen para-Toluolsulfonsäure am Wasserabscheider.

Komplexer substituierte Verbindungen der Formel Xa mit R5, R6, R7, R8 oder R9 gleich Pentafluorsulfanyl und mit einem weiteren R5, R6, R7, R8 oder R9 ungleich Wasserstoff lassen sich ausgehend von käuflichen Pentafluorsulfanyl-Derivaten gewinnen. Nicht käufliche Derivate können in Analogie zu bekannten Herstellungsverfahren gewonnen werden (Tetrahedron 56 (2000) 3399; Organic Letters 4 (17) (2002) 3013; WO 2005/047240). Für das bisher nicht beschriebene 1-(3-Dimethylamino-5-pentafluorsulfanyl-phenyl)-ethanon ist ein Syntheseweg in Schema 9 aufgezeigt.

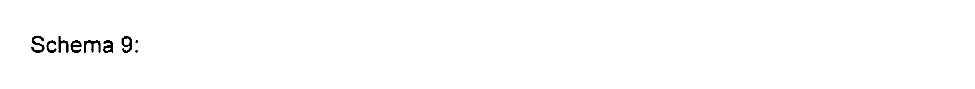

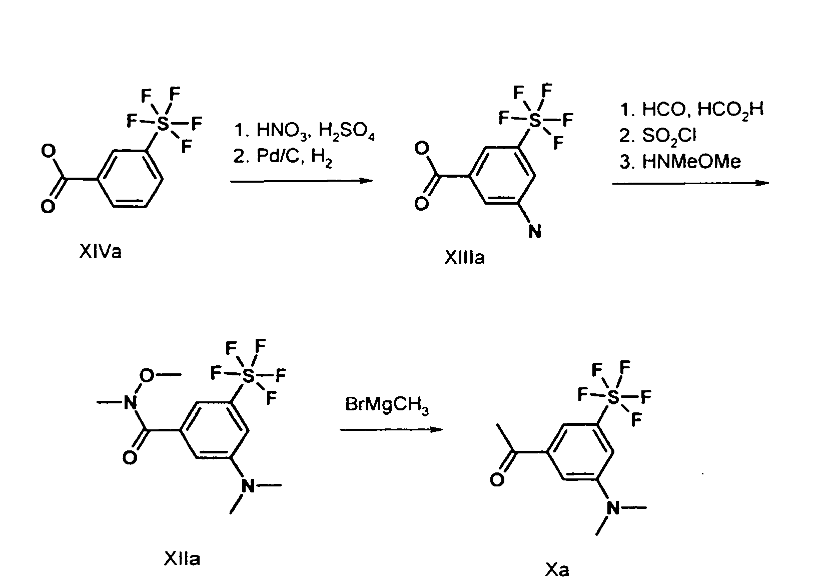

Ausgehend von käuflicher 3-(Pentafluorsulfanyl)-benzoesäure XIVa wurde mit dem Fachmann bekannten Umsetzungen zunächst nitriert und anschließend mit Palladium auf Kohle in Gegenwart von Wasserstoff zum Amin reduziert. Die erhaltene 3-Amino-5-pentafluorsulfanyl-benzoesäure XIIIa wurde dann unter Eschweiler-Clark-Bedingungen am Aminstickstoff dimethyliert, die Carbonsäure mit Thionylchlorid ins Säurechlorid übergeführt und anschließend mit O,N-Dimethyl-hydroxylamin umgesetzt. Das so gewonnene 3-Dimethylamino-N-methoxy-N-methyl--5-pentafluorsulfanyl-benzamid XIIa wurde mit Methylmagnesiumbromid in die entsprechenden Pentafluorsulfanyl-Derivate der Formel Xa übergeführt.

Eine nach den Schemata 1 oder 4 hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren und Abspaltung der chiralen Hilfsgruppen in die reinen Enantiomeren aufgetrennt werden (Verfahren c), oder die nach den Schemata 1 oder 4 hergestellte Verbindung der Formel I kann entweder in freier Form isoliert oder im Falle des Vorliegens von sauren oder basischen Gruppen in physiologisch verträgliche Salze umwandelt werden (Verfahren d).

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze oder Hydrochloride, Sulfate, Hemisulfate, Methylsulfonate, p-Toluolsulfonate, alle möglichen Phosphate sowie Salze der Aminosäuren, natürlicher Basen oder Carbonsäuren wie Lactate, Citrate, Tartrate, Acetate, Adipinate, Fumarate, Gluconate, Glutamate, Maleinate oder Pamoate.
Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Enthalten Verbindungen der Formel I saure Funktionalität, so lassen sich mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze bilden. Basische Gruppen der Verbindungen der Formel I, bilden mit Säuren Säureadditionssalze. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin- oder Trifluoressigsäure in Frage.

Im Verfahrensschritt d) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, kann auch eine fraktionierte Kristallisation der mit einer optisch aktiven Base oder Säure als Hilfsstoff gebildeten diastereomeren Salze durchgeführt werden. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-Funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der lsomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung des Protease-aktivierten Rezeptors 1 (PAR1) behandelbar sind. So eignen sich die erfindungsgemäßen Verbindungen sowohl für eine prophylaktische als auch für eine therapeutische Anwendung am Menschen. Sie eignen sich sowohl für eine akute Behandlung als auch für eine Langzeittherapie. Die Verbindungen der Formel I können eingesetzt werden in Patienten, die an Störungen des Wohlbefindens oder Krankheiten leiden, die mit Thrombosen, Embolien, Hyperkoagulabilität oder fibrotischen Veränderungen einhergehen.
Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können die Verbindungen der Formel I eingesetzt werden bei allen Eingriffen, die zu einem Kontakt des Blutes mit Fremdoberflächen führen wie bei Dialysepatienten und Patienten mit Verweilkathetern. Verbindungen der Formel I können eingesetzt werden, um die Thrombosegefahr nach chirurgischen Eingriffen wie bei Knie- und Hüftgelenksoperationen zu reduzieren. Verbindungen der Formel I eignen für die Behandlung von Patienten mit disseminierter intravaskulärer Koagulation, Sepsis und anderen intravaskulären Ereignissen, die mit einer Entzündung einhergehen. Weiterhin eignen sich Verbindungen der Formel I für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. Verbindungen der Formel I eignen sich zur Verlangsamung oder Verhinderung solcher Prozesse.

Weitere Indikationen für den Einsatz der Verbindungen der Formel I sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formel I eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung von Stents mit Verbindungen der Formel I und anderen Oberflächen, die im Körper mit Blut in Kontakt kommen, ist möglich. Die Erfindung betrifft auch ein Verfahren zur Herstellung eines Arzneimittels, das dadurch gekennzeichnet ist, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.
Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.
Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Verbindungen der Formel I können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

### Beispiele

Endprodukte wurden in der Regel durch eine chromatographisch-massenspektroskopische Methode (LCUV/ESI-MS-Kopplung) und ¹H-NMR charakterisiert. Beschrieben sind die Verbindungen durch Angabe der zugehörigen Retentionszeit im lonenstrom (LCMS-rt) und des entsprechenden M+H⁺-Signals im zugehörigen Massenspektrum. Konnte unter den beschriebenen Bedingungen kein M+H⁺-Massensignal erhalten werden, wurden alternativ die ¹H-NMR-Daten angegeben. Verwendete Abkürzungen sind entweder erläutert oder entsprechen den üblichen Konventionen. Soweit nicht anders angegeben, wurden chromatographische Trennungen an Kieselgel mit Ethylacetat/Heptan- oder DCM/Methanol-Gemischen als Laufmittel durchgeführt.
Das Abdampfen von Lösungsmitteln geschah in der Regel unter vermindertem Druck bei 35 °C bis 45 °C am Rotationsverdampfer und wird mit "vom Lösungsmittel befreit", "eingeengt" oder "Lösungsmittel entfernt" umschrieben.
Wenn nicht anders aufgeführt, wurden die LCUV/MS-Analysen unter folgenden Bedingungen durchgeführt:

| | |
|---|---|
| Säule: | YMC J'shere ODS H80 20x2,1 mm, Waters GmbH, Helfmann-Park 10, 65760 Eschborn, Deutschland; Packungsmaterial 4 µm, |
| Eluent: | ACN:H₂O+0,05% TFA (Fluss 1 ml/min) |
| Gradient: | 4:96 (0 min) → 95:5 (2 min) → 95:5 (2,4 min) →4:96 (2,45 min) |
| lonisierung: | ESI⁺ |

Davon abweichend wurden unter folgenden Bedingungen - im Text mit "Methode B" gekennzeichnet - LCUV/MS-Analysen durchgeführt:

| | |
|---|---|
| Säule: | YMC J'sphere 33 x 2; Packungsmaterial 4 µM |
| Eluent: | ACN + 0,05% TFA : H₂O + 0,05% TFA (Fluss 1 ml/min) |
| Gradient: | 5:95(0 min) → 95:5(2,5 min) → 95:5(3,0 min) |
| lonisierung: | ESI⁺ |

Präparative HPLC mit Reversed-Phase-(RP)-Kieselgel wurde mit den folgenden Methoden durchgeführt:

Methode A, Standard-Methode wenn im Text keine andere erwähnt ist

| | |
|---|---|
| Säule: | Merck (Darmstadt, Deutschland) Purosphere® RP18 25x250 mm, 10 µm |
| Lösungsmittel: | ACN:H₂O+0,05%TFA (Fluss 25 ml/min) |
| Gradient: | 10:90 (0 min) → 90:10 (40 min) |
| Methode B | |
| Säule: | Merck Purosphere® RP18 25x250 mm, 10 µm |
| Lösungsmittel: | ACN:H₂O+0,05%TFA (Fluss 25 ml/min) |
| Gradient: | 0:100 (0 min) → 0:100 (5 min) → 20:80 (20 min) |

Die Umsetzungen fanden in Standard-Reaktionsapparaturen wie Ein- oder Mehrhalskolben statt, die wenn nicht anders beschrieben, dem Bedarf angemessen 5 ml bis 2000 ml Volumen fassten und je nach Anforderung mit Septum, Stopfen, Kühler, Rührer oder anderen Ausrüstungsgegenständen bestückt waren. Wenn nicht anders erwähnt, fanden alle Reaktionen unter Argon als Schutzgas statt und wurden mit Magnetrührern gerührt.

### Verwendete Abkürzungen:

- abs.: absolut
- ACN: Acetonitril
- Boc: Butoxycarbonyl
- DCM: Dichlormethan
- DIPEA: N,N-Diisopropylethylamin (Hünig-Base)
- DMF: Dimethylformamid
- DMSO: Dimethylsulfoxid
- LCMS-Rt: Retentionszeit der Verbindung im lonenstrom
- LCUV/MS: Flüssigkeitschromatographie Ultraviolett-/Massenspektroskopie
- MeOH: Methanol
- RT: Raumtemperatur (20 °C bis 25 °C)
- TFA: Trifluoressigsäure
- THF: Tetrahydrofuran

### Beispiel 1

1-(3,5-Di-tert-butyl-4-hydroxy-phenyl)-2-(3-imino-imidazo[1,5-a]pyridin-2-yl)-ethanon als Trifluoressigsäuresalz 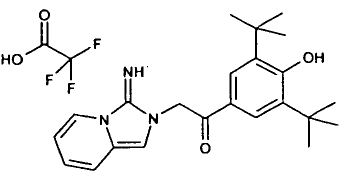
1a) 1-(3,5-Di-tert-butyl-4-hydroxy-phenyl)-2-[(pyridin-2-ylmethyl)-amino]-ethanon-Hydrobromid 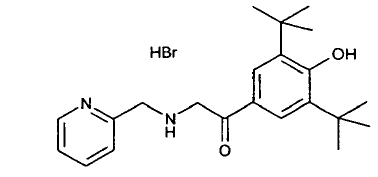 2-(Aminomehtyl)pyridin (100 mg) wurde in absolutem THF (12 ml) vorgelegt und bei RT unter Rühren und Argon mit Lithiumhexamethyldisilazan-Lösung (1,01 ml, 1 M in THF) versetzt. Nach 30 min wurde 2-Brom-1-(3,5-di-tert-butyl-4-hydroxy-phenyl)-ethanon (0,5 eq., 151 mg, gelöst in 1,5 ml absolutem THF) zugetropft. Zur Vervollständigung der Reaktion wurde die Restmenge Bromid (151 mg) zugegeben und nach 2 Stunden der gebildete Niederschlag abgesaugt und getrocknet. Die erhaltenen 98 mg der Titelverbindung sind mit geringen Mengen THF verunreinigt, die für die weitere Umsetzung aber nicht stören.

| | |
|---|---|
| LCMS-Rt: 1,21 min | [M+H⁺]: 355,1 |

1b) 1-(3,5-Di-tert-butyl-4-hydroxy-phenyl)-2-(3-imino-imidazo[1,5-a]pyridin-2-yl)-ethanon als Trifluoressigsäuresalz
1-(3,5-Di-tert-butyl-4-hydroxy-phenyl)-2-[(pyridin-2-ylmethyl)-amino]-ethanon-Hydrobromid (95 mg) wurde in Wasser gelöst. Nach Zugabe von gesättigter Natriumhydrogencarbonat-Lösung wurde mit Essigester extrahiert (drei Mal). Die vereinigten Essigester-Phasen wurden über Magnesiumsulfat getrocknet, filtriert und eingeengt. Es wurden 67 mg der freien Base erhalten.
Diese wurde mit Toluol (5 ml) aufgenommen und unter Rühren mit Bromcyan (22 mg, gelöst in 1,5 ml Toluol) versetzt. Nach 1,5 h wurde das Lösungsmittel eingeengt und der Rückstand mittels präparativer HPLC gereinigt. Die produktenthaltenden Fraktionen wurden vereinigt, vom Acetonitril befreit und gefriergetrocknet. Es wurden 40 mg der gewünschten Verbindung erhalten.

| | |
|---|---|
| LCMS-Rt: 1,30 min | [M+H⁺]: 380,1 |

Alternativ lässt sich 1-(3,5-Di-tert-butyl-4-hydroxy-phenyl)-2-(3-imino-imidazo[1,5-a]pyridin-2-yl)-ethanon als Trifluoressigsäuresalz wie folgt herstellen:
1c) Imidazo[1,5-a]pyridin-3-ylamin als Trifluoressigsäuresalz 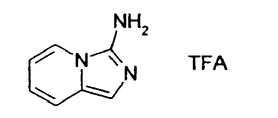 2-Aminomethylpyridin (1 g) wurde in abs. Toluol (5 ml) gelöst und unter Rühren mit Bromcyan (1 g), gelöst in abs. Toluol (5 ml), versetzt. Nach 4 h Rühren bei RT wurde über das Wochenende stehen gelassen. Dann wurde das Lösungsmittel vom entstandenen Niederschlag dekantiert und der Rückstand mit Acetonitril gewaschen. Ungefähr 300 mg von 1,7 g des Niederschlags wurden anschließend mittels präparativer HPLC (Methode B) gereinigt. Die produktenthaltenden Fraktionen wurden vereinigt, vom Acetonitril befreit und gefriergetrocknet. Es wurden 74 mg der gewünschten Verbindung erhalten.

| | |
|---|---|
| LCMS-Rt: 0,34 min | [M+H⁺]: 134,1 |

1d) 1-(3,5-Di-tert-butyl-4-hydroxy-phenyl)-2-(3-imino-imidazo[1,5-a]pyridin-2-yl)-ethanon als Trifluoressigsäuresalz

Bei RT und unter Rühren wurde Imidazo[1,5-a]pyridin-3-ylamin-Trifluoressigsäure-salz (10 mg) in abs. DMF (1,5 ml) gelöst und mit Hünig-Base (7µl) versetzt. Dann wurde 2-Brom-1-(3,5-di-tert-butyl-4-hydroxy-phenyl)-ethanon (14 mg, gelöst in 0,5 ml DMF) zugetropft. Nach 4 h Rühren bei RT wurde über Nacht stehen gelassen und dann mit gesättigter Kochsalzlösung versetzt und drei Mal mit Essigester extrahiert. Die vereinigten organischen Phasen wurden über Magnesiumsulfat getrocknet, filtriert und eingeengt. Reste von DMF wurde im Hochvakuum beseitigt. Das erhaltene Rohprodukt wurde mittels präparativer HPLC gereinigt. Dieproduktenthaltenden Fraktionen wurden vereinigt, vom Acetonitril befreit und gefriergetrocknet. Es wurden 9 mg der gewünschten Verbindung erhalten.

| | |
|---|---|
| LCMS-Rt: 1,33 min | [M+H⁺]: 380,2 |

### Beispiel 2

2-(3-Imino-imidazo[1,5-a]pyridin-2-yl)-1-(3-pentafluorsulfanyl-phenyl)-ethanon als Trifluoressigsäuresalz 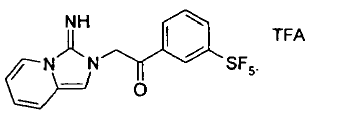
2a) 2-Brom-1-(3-pentafluorsulfanyl-phenyl)-ethanon 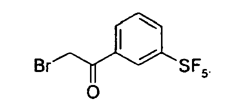 3-Pentafluorsulfanyl-acetophenon (400 mg) wurde in Eisessig (10 ml) vorgelegt und Brom (91 µl, gelöst in 1 ml Eisessig) langsam zugetropft. Nach 4 Stunden Rühren bei RT wurde über Nacht stehen gelassen und dann vom Lösungsmittel befreit. Der Rückstand wurde zwei Mal mit Toluol aufgenommen und zur Trockne gebracht. Das erhaltene Rohprodukt wurde mittels präparativer HPLC aufgereinigt. Die produktenthaltenden Fraktionen wurden vereinigt, vom Acetonitril befreit und gefriergetrocknet. Es wurden 252 mg der gewünschten Verbindung erhalten.

| | |
|---|---|
| LCMS-Rt: 1,69 min | [M+H⁺]: 324,9 |

2b) 2-(3-Imino-imidazo[1,5-a]pyridin-2-yl)-1-(3-pentafluorsulfanyl-phenyl)-ethanon als Trifluoressigsäuresalz Imidazo[1,5-a]pyridin-3-ylamin als Trifluoressigsäuresalz (20 mg, Beispiel 1c) wurde entsprechend Beispiel 1d) mit 2-Brom-1-(3-pentafluorsulfanyl-phenyl)-ethanon (26 mg) in Gegenwart von Hünig-Base umgesetzt. Statt DMF wurde THF als Lösungsmittel verwendet. Es wurden 25 mg der gewünschten Verbindung isoliert.

| | |
|---|---|
| LCMS-Rt: 1,13 min | [M+H⁺]: 378,0 |

### Beispiel 3

2-(1-Cyclopropyl-3-imino-imidazo[1,5-a]pyridin-2-yl)-1-(3,5-di-tert-butyl-4-hydroxyphenyl)-ethanon als Trifluoressigsäuresalz 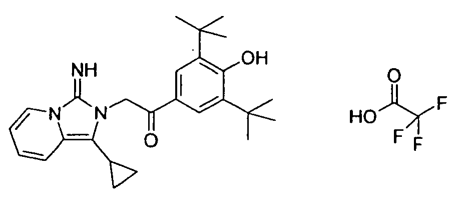
3a) 2-[(Cyclopropyl-pyridin-2-yl-methyl)-amino]-1-(3,5-di-tert-butyl-4-hydroxyphenyl)-ethanon 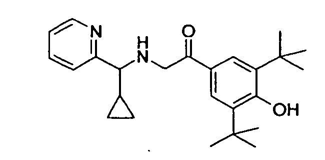 C-Cyclopropyl-C-pyridin-2-yl-methylamin (200 mg) wurde in abs. THF (15 ml) unter Rühren fast vollständig gelöst. Nach Zugabe von Lithiumhexamethyldisilazan-Lösung (1,8 ml, 1 M in THF) wurde 30 min später 2-Brom-1-(3,5-di-tert-butyl-4-hydroxyphenyl)-ethanon (296 mg, gelöst in 3 ml abs. THF) zugetropft. Nach 2 h Rühren wurde Wasser zugegeben und die wässrige Phase drei Mal mit Essigester extrahiert. Die vereinigten Essigesterphasen wurden über Magnesiumsulfat getrocknet, filtriert und eingeengt. Das erhaltene Rohprodukt wurde mittels präparativer HPLC aufgereinigt. Die produktenthaltenden Fraktionen wurden vereinigt, vom Acetonitril befreit, mit gesättigter Kaliumcarbonat-Lösung basisch gestellt und drei Mal mit Essigester extrahiert. Die vereinigten Essigesterphasen wurden über Magnesiumsulfat getrocknet, filtriert und eingeengt. Es wurden 13 mg der gewünschten Verbindung erhalten.

| | |
|---|---|
| LCMS-Rt: 1,33 min | [M+H⁺]: 395,1 |

3b) 2-(1-Cyclopropyl-3-imino-imidazo[1,5-a]pyridin-2-yl)-1-(3,5-di-tert-butyl-4-hydroxy-phenyl)-ethanon als Trifluoressigsäuresalz 2-[(Cyclopropyl-pyridin-2-yl-methyl)-amino]-1-(3,5-di-tert-butyl-4-hydroxy-phenyl)-ethanon (13 mg) wurden wie in Beispiel 1b) beschrieben mit Bromcyan (4 mg) in Toluol cyclisiert. Es wurden 4 mg der gewünschten Verbindung erhalten.

| | |
|---|---|
| LCMS-Rt: 1,42 min | [M+H⁺]: 420,2 |

### Beispiel 4

1-(3,5-Di-tert-butyl-4-hydroxy-phenyl)-2-(3-imino-1-phenyl-imidazo[1,5-a]pyridin-2-yl)-ethanon als Trifluoressigsäuresalz 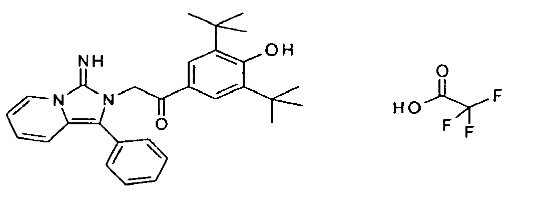
4a) 1-(3,5-Di-tert-butyl-4-hydroxy-phenyl)-2-[(phenyl-pyridin-2-yl-methyl)-amino]-ethanon als Trifluoressigsäuresalz 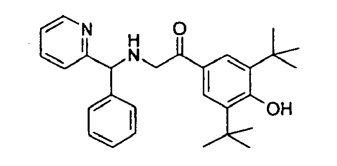 Phenyl-(2-pyridyl)-methylamin-Hydrochlorid (150 mg) wurde in weinig Wasser gelöst, mit gesättigter Natriumhydrogencarbonat-Lösung alkalisch gemacht und drei Mal mit Essigester extrahiert. Die vereinigten Essigesterphasen wurden über Magnesiumsulfat getrocknet, filtriert und eingeengt. Es wurden 113 mg der freien Base erhalten. Davon wurden 55 mg in THF (4 ml) gelöst und unter Rühren 2-Brom-1-(3,5-di-tert-butyl-4-hydroxy-phenyl)-ethanon (96 mg, gelöst in 2 ml THF) zugetropft. Nach 4 h Rühren bei RT wurde über Nacht stehen gelassen und dann der gebildete Niederschlag abfiltriert. Die Mutterlauge wurde eingeengt und mittels präparativer Chromatographie aufgereinigt. Die produktenthaltenden Fraktionen wurden vereinigt, vom Acetonitril befreit und gefriergetrocknet. Es wurden 12 mg der gewünschten Verbindung isoliert.

| | | |
|---|---|---|
| | LCMS-Rt: 1,40 min | [M+H⁺]: 431,3 |

4b) 1-(3,5-Di-tert-butyl-4-hydroxy-phenyl)-2-(3-imino-1-phenyl-imidazo[1,5-a]pyridin-2-yl)-ethanon als Trifluoressigsäuresalz1-(3,5-Di-tert-butyl-4-hydroxy-phenyl)-2-[(phenyl-pyridin-2-yl-methyl)-amino]-ethanon-Trifluoressigsäuresalz (12 mg) wurde in Toluol analog Beispiel 1b) umgesetzt. Es wurden 3 mg der gewünschten Verbindung erhalten.

| | |
|---|---|
| LCMS-Rt: 1,50 min | [M+H⁺]: 456,2 |

### Beispiel 5

1-(3,5-Di-tert-butyl-4-hydroxy-phenyl)-2-(3-imino-8-methyl-imidazo[1,5-a]pyridin-2-yl)-ethanon als Trifluoressigsäuresalz oder Hydrobromid 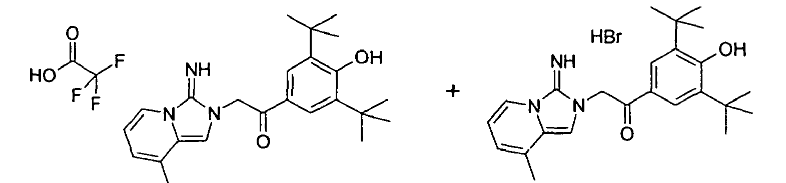
5a) 8-Methyl-imidazo[1,5-a]pyridin-3-ylamin als Trifluoressigsäuresalz 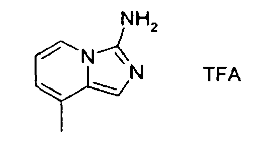 2-Aminomethyl-3-methyl-pyridin (500 mg) wurde in abs. Toluol (15 ml) gelöst und unter Rühren Bromcyan (455 mg, gelöst in 5 ml Toluol) zugetropft. Nach einer Stunde wurde der Niederschlag abgesaugt und die Mutterlauge verworfen. Der Niederschlag und der klebrige Kolbenrückstand wurden mittels präparativer HPLC aufgereinigt. Die produktenthaltenden Fraktionen wurden vereinigt, vom Acetonitril befreit und gefriergetrocknet. Es wurden 191 mg der gewünschten Verbindung alsTrifluoressigsäuresalz isoliert.

| | | |
|---|---|---|
| | LCMS-Rt: 0,68 min | [M+H⁺]: 148,1 |

5b) 1-(3,5-Di-tert-butyl-4-hydroxy-phenyl)-2-(3-imino-8-methyl-imidazo[1,5-a]pyridin-2-yl)-ethanon als Hydrobroimid oder
1-(3,5-Di-tert-butyl-4-hydroxy-phenyl)-2-(3-imino-8-methyl-imidazo[1,5-a]pyridin-2-yl)-ethanon als Trifluoressigsäuresalz
8-Methyl-imidazo[1,5-a]pyridin-3-ylamin (30 mg als Trifluoressigsäuresalz) wurde in abs. THF (5 ml) gelöst, mit Hünig-Base (19 µl) versetzt und langsam 2-Brom-1-(3,5-di-tert-butyl-4-hydroxy-phenyl)-ethanon (38 mg, gelöst in 1,5 ml abs. THF) zugetropft. Nach 2 Stunden Rühren bei RT wurde über Nacht stehen gelassen und dann zur Vervollständigung der Reaktion auf 60°C erwärmt und noch einmal etwas Bromid (4 mg) zugegeben. Der gebildete Niederschlag wurde abgesaugt und getrocknet. Es wurden 8,5 mg der Titelverbindung als Hydrobromid erhalten.

| | |
|---|---|
| LCMS-Rt: 1,30 min | [M+H⁺]: 394,2 |

Zur Gewinnung des Trifluoressigsäuresalz wurde die Mutterlauge eingeengt und mittels präparativer HPLC aufgereinigt. Die produktenthaltenden Fraktionen wurden vereinigt, vom Acetonitril befreit und gefriergetrocknet. Es wurden 23 mg der gewünschten Verbindung als Trifluoressigsäuresalz erhalten.

| | |
|---|---|
| LCMS-Rt: 1,30 min | [M+H⁺]: 394,2 |

### Beispiel 6a und 6b

1-(3,5-Di-tert-butyl-4-hydroxy-phenyl)-2-(3-imino-7-trifluormethyl-imidazo[1,5-a]pyridin-2-yl)-ethanon als Trifluoressigsäuresalz (6a) oder Hydrobromid (6b) 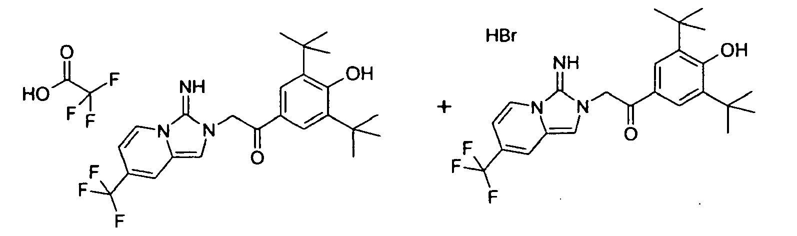
6a) 7-Trifluormethyl-imidazo[1,5-a]pyridin-3-ytamin 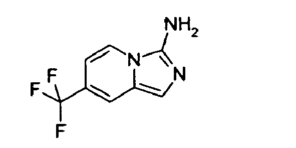 C-(4-Trifluormethyl-pyridin-2yl)methylamin-hydrochlorid (600 mg) wurden in Wasser gelöst, mit gesättigter Natriumhydrogencarbonat-Lösung versetzt und dann drei Mal mit Essigester extrahiert. Die vereinigten Essigesterphasen wurden über Magnesiumsulfat getrocknet, filtriert und eingeengt. Es wurden 300 mg der freien Base erhalten. Diese wurde in abs. Toluol (15 ml) gelöst und Bromcyan (191 mg, gelöst in 5 ml Toluol) langsam zugetropft. Nach 24 Stunden wurde der gebildete Niederschlag abgesaugt, getrocknet und mittels präparativer HPLC gereinigt. Die produktenthaltenden Fraktionen wurden vereinigt, vom Acetonitril befreit und gefriergetrocknet. Es wurden 168 mg der gewünschten Verbindung alsTrifluoressigsäuresalz erhalten.

| | | |
|---|---|---|
| | LCMS-Rt: 0,76 min | [M+H⁺]: 202,1 |

6b) 1-(3,5-Di-tert-butyl-4-hydroxy-phenyl)-2-(3-imino-7-trifluoromethyl-imidazo[1,5-a]pyridin-2-yl)-ethanon als Trifluoressigsäuresalz und Hydrobromid Entsprechend Beispiel 5b) wurde 7-Trifluormethyl-imidazo[1,5-a]pyridin-3-ylamin (25 mg) umgesetzt. Es wurden 11,6 mg des Hydrobromids und nach Chromatographie 9 mg des Trifluoressigsäure-Derivats erhalten.

| | |
|---|---|
| TFA-Salz: LCMS-Rt: 1,38 min | [M+H⁺]: 448,2 |
| HBr-Salz: LCMS-Rt: 1,38 min | [M+H⁺]: 448,2 |

### Beispiel 7

2-[2-(3,5-Di-tert-butyl-4-hydroxy-phenyl)-2-oxo-ethyl]-3-imino-2,3-dihydro-imidazo[1,5-a]pyridine-6-carbonsäuremethylester als Trifluoressigsäuresalz 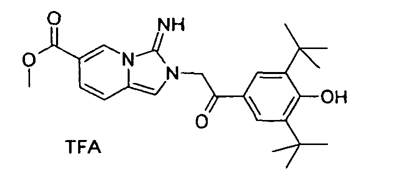
7a) 6-Aminomethyl-nicotinsäure-Trifluoracetat 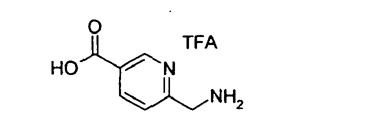 6-Cyano-nicotinsäure als Trifluoracetat (1 g) wurde in Methanol (300 ml) gelöst, mit Raney-Nickel (0,224 g) versetzt und in einem Schüttelautoklaven bei 80 °C 20 h unter 10 bar Wasserstoffdruck hydriert. Anschließend wurde das Gemisch filtriert, der Rückstand mit einem Wasser/Methanol-Gemisch gewaschen und das Filtrat eingeengt. Der Rückstand wurde mit ACN/Wasser aufgenommen und gefriergetrocknet. Es wurden 500 mg Produkt erhalten, die ausreichend sauber für den Einsatz in der nächsten Stufe waren.

| | | |
|---|---|---|
| | LCMS-Rt: 0,14 min | [M+H⁺]: 153,1 |

7b) 6-Aminomethyl-nicotinsäuremethylester als Trifluoressigsäuresalz 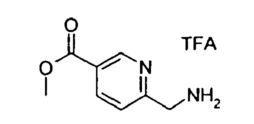 6-Aminomethyl-nicotinsäure-Trifluoracetat (500 mg) wurde in Methanol (45 ml) gelöst. Nach Zugabe von konzentrierter Schwefelsäure (10 Tropfen) wurde die Lösung 24 h rückflussiert. Nach Abkühlen wurde das Lösungsmittel abgezogen und der Rückstand in Wasser gelöst und mittels präparativer HPLC gereinigt. Die produktenthaltenden Fraktionen wurden vereinigt, vom ACN befreit und gefriergetrocknet. Es wurden 395 mg der gewünschten Verbindung als Trifluoressigsäuresalz erhalten. LCMS-Rt: 0,27 min [M+H⁺]: 167,1
7c) 3-Amino-imidazo[1,5-a]pyridin-6-carbonsäuremethylester 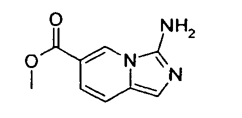 6-Aminomethyl-nicotinsäuremethylester-Trifluoracetat (390 mg) wurde in Toluol (35 ml) gelöst und mit Hünig-Base (230 µl) versetzt. Dann wurde Bromcyan (0,35 ml einer 5 M Lösung in Acetonitril) langsam zugetropft. Nach 3 h Rühren bei RT wurde das Lösungsmittel abgezogen und der Rückstand mit Wasser und gesättigter Natriumhydrogencarbonat-Lösung aufgenommen und drei Mal mit Essigester extrahiert. Die vereinigten organischen Phasen wurden über Natriumsulfat getrocknet, filtriert und eingeengt. Der Rückstand wurde mittels präparativer HPLC gereinigt. Die produktenthaltenden Fraktionen wurden vereinigt, vom Acetonitril befreit und gefriergetrocknet. Der Rückstand wurde dann mit wenig Wasser aufgenommen, mit gesättigter Natriumhydrogencarbonat-Lösung versetzt und drei Mal mit Essigester extrahiert. Die vereinigten organischen Phasen wurden über Natriumsulfat getrocknet, filtriert und eingeengt. Es wurden 110 mg der gewünschten Verbindung erhalten.

| | |
|---|---|
| LCMS-Rt: 0,67 min | [M+H⁺]: 192,1 |

7d) 2-[2-(3,5-Di-tert-butyl-4-hydroxy-phenyl)-2-oxo-ethyl]-3-imino-2,3-dihydro-imidazo[1,5-a]pyridin-6-carbonsäuremethylester als Trifluoressigsäuresalz 3-Amino-imidazo[1,5-a]pyridin-6-carbonsäuremethylester (26 mg) wurde in abs. DMF (2,5 ml) gelöst und 2-Brom-1-(3,5-di-tert-butyl-4-hydroxy-phenyl)-ethanon (45 mg, in 1,5 ml abs. DMF gelöst) langsam zugetropft. Nach 5 Stunden Rühren wurde das Reaktionsgemisch über Nacht stehen gelassen und dann zur Vervollständigung der Reaktion weiteres Bromid (10 mg, in 0,5 ml abs. DMF gelöst) zugegeben. Nach weiteren 2 h Rühren wurde das Lösungsmittel abgezogen und der Rückstand mittels präparativer HPLC gereinigt. Die produktenthaltenden Fraktionen wurden vereinigt, vom Acetonitril befreit und gefriergetrocknet. Es wurden 50 mg der gewünschten Verbindung als Trifluoressigsäuresalz erhalten.

| | |
|---|---|
| LCMS-Rt: 1,30 min | [M+H⁺]: 438,3 |

### Beispiel 8

2-[2-(3-tert-Butyl-4-methoxy-5-morpholin-4-yl-phenyl)-2-oxo-ethyl]-3-imino-2,3-dihydro-imidazo[1,5-a]pyridin-6-car6onsäuremethylester als Trifluoressigsäuresalz 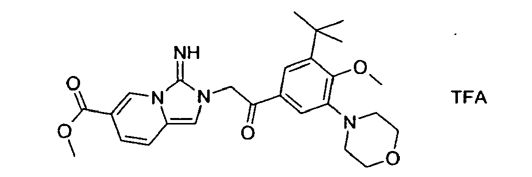 3-Amino-imidazo[1,5-a]pyridin-6-carbonsäuremethylester (20 mg) wurde in abs. DMF (1 ml) gelöst und 2-Brom-1-(3-tert-butyl-4-methoxy-5-morpholin-4-yl-phenyl)-ethanon (45 mg, in 1 ml abs. DMF gelöst; hergestellt nach WO 2004/078721) langsam zugetropft. Nach 4 h Rühren bei RT wurde noch eine Stunde auf 40 °C erwärmt. Das Reaktionsgemisch wurde über das Wochenende stehen gelassen und dann das Lösungsmittel im Hochvakuum abgezogen. Der Rückstand wurde mittels präparativer HPLC gereinigt. Die produktenthaltenden Fraktionen wurden vereinigt, vom Acetonitril befreit und gefriergetrocknet. Es wurden 40 mg der gewünschten Verbindung als Trifluoressigsäuresalz erhalten.

| | |
|---|---|
| LCMS-Rt: 1,27 min | [M+H⁺]: 481,2 |

### Beispiel 9

1-(3-tert-Butyl-4-methoxy-5-morpholin-4-yl-phenyl)-2-(3-imino-imidazo[1,5-a]pyridin-2-yl)-ethanon als Trifluoressigsäuresalz

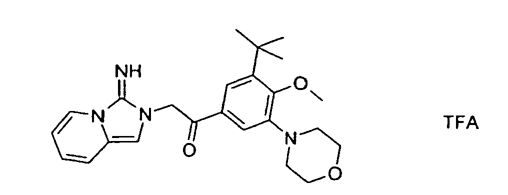

Imidazo[1,5-a]pyridin-3-ylamin als Trifluoressigsäuresalz (16 mg, Beispiel 1c) wurde in abs. DMF (1 ml) gelöst, mit Hünig-Base (31 µl) versetzt und 2-Brom-1-(3-tert-butyl-4-methoxy-5-morpholin-4-yl-phenyl)-ethanon (45 mg, in 1 ml abs. DMF gelöst; hergestellt nach WO 2004/ 078721) langsam zugetropft. Nach 2 h Rühren bei RT wurde über Nacht stehen gelassen und dann das Lösungsmittel im Hochvakuum abgezogen. Der Rückstand wurde mittels präparativer HPLC gereinigt. Die produktenthaltenden Fraktionen wurden vereinigt, vom Acetonitril befreit und gefriergetrocknet. Es wurden 12 mg der gewünschten Verbindung als Trifluoressigsäuresalz erhalten.

| | |
|---|---|
| | LCMS-Rt: 1,28 min [M+H⁺]: 423,3 |

### Beispiel 10

1-(3-tert-Butyl-4-methoxy-5-morpholin-4-yl-phenyl)-2-(3-imino-8-methyl-imidazo[1,5-a]pyridin-2-yl)-ethanon als Trifluoressigsäuresalz

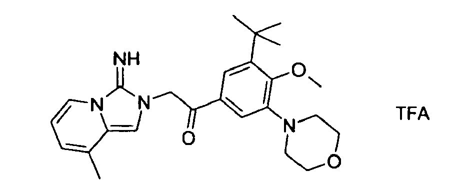

8-Methyl-imidazo[1,5-a]pyridin-3-ylamin als Trifluoressigsäuresalz (30 mg, Beispiel 5a) wurde in abs. DMF (2 ml) gelöst, mit Hünig-Base (19 µl) vesetzt und 2-Brom-1-(3-tert-butyl-4-methoxy-5-morpholin-4-yl-phenyl)-ethanon (43 mg, in 1 ml abs. DMF gelöst; hergestellt nach WO 2004/ 078721) langsam zugetropft. Nach 4,5 h wurde zur Vervollständigung der Reaktion weiteres Bromid (4 mg in 0,1 ml DMF) zugegeben. Nach weiteren 3 h Rühren wurde über Nacht stehen gelassen und dann das Lösungsmittel im Hochvakuum abgezogen. Der Rückstand wurde mittels präparativer HPLC gereinigt. Die produktenthaltenden Fraktionen wurden vereinigt, vom Acetonitril befreit und gefriergetrocknet. Es wurden 26 mg der gewünschten Verbindung als Trifluoressigsäuresalz erhalten.

| | |
|---|---|
| LCMS-Rt: 1,33 min | [M+H⁺]: 437,1 |

### Beispiel 11

1-(3-Dimethylamino-5-pentafluorsulfanyl-phenyl)-2-(3-imino-imidazo[1,5-a]pyridin-2-yl)-ethanon als Trifluoressigsäuresalz 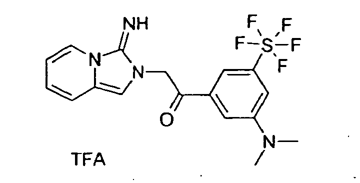
11a) 3-Amino-5-pentafluorsutfanyl-benzoesäure 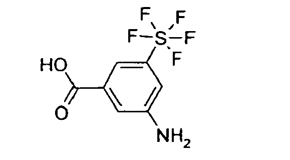 3-Pentafluorsulfanyl-benzoesäure (3,0 g) wurde in rauchender Salpetersäure (20 ml) gelöst und bei RT unter Feuchtigkeitsausschluss gerührt. Dann wurde konzentrierte Schwefelsäure (1,5 ml) zugegeben und bei 75 °C gerührt. Nach 6 h Rühren bei 75°C wurde über Nacht stehen gelassen, dann weitere Schwefelsäure (1,5 ml) zugegeben und 8 h auf 75°C erhitzt. Nach Stehenlassen über Nacht wurde auf Eiswasser gegeben und 2 h gerührt. Die einsetzende Kristallisation wurde über Nacht im Kühlschrank vervollständigt. Dann wurde der Niederschlag abgesaugt und am Hochvakuum getrocknet. Es wurden 2,7 g 3-Pentafluorsulfanyl-5-nitro-benzoesäure erhalten. Weitere 530 mg konnten aus der Mutterlauge nach dreimaligem Extrahieren mit Methylenchlorid, Trockenen der vereinigten Methylenchlorid-Phasen über Magnesiumsulfat und Einengen des Lösungsmittels erhalten werden.
Anschließend wurden die 2,7 g in Methanol (70 ml) gelöst, Raney- Nickel (etwa 500 mg) zugegeben und unter Wasserstoffatmosphäre (Wasserstoffballon) hydriert. Nach 2 h wurde der Katalysator abfiltriert und der Filterrückstand gut mit Methanol gewaschen. Das Filtrat wurde eingeengt und am Hochvakuum getrocknet. Es wurden 2,3 g Rohprodukt erhalten, das direkt in der nächsten Stufe umgesetzt wurde.

| | |
|---|---|
| LCMS-Rt: 1,21 min | [M+H⁺]: 264,0 |

11b) 3-Dimethylamino-5-pentafluorsulfanyl-benzoesäure 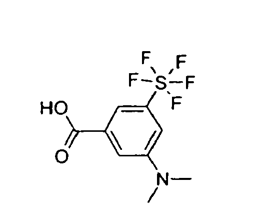 In zwei Mikrowellengefäßen wurden jeweils 3-Amino-5-pentafluorsulfanylbenzoe-säure (800 mg), Ameisensäure (12 ml) und 37 %ige Formalin-Lösung (8 ml) gegeben. Beide Gefäße wurden dann 30 min auf 110 °C erhitzt. Nach Abkühlen wurden die Lösungen vereinigt und auf Eiswasser gegeben. Nach dreimaliger Extraktion mit Essigester wurden die vereinigten organischen Phasen mit Magnesiumsulfat getrocknet, filtriert und eingeengt. Es wurden 1,76 g der Titelverbindung erhalten, die direkt in der nächsten Stufe umgesetzt wurden.

| | |
|---|---|
| LCMS-Rt: 1,48 min | [M+H⁺]: 292,0 |

11c) 3-Dimethylamino-N-methoxy-N-methyl-5-pentafluorsulfanyl-benzamid 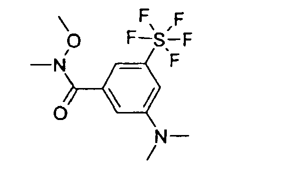 3-Dimethylamino-5-pentafluorsulfanyl-benzoesäure (1,0 g) wurde in Methylenchlorid (60 ml) gelöst. Unter Rühren wurde Thionylchlorid (5 ml) zugegeben und 2 h bei RT gerührt. Zur Vervollständigung der Reaktion wurde anschleißend 3 h auf Rückfluss erhitzt. Nach Abkühlen wurde das Lösungsmittel abgezogen, der Rückstand in Methylenchlorid (50 ml) gelöst, mit N,O-Dimethylhydroxylamin-Hydrochlorid versetzt und Hünig-Base (1 ml) zugegeben. Nach einer Stunde Rühren wurde das Lösungsmittel abgezogen, der Rückstand mit Essigester aufgenommen und 5 Mal mit Wasser gewaschen. Die organische Phase wurde mit Magnesiumsulfat getrocknet, filtriert und eingeengt. Es wurden 980 mg der Titelverbindung erhalten, die direkt in der nächsten Stufe umgesetzt wurden.

| | |
|---|---|
| LCMS-Rt: 1,53 min | [M+H⁺]: 335,0 |

11d) 1-(3-Dimethylamino-5-pentafluorsutfanyl-phenyl)-ethanon 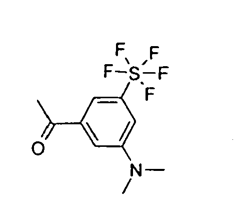 3-Dimethylamino-N-methoxy-N-methyl-5-pentafluorsulfanyl-benzamid (980 mg) wurde in abs. THF (50 ml) gelöst und bei 0 °C unter Rühren Methylmagnesium-bromid-Lösung (2,1 ml; 3 M Lösung in Diethylether) zugetropft. Nach beendeter Zugabe wurde das Eisbad entfernt und 1 h bei RT gerührt. Zur Vervollständigung der Reaktion wurde mehr Methylmagnesiumbromid-Lösung (0,3 ml) zugegeben und weitere 2 h gerührt. Nach Lagerung über Nacht im Kühlschrank wurde das Reaktionsgemisch unter Kühlung mit 1 N Salzsäure versetzt. Nach Zugabe von Wasser und Essigester wurde noch zwei Mal mit Essigester extrahiert, die vereinigten organischen Phasen mit Magnesiumsulfat getrocknet, filtriert und eingeengt. Der Rückstand wurde mittels Chromatographie an Kieselgel (n-Heptan/Essigester 100/0 auf 50/50 in 30 min) gereinigt. Die produktenthaltenden Fraktionen wurden vereinigt, das Lösungsmittel entfernt und der Rückstand im Hochvakuum getrocknet. Es wurden 650 mg der Titelverbindung erhalten.

| | |
|---|---|
| LCMS-Rt: 1,69 min | [M+H⁺]: 290,0 |

11e) [3-(1,1-Dimethoxy-ethyl)-5-pentafluosulfanyl-phenyl]-dimethyl-amin 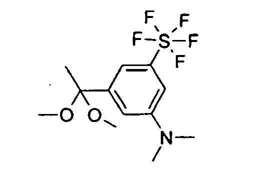 1-(3-Dimethylamino-5-pentafluorsulfanyl-phenyl)-ethanon (650 mg) wurde in Methanol (50 ml) gelöst und unter Rühren mit Trimethylorthoformiat (715 mg) und DL-10-Camphersulfonsäure (10 mg) versetzt. Nach 3 h Rühren wurden mehr Orthoformiat (200 mg) zugegeben, 2 h gerührt und dann über Nacht stehen gelassen. Dann wurde das Lösungsmittel abgezogen und der Rückstand im Hochvakuum getrocknet. Es wurden 730 mg Rohprodukt erhalten, die direkt in der nächsten Stufe umgesetzt wurden.
¹H-NMR (400 MHz, DMSO-d₆) [ppm]: 7,01 (1 H); 6,79 (1 H); 6,93 (1H); 3,10 (6 H); 2,98 (6 H); 1,47 (3 H)
11f) [3-(2-Brom-1,1-dimethoxy-ethyl)-5-pentafluorsulfanyl-phenyl]-dimethyl-amin und [3-(2-Brom-1,1-dimethoxy-ethyl)-5-pentafluorsulfanyl-phenyl]-methyl-amin 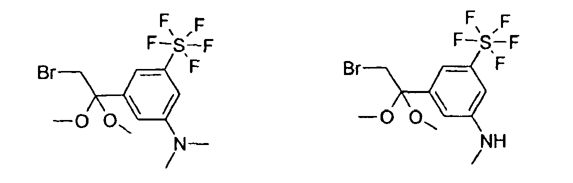 [3-(1,1-Dimethoxy-ethyl)-5-pentafluosulfanyl-phenyl]-dimethyl-amin (730 mg) wurde in einem Gemisch aus Methanol (15 ml) und THF (15 ml) gelöst. Unter Rühren wurde Phenyl-trimethyl-tribromid (818 mg) dazugegeben. Nach 3 h wurde zur Vervollständigung der Reaktion mehr Phenyl-trimethyl-tribromid (205 mg) zugegeben und 2 h bei 60°C gerührt. Nach Stehen über Nacht wurden Natriumthiosulfat-Lösung, Wasser und Essigester zugegeben. Die wässrige Phase wurde noch drei Mal mit Essigester extrahiert. Die vereinigten Extrakte wurden mit Magnesiumsulfat getrocknet, filtriert und eingeengt. Der Rückstand wurde mittels Chromatographie an Kieselgel (n-Heptan/Essigester 100/0 auf 50/50 in 30 min) gereinigt. Die produktent-haltenden Fraktionen wurden vereinigt, das Lösungsmittel entfernt und der Rückstand im Hochvakuum getrocknet.
Es wurden 490 mg der Dimethylamino- und 144 mg der Monomethylamino-Verbindung erhalten.
Dimethylamin-Derivat:
¹H-NMR (500 MHz, DMSO-d₆) [ppm]: 7,14 (1 H); 7,00 (1 H); 6,95 (1H); 3,85 (2 H); 3,14 (6 H); 2,99 (6 H)
Monomethylamin-Derivat:
¹H-NMR (500 MHz, DMSO-d₆) [ppm]: 7,02 (1 H); 6,91 (1 H); 6,84 (1H); 6,34 (1H); 3,80 (2 H); 3,14 (6 H); 2,71 (3 H)
11g) 2-Brom-1-(3-dimethylamino-5-pentafluorsulfanyl-phenyl)-ethanon 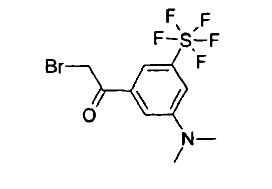 [3-(2-Brom-1,1-dimethoxy-ethyl)-5-pentafluorsulfanyl-phenyl]-dimethyl-amin (230 mg) wurde in Wasser (2,3 ml) suspendiert und dann unter Kühlung konzentrierte Schwefelsäure (2,3 ml) zugetropft. Nach 2 h Rühren bei RT wurde mit Wasser verdünnt (20 ml) und drei Mal mit Essigester extrahiert. Die vereinigten organischen Phasen wurden zwei Mal mit gesättigter Natriumhydrogencarbonat-Lösung gewaschen, über Magnesiumsulfat getrocknet, filtriert und eingeengt. Der Rückstand wurde in Acetonitril/Wasser gelöst, eingefroren und über Nacht gefriergetrocknet. Es wurden 170 mg der gewünschten Verbindung erhalten.

| | |
|---|---|
| LCMS-Rt: 1,80 min | [M+H⁺]: 367,9; 369,9 |

11h) 1-(3-Dimethylamino-5-pentafluorsulfanyl-phenyl)-2-(3-imino-imidazo[1,5-a]pyridin-2-yl)-ethanon als Trifluoressigsäuresalz Imidazo[1,5-a]pyridin-3-ylamin als Trifluoressigsäuresalz (20 mg, Beispiel 1 c) wurde in abs. THF (5 ml) gelöst und 2-Brom-1-(3-dimethylamino-5-pentafluorsulfanyl-phenyl)-ethanon (29 mg) und Hünig-Base (14 µl) zugegeben. Nach 6 h Rühren bei RT und Stehen lassen über Nacht wurde Wasser zugesetzt und drei Mal mit Essigester extrahiert. Die vereinigten organischen Phasen wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit und gefriergetrocknet. Es wurden 18 mg der gewünschten Verbindung als Trifluoressigsäuresalz erhalten. LCMS-Rt: 1,21 min [M+H⁺]: 421,0

### Beispiel 12

2-[2-(8-tert-Butyl-4-methyl-3,4-dihydro-2H-benzo[1,4]oxazin-6-yl)-2-oxo-ethyl]-7-ethoxy-3-imino=2,3-dihydro-imidazo[1,5-a]pyridin-6-carbonsäure-methylamid als Trifluoressigsäuresalz 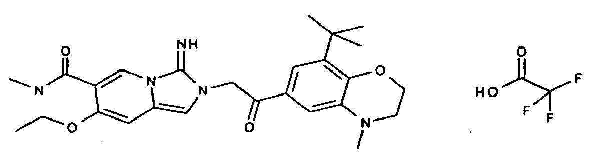
12a) 4-Chlor-6,N-dimethyl-nicotinamid 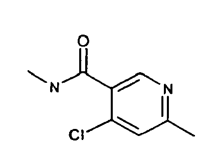 4-Hydroxy-6-methylnicotinsäure (5 g) wurde in abs. DCM (30 ml) gelöst und mit 10 Tropfen DMF versetzt. In dieses Gemisch wurde vorsichtig Oxalylchlorid (15 ml) getropft. Nach vier Stunden Rühren und Stehen lassen über Nacht wurde das Lösungsmittel abgezogen und zur weiteren Trocknung ans Hochvakuum gehängt. Das erhaltene 4-Chlor-6-methyl-nicotinoyl-chlorid (8 g) wurde in abs. DCM (160 ml) gelöst. Nach Abkühlen auf 0 °C wurde eine 40 %-ige methanolische Methylamin-Lösung (66 ml) innerhalb von 15 min. zugetropft. Nach Entfernen der Kühlung wurde noch 2 h bei RT nachgerührt. Nach Zugabe von Wasser (150 ml) wurde mit DCM extrahiert (drei Mal). Die vereinigten organischen Phasen wurden über Natriumsulfat getrocknet, filtriert und eingeengt. Es wurden 4,4 g der gewünschten Verbindung erhalten.

| | |
|---|---|
| LCMS-Rt: 0,34 min | [M+H⁺]: 185,1 |

12b) 4-Ethoxy-6,N-dimethyl-nicotinamid 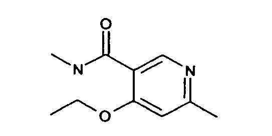 4-Chlor-6,N-dimethyl-nicotinamid (2,4 g) wurde unter Rühren in abs. Ethanol (40 ml) gelöst und mit Natriumethylat (1,8 g) versetzt. Nach 4 h Rühren bei RT wurde über Nacht stehen gelassen. Nach Zugabe von Wasser wurde 3 Mal mit DCM extrahiert. Die vereinigten organischen Phasen wurden über Natriumsulfat getrocknet, filtriert und eingeengt. Es wurden 2,2 g der gewünschten Verbindung erhalten.

| | |
|---|---|
| LCMS-Rt: 0,32 min | [M+H⁺]: 195,1 |

12c) 4-Ethoxy-6,N-dimethyl-1-oxy-nicotinamid 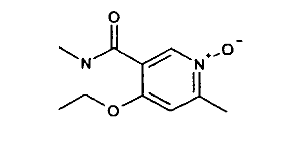 4-Ethoxy-6,N-dimethyl-nicotinamid (2,2 g) wurde in Chloroform (160 ml) gelöst, und unter Eiskühlung und Rühren portionsweise mit meta-Chlorperbenzoesäure (2,7 g ) versetzt. Nach Entfernen des Eisbads wurde 4 h nachgerührt und über Nacht stehen gelassen. Nach Zugabe 5-%iger Natriumcarbonat-Lösung wurde drei Mal mit Chloroform extrahiert. Die vereinigten organischen Phasen wurden über Natriumsulfat getrocknet, filtriert und eingeengt, so dass 979 mg Rohprodukt erhalten wurden. Dieses wurde über Kieselgel chromatographiert (DCM/MeOH-Gradient). Die produktenthaltenden Fraktionen wurden vereinigt und zur Trockne gebracht. Es wurden 717 mg der gesuchten Verbindung erhalten.
Die wässrige Phase wurde über Nacht gefriergetrocknet und der Rückstand mit DCM verrührt. Nach Filtration wurde zur Trockne gebracht. Der Rückstand entsprach weiteren 720 mg des gewünschten Produkts. LCMS-Rt: 0,52 min [M+H⁺]: 211,1
12d) 4-Ethoxy-6-hydroxymethyl-N-methyl-nicotinamid als Trifluoressigsäuresalz 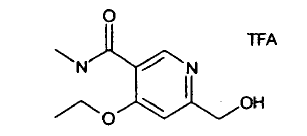 4-Ethoxy-6,N-dimethyl-1-oxy-nicotinamid (720 mg) wurde in abs. DCM (45 ml) unter Rühren gelöst und mit Trifluoressigsäureanhydrid (4,8 ml) versetzt. Nach Rühren bei RT wurde über Nacht stehen gelassen und anschließend 75 % des Lösungsmittels abgezogen. Der Rückstand wurde mit gesättigter Natriumchlorid-Lösung versetzt und mit Kaliumcarbonat auf pH 9 gestellt. Nach Extrahieren mit DCM (drei Mal) wurde über Natriumsulfat getrocknet, filtriert und eingeengt. Das Rohprodukt wurde mittel präparativer HPLC gereinigt. Die produktenthaltenden Fraktionen wurden vereinigt, vom Acetonitril befreit und gefriergetrocknet. Es wurden 696 mg der gewünschten Verbindung erhalten.

| | | |
|---|---|---|
| | LCMS-Rt: 0,25 min | [M+H⁺]: 211,1 |

12e) Methansulfonsäure-4-ethoxy-5-methylcarbamoyl-pyridin-2-ylmethyl-ester 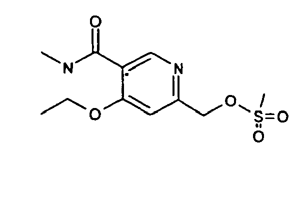 4-Ethoxy-6-hydroxymethyl-N-methyl-nicotinamid (696 mg als Trifluoressigsäuresalz) wurde in wenig DCM aufgenommen, mit gesättigter Kochsalzlösung versetzt und mit festem Kaliumcarbonat auf pH 9 gestellt. Nach Extraktion mit DCM (drei Mal) wurden die vereinigten organischen Phasen über Natriumsulfat getrocknet, filtriert und eingeengt. Es wurden 445 mg der TFA-freien Verbindung erhalten. Diese wurden in absoluten CM (30 ml) gelöst und nacheinander mit Methansulfonsäureanhydrid (1,3 g in 5 ml abs. DCM gelöst) und Triethylamin (1,44 ml) versetzt. Nach 2 h wurden Wasser und gesättigte Kochsalzlösung zugegeben. Nach Extraktion mit DCM (3 Mal) wurden die vereinigten organischen Phasen über Natriumsulfat getrocknet, filtriert und eingeengt. Zur weitestgehenden Entfernung des Triethylamins wurde der Rückstand in Gegenwart von 10-%iger Zitronensäure erneut extrahiert. Es wurden 485 mg der gewünschten Verbindung erhalten. LCMS-Rt: 0,72 min [M+H⁺]: 289,0
12f) 6-Aminomethyl-4-ethoxy-N-methyl-nicotinamid 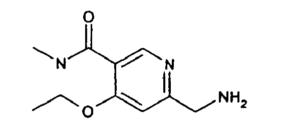 Bei RT und unter Rühren wurde innerhalb von 10 min Methansulfonsäure-4-ethoxy-5-methylcarbamoyl-pyridin-2-ylmethyl-ester (436 mg), in abs. MeOH (7 ml) gelöst, zu einer methanolischen Ammoniaklösung(7,1 ml; 7N) in einem Mikrowellengefäß getropft. Nach Stehen über Nacht wurde das Lösungsmittel abgezogen und der Rückstand mittels präparativer HPLC gereinigt. Die produktenthaltenden Fraktionen wurden vereinigt, vom Acetonitril befreit und gefriergetrocknet. Das Produkt wurde mit gesättigter Natriumchlorid-Lösung versetzt und mit Kaliumcarbonat alkalisch gestellt. Nach Extraktion mit Methylenchlorid würden die vereinigten organischen Phasen mit Natriumsulfat getrocknet, filtriert und eingeengt. Es wurden 71 mg der Titelverbindung erhalten.
LCMS-Rt: 0,27 min [M+H⁺]: 210,1
12g) 3-Amino-7-ethoxy-imidazo[1,5-a]pyridin-6-carbonsäure-methylamid als Trifluoressigsäuresalz 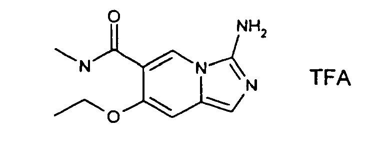 6-Aminomethyl-4-ethoxy-N-methyl-nicotinamid (68 mg) wurde in abs. Toluol (10 ml) gelöst vorgelegt. Bei RT unter Rühren und Argonatmosphäre wurde dann tropfenweise mit Bromcyan (48 mg), gelöst in abs. Toluol (1 ml), versetzt. Nach vier h Rühren bei RT wurde das Lösungsmittel unter verminderten Druck entfernt, der Rückstand mit Methylenchlorid verrührt und das Unlösliche abfiltriert. Das Filtrat wurde zur Trockne gebracht und anschließend mittels präparativer HPLC gereinigt Der Filterrückstand wurde erneut mit Methylenchlorid extrahiert und der nach Filtration erhaltene Rückstand ebenfalls mittels präparativer HPLC gereinigt. Die produktenthaltenden Fraktionen wurden vereinigt und das Acetonitril unter verminderten Druck abgezogen. Nach Gefriertrocknung wurden 15 mg der Titelverbindung neben 21 mg Ausgangsmaterial erhalten.

| | |
|---|---|
| LCMS-Rt: 0,69 min | [M+H⁺]: 235,0 |

12h) 2-Brom-1-(8-tert-butyl-4-methyl-3,4-dihydro-2H-benzo[1,4]oxazin-6-yl)-ethanon 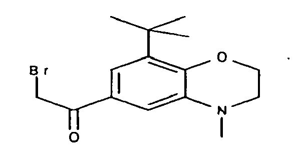 1-(8-tert-Butyl-4-methyl-3,4-dihydro-2H-benzo[1,4]oxazin-6-yl)-ethanon (250 mg, gekauft von Chembiotek, Indien) wurde in einer Mischung aus Essigsäure (4 ml) und Toluol (8 ml) auf 50 °C bis 55°C erwärmt. Bei dieser Temperatur wurde vorsichtig Brom (200 mg in Essigsäure gelöst) zugetropft. Nach 2,5 h wurde die Heizung entfernt, bei RT mit Eiswasser versetzt und drei Mal mit Toluol extrahiert. Die vereinigten organischen Phasen wurden über Natriumsulfat getrocknet, filtriert und eingeengt. Das Rohprodukt wurde über Kieselgel gereinigt, so dass 65 mg der gewünschten Verbindung erhalten wurden, neben weiteren 43 mg Produkt, die leicht verunreinigt waren und 37 mg Edukt.

| | |
|---|---|
| LCMS-Rt: 1,81 min | [M+H⁺]: 326,0; 328,0 |

12i) 2-[2-(8-tert-Butyl-4-methyl-3,4-dihydro-2H-benzo[1,4]oxazin-6-yl)-2-oxo-ethyl]-7-ethoxy-3-imino-2,3-dihydro-imidazo[1,5-a]pyridin-6-carbonsäure-methylamid als Trifluoressigsäuresalz
3-Amino-7-ethoxy-imidazo[1,5-a]pyridin-6-carbonsäure-methylamid als Trifluoressigsäuresalz (12 mg) wurde in abs. DMF (3 ml) unter Rühren und Argon mit Hünig-Base (6,3 µl) versetzt. Anschließend wurde 2-Brom-1-(8-tert-butyl-4-methyl-3,4-dihydro-2H-benzo[1,4]oxazin-6-yl)-ethanon (12,5 mg) gelöst in abs. DMF (0,5 ml) zu der Lösung getropft. Nach 2,5 h Rühren bei RT wurde 30 min auf 40 °C erwärmt und anschließend über Nacht stehen gelassen. Dann wurde das Lösungsmittel im Hochvakuum entfernt und der Rückstand mittels präparativer HPLC gereinigt. Die produktenthaltenden Fraktionen wurden vereinigt und das Acetonitril unter vermindertem Druck abgezogen. Nach Gefriertrocknung wurden 10 mg der Titelverbindung erhalten. LCMS-Rt: 1,24 min [M+H⁺]: 480,2

### Beispiel 13

2-[2-(3-tert-Butyl-4-methoxy-5-morpholin-4-yl-phenyl)-2-oxo-ethyl]-7-chlor-3-imino-2,3-dihydro-imidazo[1,5-a]pyridin-6-carbonsäure-methylamid als Trifluoressigsäuresalz 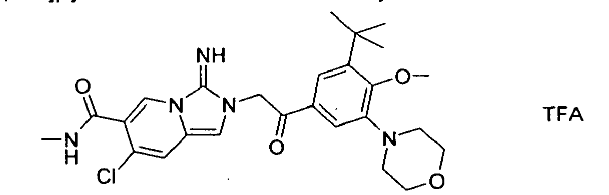
13a) 3-Amino-7-chlor-imidazo[1,5-a]pyridin-6-carbonsäuremethylamid als Trifluoressigsäuresalz 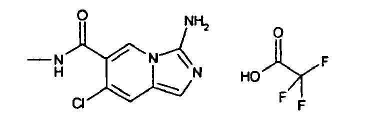 3-Amino-7-chlor-imidazo[1,5-a]pyridin-6-carbonsäuremethylamid wurde analog zu Beispiel 12 a-g) synthetisiert, wobei der Schritt 12b) ausgelassen wurde. Es wurden 9 mg der Titelverbindung erhalten. LCMS-Rt: 0,32 min [M+H⁺]: 225,0
13b) 2-[2-(3-tert-Butyl-4-methoxy-5-morpholin-4-yl-phenyl)-2-oxo-ethyl]-7-chlor-3-imino-2,3-dihydro-imidazo[1,5-a]pyridin-6-carbonsäure-methylamid als TrifluoressigsäuresalzAnalog wie in Beispiel 12 beschrieben, wurde 3-Amino-7-chlor-imidazo[1,5-a]pyridin-6-carbonsäuremethylamid (9 mg als TFA-Salz) mit 2-Brom-1-(3-tert-butyl-4-methoxy-5-morpholin-4-yl-phenyl)-ethanon (10 mg, hergestellt nach WO 2004/078721) in Gegenwart von Hünig-Base gekoppelt. Es wurden 4 mg der Titelverbindung erhalten. LCMS-Rt: 1,24 min [M+H⁺]: 514,2

### Beispiel 14

2,2,2-Trifluor-N-{3-[2-(3-imino-imidazo[1,5-a]pyridin-2-yl)-acetyl]-5- pentafluorsulfanyl-phenyl}-acetamid als Trifluoressigsäuresalz 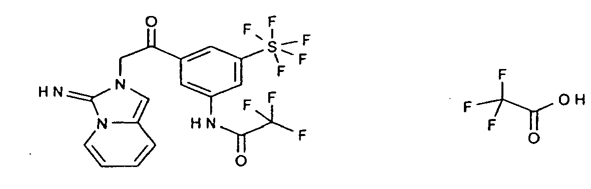
14a) 3-Amino-N-methoxy-N-methyl-5-pentafluorsulfanyl-benzamid 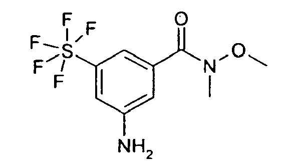 3-Amino-5-pentafluorsulfanyl-benzoesäure (200 mg, Beispiel 11a) wurde in Methylenchlorid (12 ml) gelöst. Unter Rühren wurden nacheinander zugegeben: N,O-Dimethylhydroxylamin x HCl (148 mg), 1-Propanphosphorsäureanhydrid (242 mg) und Triethylamin (292 µl). Die klare Lösung wurde 4 h bei RT gerührt und übers Wochenende stehen gelassen. Zur Aufarbeitung wurde das Reaktionsgemisch eingeengt, der Rückstand in Essigester aufgenommen und zwei Mal mit Kaliumhydrogensulfat-Lösung extrahiert. Nachdem anschließend die Essigester-Phase zwei Mal mit Natriumcarbonat-Lösung extrahiert worden war, wurden sie über Magnesiumsulfat getrocknet, filtriert und eingeengt. Das erhaltene Rohprodukt wurde mittels präparativer Chromatographie aufgereinigt. Die produktenthaltenden Fraktionen wurden vereinigt, vom Acetonitril befreit, mit gesättigter Natrium-hydrogencarbonat-Lösung basisch gestellt und mit Essigester drei Mal extrahiert. Die vereinigten Extrakte wurden über Magnesiumsulfat getrocknet, filtriert und eingeengt. Es wurden 77 mg der gewünschten Verbindung erhalten.
LCMS-Rt: 1,30 min [M+H⁺]: 307,0
14b) N-Methoxy-N-methyl-5-pentafluorsulfanyl-3-(2,2,2-trifluor-acetylamino)-benzamid 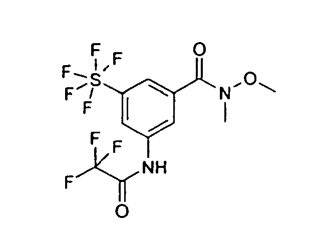 3-Amino-N-methoxy-N-methyl-5-pentafluorsulfanyl-benzamid (77 mg) wurde in Methylenchlorid (3 ml) gelöst und unter Rühren Triethylamin (42 µl) gefolgt von Trifluoressigsäureanhydrid (45 µl) unter Feuchtigkeitsausschluss zugesetzt. Nach 3 h Rühren bei RT und Stehen über Nacht wurden Wasser und gesättigte Natriumhydrogencarbonat-Lösung zugegeben, die Phasen getrennt und die MethylenchloridPhase noch drei Mal mit Wasser gewaschen, über Magnesiumsulfat getrocknet, filtriert und eingeengt. Das erhaltene Produkt (86 mg) wurde ohne weitere Reinigung in der nächsten Stufe eingesetzt. LCMS-Rt: 1,53 min [M+H⁺]: 403,0
14c) N-(3-Acetyl-5-pentafluorsulfanyl-phenyl)-2,2,2-trifluor-acetamid 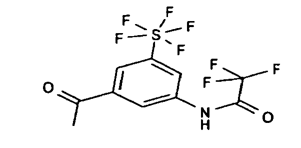 N-Methoxy-N-methyl-5-pentafluorsulfanyl-3-(2,2,2-trifluor-acetylamino)-benzamid (20 mg) wurde in absolutem THF (2 ml) gelöst und bei RT mit Lithiumhexmethyldi-silazan-Lösung (45 µl, 1 M in THF) 30 min gerührt. Bei 0°C wurde dann unter Rühren Methylmagnesiumbromid-Lösung (17 µl, 3 M in Diethylether) zugetropft. Nach 1 h Rühren bei RT wurde unter Kühlung 1 N Salzsäure zugetropft, gefolgt von Wasser und Essigester. Die organische Phase wurde abgetrennt und die Wasserphase noch zwei Mal mit Essigester extrahiert. Die vereinigten Essigester-phasen wurden über Magnesiumsulfat getrocknet, filtriert und eingeengt. Es wurden 17 mg Rohprodukt erhalten. Nachdem weiteres N-Methoxy-N-methyl-5-pentafluorsulfanyl-3-(2,2,2-trifluoracetylamino)-benzamid (60 mg) in beschriebener Weise umgesetzt wurde (erhaltenes Rohprodukt: 60 mg), wurden die Rohprodukte vereinigt und mittels präparativer Chromatographie aufgereinigt. Die produktent-haltenden Fraktionen wurden vereinigt, vom Acetonitril befreit, mit gesättigter Natriumhydrogencarbonat-Lösung basisch gestellt und mit Essigester drei Mal extrahiert. Die vereinigten Extrakte wurden über Magnesiumsulfat getrocknet, filtriert und eingeengt. Es wurden 37 mg der gewünschten Verbindung erhalten.

| | |
|---|---|
| LCMS-Rt: 1,61 min | [M+H⁺]: 358,0 |

14d) N-[3-(2-Brom-acetyl)-5-pentafluorsulfanyl-phenyl]-2,2,2-trifluor-acetamid 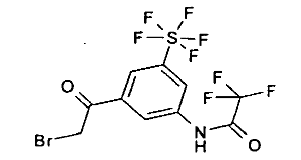 N-(3-Acetyl-5-pentafluorsulfanyl-phenyl)-2,2,2-trifluor-acetamid (20 mg) wurde in Eisessig (1 ml) gelöst vorgelegt und Brom (9 mg) gelöst in Eisessig (90 µl) langsam zugetropft. Nach 30 min Rühren bei RT wurde 1 h auf 60 °C erwärmt. Nach Kühlen auf RT wurde der Eisessig mit viel Wasser verdünnt und drei Mal mit Essigester extrahiert. Die vereinigten Extrakte wurden mit Natriumhydrogencarbonat-Lösung säurefrei gewaschen, über Magnesiumsulfat getrocknet, filtriert und eingeengt. Das erhaltene Rohrodukt (24 mg) wurde ohne weitere Reinigung in der nächsten Stufe eingesetzt. ¹H-NMR (400 MHz, CDCl₃) [ppm]: 8,45 [1H], 8,35 [1H], 8,25 [1H], 4,45 [s, 2H]
14e) 2,2,2-Trifluor-N-{3-[2-(3-imino-imidazo[1,5-a]pyridin-2-yl)-acetyl]-5- pentafluorsulfanyl-phenyl}-acetamid als Trifluoressigsäuresalz Imidazo[1,5-a]pyridin-3-ylamin-Trifluoracetat (20 mg, Beispiel 1c) wurde in absolutem THF (4 ml) gelöst und unter Rühren N-[3-(2-Brom-acetyl)-5-pentafluor-sulfanyl-phenyl]-2,2,2-trifluor-acetamid (26 mg) zugegeben. Nach Zugabe von DIPEA (10 µl) wurde 1 h bei RT gerührt und über Nacht stehen gelassen. Danach wurde das Reaktionsgemisch mit Wasser versetzt, drei Mal mit Essigester extrahiert und die vereinigten Essigester-Phasen mit Magnesiumsulfat getrocknet, filtriert und eingeengt. Der ölige Rückstand wurde mittels präparativer Chromatographie aufgereinigt. Die produktenthaltenden Fraktionen wurden vereinigt, vom Acetonitril befreit und gefriergetrocknet. Es wurden 7 mg der gewünschten Verbindung erhalten. LCMS-Rt: 1,22 min [M+H⁺]: 489,0

### Beispiel 15

1-(3-Brom-4-methoxy-5-trifluormethyl-phenyl)-2-(3-imino-imidazo[1,5-a]pyridin-2-yl)-ethanon als Trifluoressigsäuresalz 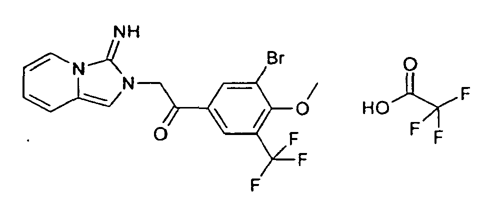
15a) 2-Brom-1-(3-brom-4-methoxy-5-trifluormethyl-phenyl)-ethanon 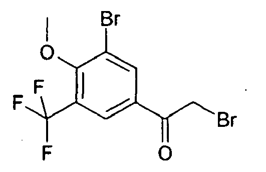 Ein Gemisch aus Wasser (0,4 ml) und konzentrierter Schwefelsäure (0,4 ml) wurde vorgelegt und bei RT 4-Methoxy-3-trifluormethyl-acetophenon (100 mg) zugegeben. Anschließend wurde unter Rühren Kaliumbromat (77 mg) portionsweise eintragen. Nach 4 h Rühren wurde das Reaktionsgemisch über Nacht in den Tiefkühlschrank gestellt. Danach wurde mit Wasser und Essigester versetzt und die wässrige Phase drei Mal mit Essigester extrahiert. Die vereinigten Essigesterphasen wurden über Magnesiumsulfat getrocknet, filtriert und eingeengt. Das Rohprodukt wurde über Kieselgel (n-Heptan/Essigester-Gradient) vorgereinigt und anschließend mittels präparativer Chromatographie endgereinigt. Die produktenthaltenden Fraktionen wurden vereinigt, vom Acetonitril befreit und mit Essigester drei Mal extrahiert. Die vereinigten Extrakte wurden über Magnesiumsulfat getrocknet, filtriert und eingeengt. Es wurden 12 mg der gewünschten Verbindung erhalten.
   LCMS-Rt: 1,74 min [M+H⁺]: 374,8 (50 %), 376,9 (100 %), 378,8 (45 %)
15b) 1-(3-Brom-4-methoxy-5-trifluormethyl-phenyl)-2-(3-imino-imidazo[1,5-a]pyridin-2-yl)- ethanon als Trifluoressigsäuresalz
   Imidazo[1,5-a]pyridin-3-ylamin-Trifluoracetat (8 mg, Beispiel 1c) wurde in absolutem DMF (0,5 ml) zusammen mit Hünig-Base (5,5 µl) bei RT vorgelegt. 2-Brom-1-(3-brom-4-methoxy-5-trifluormethyl-phenyl)-ethanon (12 mg, in 0,5 ml absolutem DMF gelöst) wurde unter Rühren zugetropft. Nach 3,5 h Rühren bei RT wurde das Lösungsmittel abgezogen und der Rückstand mittels präparativer Chromatographie aufgereinigt. Die produktenthaltenden Fraktionen wurden vereinigt, vom Acetonitril befreit und gefriergetrocknet. Es wurden 4 mg der gewünschten Verbindung erhalten.
   LCMS-Rt: 1,21 min [M+H⁺]: 428,0;430,0

### Beispiel 16

2-(3-Imino-imidazo[1,5-a]pyridin-2-yl)-1-(4-methoxy-3-morpholin-4-yl-5-trifluormethylphenyl)-ethanon als Trifluoressigsäuresalz 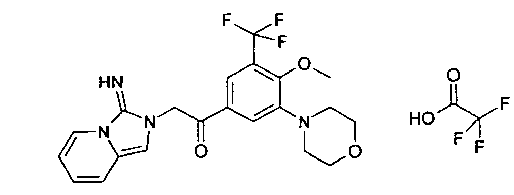
16a) 1-(3-Brom-4-hydroxy-5-trifluormethylphenyl)-ethanon 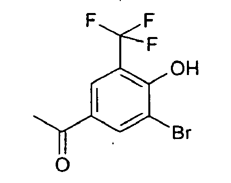 4-Hydroxy-3-(trifluormethyl)-acetophenon (4 g) wurde in Acetonitril (150 ml) vorgelegt und bei -10°C N-Bromsuccinimid (4,5 g, in 100 ml Acetonitril gelöst) zugetropft. Nach beendeter Zugabe wurde das Kältebad entfernt und 5 h nachgerührt. Nach Stehen über Nacht wurde das Lösungsmittelvolumen auf ein Viertel reduziert und der Rückstand mit n-Heptan/Wasser versetzt. Nach Abtrennen der organischen Phase wurde diese ein Mal mit 5 %-iger Natriumthiosulfat-Lösung und ein Mal mit Wasser gewaschen. Der dabei gebildete Niederschlag (6,9 g) wurde abgesaugt und direkt in der nächsten Stufe weiter umgesetzt.
¹H-NMR (500 MHz, DMSO-d₆) [ppm]: 11,3 [br, 1H], 8,37 [1H], 8,06 [1H], 2,57 [s, 3H]
16b) 1-Brom-5-(1,1-dimethoxy-ethyl)-2-methoxy-3-trifluormethyl-benzol 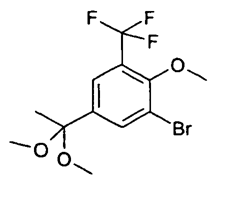 1-(3-Brom-4-hydroxy-5-trifluormethyl-phenyl)-ethanon (6,8 g) wurde in absolutem Methanol (50 ml) gelöst und nacheinander mit DL-10-Camphersulfonsäure (111 mg) und Trimethylorthoformat (8 ml) versetzt. Nach 2 h Rühren bei RT wurden DMF (75 ml) und Kaliumcarbonat (5,0 g) zugegeben, gefolgt von lodmethan (3 ml). Nach 6 h Rühren wurde der Ansatz über Nacht stehen gelassen und mit einem 1:1-Gemisch aus n-Heptan/Wasser versetzt. Nach Abtrennen der organischen Phase wurde noch ein Mal mit n-Heptan extrahiert und dann die vereinigten Heptan-Phasen über Magnesiumsulfat getrocknet, filtriert und eingeengt. Es wurden 7g des gewünschten Produkts erhalten.
¹H-NMR (500 MHz, DMSO-d₆) [ppm]: 7,92 [1H], 7,64 [1H], 3,90 [s, 3H], 3,09 [s, 6H], 1,50 [s, 3H]
16c) 4-[5-(1,1-Dimethoxy-ethyl)-2-methoxy-3-trifluormethyl-phenyl]-morpholin 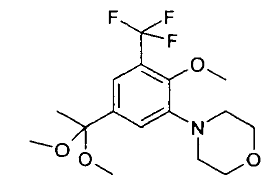 1-Brom-5-(1,1-dimethoxy-ethyl)-2-methoxy-3-trifluormethyl-benzol (2 g) wurde vorgelegt und unter Rühren 0,5 h Argon durchgeleitet. Anschließend wurde mit Pd(II)acetat (13 mg), BINAP (54 mg) und Natrium-tertiärbutylat (784 mg) versetzt, gefolgt von Morpholin (0,6 ml). Das erhaltene Gemisch wurde 8 h bei 85°C unter Argon gerührt und nach Stehen über Nacht bei RT wurde weitere 8 h bei 85°C gerührt und über Nacht stehen gelassen. Dann wurde das Lösungsmittel abgezogen und der Rückstand über Kieselgel (n-Heptan-Essigester-Gradient) gereinigt. Es wurden 468 mg des gewünschten Produkts erhalten.
¹H-NMR (500 MHz, DMSO-d₆) [ppm]: 7,26 [1H], 7,23 [1H], 3,88 [s, 3H], 3,78 [m, 4H], 3,10 [s, 6H], 3,06 [m, 4H], 1,47 [s, 3H]
16d) 2-Brom-1-(4-methoxy-3-morpholin-4-yl-5-trifluormethyl-phenyl)-ethanon 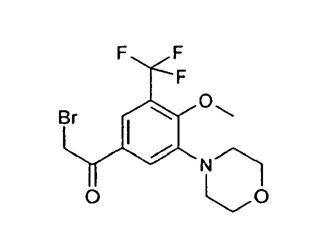 4-[5-(1,1-Dimethoxy-ethyl)-2-methoxy-3-trifluormethyl-phenyl]-morpholin (460 mg) wurde in absolutem THF (4 ml) und Methanol (1,6 ml) vorgelegt und unter Rühren bei 7 °C Phenyl-trimethyl-ammonium-bromid (530 mg) zugesetzt. Dann wurde das Kältebad entfernt und 8 h gerührt. Nach Stehen über Nacht wurde mit 5 %-iger Natriumthiosulfat-Lösung (0,8 ml) und Wasser (4 ml) versetzt. Die wässrige Phase wurde drei Mal mit Essigester extrahiert und die vereinigten Essigesterextrakte über Magnesiumsulfat getrocknet, filtriert und eingeengt. Der Rückstand wurde in Acetonitril (15 ml) gelöst und mit einem Gemisch (5 ml) aus Acetonitril (90 Teile), Wasser (10) und TFA (0,05) sowie weiterer TFA (0,5 ml) versetzt. Diese Mischung rührte etwa 4 h bei RT. Dann wurde das Lösungsmittel abgezogen und der Rückstand mit Wasser versetzt, mit gesättigter Natriumhydrogencarbonat-Lösung neutralisiert, drei Mal mit Essigester extrahiert und die vereinigten Essigesterphasen über Magnesiumsulfat getrocknet, filtriert und eingeengt. Der Rückstand wurde über Kieselgel gereinigt (n-Heptan-Essigester-Gradient). Nach dem Vereinigen der Produkt-enthaltenden Fraktionen wurde zur Trockne gebracht. Es wurden 200 mg der gewünschten Verbindung erhalten. LCMS-Rt: 1,67 min [M+H⁺]: 382,0; 384,0
16e) 2-(3-Imino-imidazo[1,5-a]pyridin-2-yl)-1-(4-methoxy-3-morpholin-4-yl-5-trifluormethyl-phenyl)-ethanon als Trifluoressigsäuresalz Imidazo[1,5-a]pyridin-3-ylamin-Trifluoracetat (30 mg, Beispiel 1c) wurde in absolutem DMF (4 ml) unter Rühren gelöst und DIPEA (20 µl) zugegeben. Nachdem 2-Brom-1-(4-methoxy-3-morpholin-4-yl-5-trifluormethyl-phenyl)-ethanon (51 mg, gelöst in 1 ml DMF) langsam innerhalb von 15 min zugetropft worden war, ließ man 3,5 h bei RT rühren. Nach Stehen über Nacht wurde das Lösungsmittel abgezogen und der Rückstand mittels präparativer Chromatographie aufgereinigt. Die produktenthaltenden Fraktionen wurden vereinigt, vom Acetonitril befreit und gefriergetrocknet. Es wurden 35 mg der gewünschten Verbindung erhalten.

| | |
|---|---|
| LCMS-Rt: 1,17 min | [M+H⁺]: 435,1 |

### Beispiel 17

6-Ethoxy-3-imino-2-[2-(3-methylamino-5-pentafluorsulfanyl-phenyl)-2-oxo-ethyl]-2,3-dihydro-1H-imidazo[1,5-a]-pyridin-5-on als Trifluoressigsäuresalz 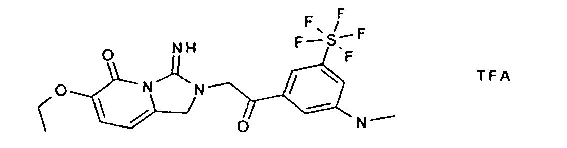
17a) 3-Ethoxy-2-fluor-6-methyl-pyridin 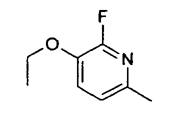 2-Fluor-3-hydroxy-6-piccolin (500 mg) wurde in absolutem DMF (20 ml) vorgelegt und Kaliumcarbonat (1 g) zugesetzt. Danach wurde unter Rühren Ethyliodid (0,6 ml) zugetropft und 1 h gerührt. Anschließend wurde dem Reaktionsgemisch Wasser und Essigester zugesetzt. Nach Abtrennen der organischen Phase wurde noch drei Mal mit Essigester extrahiert. Die vereinigten Extrakte wurden über Natriumsulfat getrocknet, filtriert und eingeengt. Das erhaltene Rohprodukt (477 mg) wurde direkt in der nächsten Stufe umgesetzt.
¹H-NMR (500 MHz, DMSO-d₆) [ppm]: 7,52 [dd, 1H], 7,10 [d, 1H], 4,10 [q, 2H], 2,32 [s, 3H], 1,33 [t, 3H]
17b) 6-Brommethyl-3-ethoxy-2-fluor-pyridin 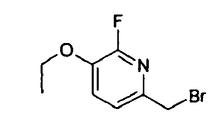 3-Ethoxy-2-fluor-6-methyl-pyridin (400 mg) wurde in Tetrachlorkohlenstoff (30 ml) vorgelegt und nach Zugabe von N-Bromsuccinimid (505 mg) und 2,2'-Azobis-(2-methylpropionitril) (85 mg) wurde das Reaktionsgemisch 7 h auf Rückfluss erhitzt. Nach Stehen über Nacht bei RT wurde mit Wasser versetzt und drei Mal mit Dichlormethan extrahiert. Die vereinigten organischen Phasen wurden über Natriumsulfat getrocknet, filtriert und eingeengt. Der Rückstand wurde über Kieselgel gereinigt (n-Heptan-Essigester-Gradient). Nach dem Vereinigen der Produkt-enthaltenden Fraktionen wurde zur Trockne gebracht. Es wurden 393 mg der gewünschten Verbindung erhalten. LCMS-Rt: 1,40 min [M+H⁺]: 233,9; 235,9
17c) C-(5-Ethoxy-6-fluor-pyridin-2-yl)-methylamin als Trifluoressigsäuresalz 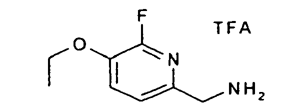 6-Brommethyl-3-ethoxy-2-fluor-pyridin (390 mg) wurde in Chloroform (50 ml) gelöst und unter Rühren mit Hexamethylentetramin (234 mg) versetzt. Nach 1 h Rühren bei 50 °C wurde das Lösungsmittel entfernt und der Rückstand mit absolutem Ethanol (35 ml) aufgenommen. Nach Zugabe von konzentrierter Salzsäure (1 ml) wurde 3 h bei 50 °C gerührt und dann über Nacht bei RT stehen gelassen. Nach Abziehen des Lösungsmittels wurde der Rückstand mit Wasser aufgenommen und gefriergetrocknet. Ein Teil des Rohprodukts wurde mittels präparativer Chromatographie aufgereinigt. Die produktenthaltenden Fraktionen wurden vereinigt, vom Acetonitril befreit und gefriergetrocknet. Es wurden 134 mg der gewünschten Verbindung erhalten. LCMS-Rt: 0,48 min [M+H⁺]: 171,1
17d) 3-Amino-6-ethoxy-1H-imidazo[1,5-a]pyridin-5-on als Trifluoressigsäuresalz 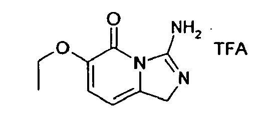 C-(5-Ethoxy-6-fluor-pyridin-2-yl)-methylamin-Trifluoracetat (134 mg) wurde mit wenig Wasser aufgenommen, mit 1,5 ml gesättigter Natriumchlorid-Lösung versetzt, mit Kaliumcarbonat (47 mg) alkalisch gestellt und dann die wässrige Phase drei Mal mit DCM extrahiert. Die vereinigten organischen Phasen wurden über Natriumsulfat getrocknet, filtriert und eingeengt. Der Rückstand wurde mit absolutem Toluol (8 ml) aufgenommen und unter Rühren gelöst. Dann wurde tropfenweise mit einer Bromcyan-Lösung (0,18 ml, 5 M in Acetonitril) versetzt. Nach 1,5 h wurde das Lösungsmittel entfernt und der Rückstand mittels präparativer Chromatographie aufgereinigt. Die produktenthaltenden Fraktionen wurden vereinigt, vom Acetonitril befreit und gefriergetrocknet. Die Gefriertrocknung mit Wasser in Gegenwart von TFA (0,05%) wurde drei Mal wiederholt. Danach wurden 55 mg der gewünschten Verbindung erhalten. LCMS-Rt: 0,31 min [M+H⁺]: 194,1
17e) 2-Brom-1-(3-methylamino-5-pentafluorsulfanyl-phenyl)-ethanon 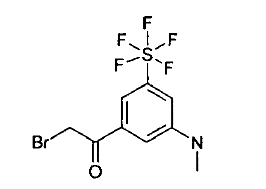 [3-(2-Brom-1,1-dimethoxy-ethyl)-5-pentafluorsulfanyl-phenyl]-methyl-amin (400 mg, hergestellt, wie in Beispiel 11f) beschrieben) wurde in Wasser (4 ml) suspendiert und dann unter Rühren und Kühlung konzentrierte Schwefelsäure (4 ml) zugetropft. Nach 4 h Rühren bei RT wurde der Ansatz auf Eiswasser gegossen und mit gesättigter Natriumhydrogencarbonat-Lösung vorsichtig auf pH 8 gestellt. Dann wurde 3 Mal mit Essigester extrahiert und die vereinigten Extrakte über Magnesium-sulfat getrocknet, filtriert und eingeengt. Es wurden 312 mg Rohprodukt erhalten. 100 mg davon wurden mittels präparativer Chromatographie aufgereinigt. Die produktenthaltenden Fraktionen wurden vereinigt, vom Acetonitril befreit, mit gesättigter Natrium-hydrogencarbonat-Lösung basisch gestellt und mit Essigester drei Mal extrahiert. Die vereinigten Extrakte wurden über Magnesiumsulfat getrocknet, filtriert und eingeengt. Es wurden 59 mg der gewünschten Verbindung erhalten.

| | |
|---|---|
| LCMS-Rt: 1,67 min | [M+H⁺]: 353,9; 355,9 |

17f) 6-Ethoxy-3-imino-2-[2-(3-methylamino-5-pentaflursulfanyl-phenyl)-2-oxo-ethyl]-2,3-dihydro-1H-imidazo[1,5-a]-pyridin-5-on
3-Amino-6-ethoxy-1H-imidazo[1,5-a]pyridin-5-on-Trifluoressigsäuresalz (25 mg) wurde in wenig Wasser gelöst und mit gesättigter Natriumhydrogencarbonat-Lösung alkalisch gestellt. Die wässrige Phase wurde drei Mal mit Essigester extrahiert und die vereinigten Extrakte über Natriumsulfat getrocknet, filtriert und eingeengt. Es wurden 14 mg der freien Base erhalten, die in DMF (2,5 ml) gelöst wurde. Zu dieser Lösung wurde unter Rühren 2-Brom-1-(3-methylamino-5-pentafluorsulfanyl-phenyl)-ethanon (26 mg, gelöst in 0,5 ml DMF) innerhalb von 5 min getropft. Es wurde 3 h bei RT gerührt und über Nacht stehen gelassen. Dann wurde das Lösungsmittel entfernt und das Rohprodukt mittels präparativer Chromatographie aufgereinigt. Die produktenthaltenden Fraktionen wurden vereinigt, vom Acetonitril befreit und gefriergetrocknet. Es wurden 17 mg der gewünschten Verbindung erhalten.

| | |
|---|---|
| LCMS-Rt: 1,18 min | [M+H⁺]: 467,0 |

### Beispiel 18

2-[2-(3-tert-Butyl-4-methoxy-5-morpholin-4-yl-phenyl)-2-oxo-ethyl]-6-ethoxy-3-imino-2,3-dihydro-1H-imidazo[1,5-a]pyridin-5-on als Trifluoressigsäuresalz 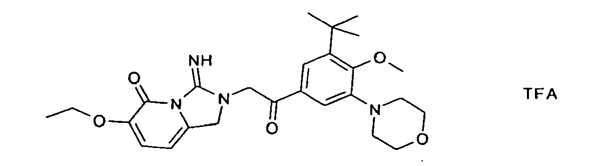 Analog zu Beispiel 17) wurde 3-Amino-6-ethoxy-1H-imidazo[1,5-a]pyridin-5-on-Trifluoressigsäuresalz (25 mg, Beispiel 17) mit 2-Brom-1-(3-tert-butyl-4-methoxy-5-morpholin-4-yl-phenyl-ethanon (30 mg, Beispiel 10) umgesetzt.
Es wurden 19 mg der Titelverbindung erhalten. LCMS-Rt: 1,27 min [M+H⁺]: 483,2

### Beispiel 19

2-[2-(3-tert-Butyl-4-methoxy-5-morpholin-4-yl-phenyl)-2-oxo-ethyl]-7-ethoxy-3-imino-2,3-dihydro-imidazo[1,5-a]pyridin-6-carbonsäure-methylamid als Trifluoressigsäuresalz 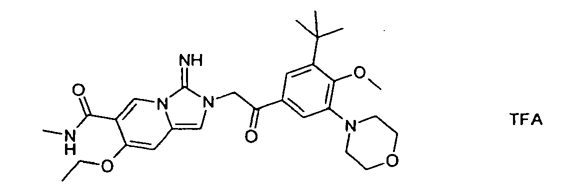
19a) N-(4-Ethoxy-5-methylcarbamoyl-pyridin-2-ylmethyl)-N'-(fluoren-9-ylmethyl-oxycarbonyl)-thioharnstoff 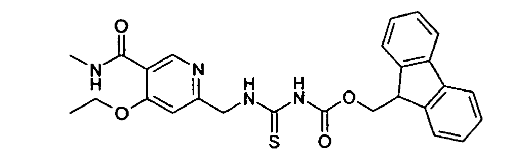 500 mg (2,39 mmol) 6-Aminomethyl-4-ethoxy-N-methyl-nicotinamid (Beispiel 12f) wurden in 25 ml Dioxan gelöst und bei RT mit 672,4 mg (2,39 mmol) Fluoren-9-ylmethyl-oxycarbonyl-isothiocyanat versetzt. Nach einer Stunde wurde vom Lösungsmittel befreit und der Rückstand in DCM aufgenommen. Es wurde dreimal mit wässriger LiCl-Lösung und einmal mit Wasser gewaschen. Nach Trocknen mit MgSO₄ wurde eingeengt und das so erhalten Rohprodukt (875 mg) ohne weitere Reinigung weiter umgesetzt. LCMS-Rt: 1,31 min [M+H⁺]: 491,2
19b) (7-Ethoxy-6-methylcarbamoyl-2H-imidazo[1,5-a]pyridin-3-yliden)-carbaminsäure-9H-fluoren-9-ylmethyl ester 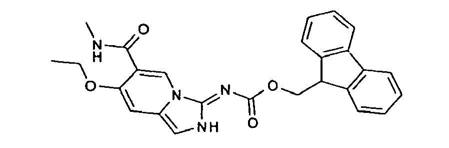 875 mg N-(4-Ethoxy-5-methylcarbamoyl-pyridin-2-ylmethyl)-N'-(fluoren-9-ylmethyl-oxycarbonyl)-thioharnstoff (Beispiel 19a, Rohprodukt) wurden in 25 ml Dioxan gelöst und bei RT mit 837 mg (1,96 mmol) Quecksilber(II)-trifluoracetat versetzt. Nach 10 Minuten wurde eingeengt und der Rückstand in DCM aufgenommen. Die organische Phase wurde dreimal mit 4%-iger LiCl-Lösung und einmal mit Wasser gewaschen, mit MgSO₄ getrocknet und einrotiert. Das so erhaltene Rohprodukt (590 mg) wurde ohne weitere Reinigung weiter umgesetzt.

| | |
|---|---|
| LCMS-Rt: 1,25 min | [M+H⁺]: 457,2 |

19c) 2-[2-(3-tert-Butyl-4-methoxy-5-morpholin-4-yl-phenyl)-2-oxo-ethyl]-7-ethoxy-3-imino-2,3-dihydro-imidazo[1,5-a]pyridin-6-carbonsäure-methylamid als Trifluoressigsäuresalz
590 mg (7-Ethoxy-6-methylcarbamoyl-2H-imidazo[1,5-a]pyridin-3-yliden)-carbaminsäure-9H-fluoren-9-ylmethylester (19b, Rohprodukt) wurden in 25 ml Dimethylacetamid gelöst und mit 573 mg (1,55 mmol) 2-Brom-1-(3-tert-butyl-4-methoxy-5-morpholin-4-yl-phenyl)-ethanon (hergestellt nach WO 2004/078721) versetzt. Die Lösung wurde drei Stunden bei 70 °C gerührt. Nach Stehen über Nacht bei RT wurde für zwei weitere Stunden bei 95 °C gerührt. Dann wurde das Lösungsmittel abgetrennt und das Rohprodukt wurde zweimal an einer präparativen HPLC gereinigt, wonach 63,5 mg der Titelverbindung isoliert werden konnten.

| | |
|---|---|
| LCMS-Rt (Methode B): 1,52 min | [M+H⁺]: 524,2 |

### Beispiel 20

2-[2-(3-tert-Butyl-4-methoxy-5-morpholin-4-yl-phenyl)-2-oxo-ethyl]-3-imino-5-methoxy-2,3-dihydro-imidazo[1,5-a]pyridin-7-carbonsäure-ethylester als Trifluoressigsäuresalz 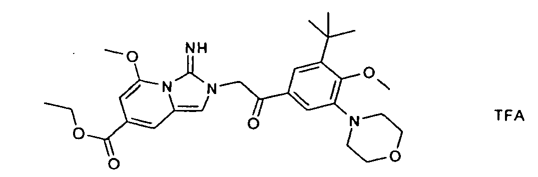
20a) 2-Hydroxy-6-methyl-isonicotinsäure-ethylester 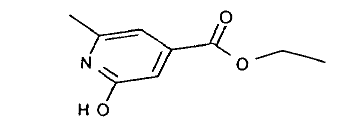 20,0 g (130,6 mmol) 2-Hydroxy-6-methyl-isonicotinsäure wurden in 200 ml Ethanol gelöst und mit 20 ml konzentrierter H₂SO₄ versetzt. Nachdem das Reaktionsgemisch für zwei Stunden refluxiert wurde, wurde vom Lösungsmittel befreit. Der Rückstand wurde in gesättigter NaHCO₃-Lösung aufgenommen und dreimal mit DCM extrahiert. Die vereinigten organischen wurden mit Na₂SO₄ getrocknet und eingeengt, wobei 20,1 g der Titelverbindung isoliert wurden.
LCMS-Rt: 0,82 min [M+H⁺]: 182,2
20b) 2-Methoxy-6-methyl-isonicotinsäure-ethylester 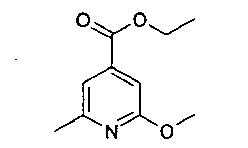 20,0 g (111,4 mmol) 2-Hydroxy-6-methyl-isonicotinsäure-ethylester (20a) wurden in 330 ml Toluol suspendiert. Nach Zugabe von 36,54 g (132,5 mmol) Ag₂CO₃ und 23,5 g (165,6 mmol) Methyliodid wurde unter KPG-Rühren auf 100 °C erhitzt. Nach 4 h Stunden ließ man das Reaktionsgemisch auf RT abkühlen und filtrierte es über Celite. Das Filtrat wurde zweimal mit Wasser gewaschen, mit Na₂SO₄ getrocknet und eingeengt, wobei 16,9 g der Titelverbindung als gelbliches Öl isoliert wurden.

| | |
|---|---|
| LCMS-Rt: 1,49 min | [M+H⁺]: 196,2. |

20c) 2-Bromomethyl-6-methoxy-isonicotinsäure-ethylester 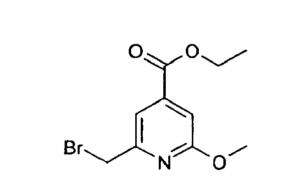 16,9 g (86,6 mmol) 2-Methoxy-6-methyl-isonicotinsäure-ethylester (19b) wurden in Tetrachlorkohlenstoff gelöst und nach Zugabe von 16,18 g (90,9 mmol) N-Bromsuccinimid und 0,28 g (1,73 mmol) AIBN zum Rückfluß erhitzt. Nach drei Stunden Rückfluss und Stehen über Nacht bei RT wurden weitere 7,7 g (43,3 mmol) N-Bromsuccinimid zugegeben und für drei Stunden refluxiert. Nach Abkühlen auf RT wurde mit DCM verdünnt, zweimal mit gesättigter NaHCO₃-Lösung und einmal mit Wasser gewaschen, mit MgSO₄ getrocknet und einrotiert. Der Rückstand wurde an einer präparativen HPLC gereinigt. Die Produktfraktionen wurden bei vermindertem Druck vom Acetonitril befreit und die wässrigen Lösungen mit DCM extrahiert. Die organische Phase wurde mit MgSO₄ getrocknet und einrotiert, wobei 10,95 g der Titelverbindung isoliert wurden.

| | |
|---|---|
| LCMS-Rt: 1,65 min | [M+H⁺]: 274,1 |

20d) 2-Aminomethyl-6-methoxy-isonicotinsäure-ethylester 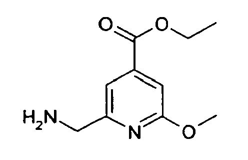 6,72 g (47,9 mmol) Hexamethylentetramin (Urotropin) wurden in 250 ml Chloroform vorgelegt und bei 0 °C eine Lösung von 10,95 g (39,95 mmol) 2-Brommethyl-6-methoxy-isonicotinsäure-ethylester (20c) in Chloroform zugetropft. Es wurde 4,5 Stunden bei RT gerührt und danach weitere 3,36 g (24,0 mmol) Hexamethylentetramin (Urotropin) zugegeben. Nach Stehen bei RT für weitere 60 Stunden wurde das Lösungsmittel unter verminderten Druck abdestilliert und der Rückstand in 500 ml Ethanol gelöst. Es wurden 50 ml konzentrierte HCl zugegeben und zwei Stunden bei RT gerührt. Nach Zugabe von weiteren 30 ml konzentrierte HCl und Rühren bei RT über Nacht wurde unter verminderten Druck vom Lösungsmittel befreit. Der Rückstand wurde in DCM aufgenommen und vorsichtig mit K₂CO₃ versetzt, bis keine Gasentwicklung mehr beobachtet wurde. Anschließend wurde zweimal mit gesättigter K₂CO₃-Lösung gewaschen, die organische Phase mit MgSO₄ getrocknet und eingeengt. Das so erhaltene Rohprodukt (7,60 g) wurde ohne weitere Reinigung weiter umgesetzt. LCMS-Rt: 0,74 min [M+H⁺]: 211,2
20e) N-(4-Ethoxycarbonyl-6-methoxy-pyridin-2-ylmethyl)-N'-(fluoren-9-ylmethyl-oxycarbonyl)-thioharnstoff 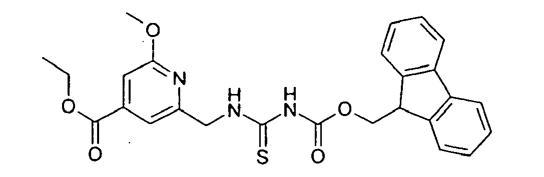 6,67 g (31,7 mmol) 2-Aminomethyl-6-methoxy-isonicotinsäure-ethylester (20d) wurden in 175 ml Toluol gelöst und bei RT mit 8,93 g (281,3 mmol) Fluoren-9-ylmethyl-oxycarbonyl-isothiocyanat versetzt. Nach 15 Minuten wurde der entstandene Niederschlag abfiltriert und bei 50 °C getrocknet, wobei 4,6 g der Titelverbindung isoliert wurden. Das Filtrat wurde eingeengt, der Rückstand in DCM aufgenommen und dreimal mit Wasser gewaschen. Die organische Phase wurde mit MgSO₄ getrocknet und eingeengt. Das so erhaltene Rohprodukt wurde an Kieselgel chromatographiert (Heptan/Ethylacetat 90:10 → 75:25), wobei weitere 8,1 g der Titelverbindung isoliert werden konnten.

| | |
|---|---|
| LCMS-Rt: 2,21 min | [M+H⁺]: 492,0 |

20f) 3-Imino-5-methoxy-2,3-dihydro-imidazo[1,5-a]pyridin-7-carbonsäure-ethylester 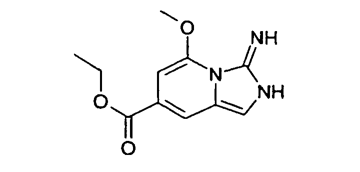 1,17 g (2,38 mmol) N-(4-Ethoxycarbonyl-6-methoxy-pyridin-2-ylmethyl)-N'-(fluoren-9-ylmethyl-oxycarbonyl)-thioharnstoff (290e) wurden in 25 ml Toluol mit 491 mg (2,38 mmol) DCC versetzt und für sechs Stunden refluxiert. Nach Stehen über Nacht bei RT wurde vom Lösungsmittel befreit und der Rückstand an Kieselgel chromato-graphiert (Heptan/Ethylacetat 75:25 → Ethylacetat/Methanol 90:10), wobei 88 mg der Titelverbindung isoliert werden konnten. LCMS-Rt: 0,81 min [M+H⁺]: 236,2 20g) 2-[2-(3-tert-Butyl-4-methoxy-5-morpholin-4-yl-phenyl)-2-oxo-ethyl]-3-imino-5-methoxy- 2,3-dihydro-imidazo[1,5-a]pyridin-7-carbonsäure-ethylester als Trifluoressigsäuresalz 88 mg (0,37 mmol) 3-Imino-5-methoxy-2,3-dihydro-imidazo[1,5-a]pyridin-7-carbonsäure-ethylester (20f) wurden in 4 ml Dimethylacetamid gelöst und mit einer Lösung von 138,5 mg (0,37 mmol) 2-Brom-1-(3-tert-butyl-4-methoxy-5-morpholin-4-yl-phenyl)-ethanon (hergestellt nach WO 2004/078721) in 2 ml Dimethylacetamid versetzt. Nach 24 Stunden bei RT wurde unter verminderten Druck vom Lösungsmittel befreit und der Rückstand an einer präparativen HPLC gereinigt, wobei 61 mg der Titelverbindung isoliert werden konnten.

| | |
|---|---|
| LCMS-Rt (Methode B): 1,66 min | [M+H⁺]: 525,3 |

### Pharmakologische Beispiele

PAR 1 Bestimmungsmethode: Hemmung der PAR1 mediierten Thrombozytenaggregation

Die pharmakologische Testung der Substanzen erfolgte in der durch TRAP (Thrombinrezeptor aktivierendes Peptid) induzierten Thrombozytenaggregation im 96-well Format. Dazu wurde von gesunden Freiwilligen Blut in 20 ml Spritzen abgenommen, in denen 2 ml 3,13 %-ige Natriumcitratlösung vorgelegt war. Nach einer 20-minütigen Zentrifugation bei 150 x g wurde das Plättchenreiche Plasma (PRP) abgetrennt und mit 1 µl PGE1-Lösung (500 µg/ml in Ethanol) / ml PRP versetzt. Nach 5 Minuten Inkubation bei RT wurde 15 Minuten bei 120 x g zentrifugiert um die Leukozyten zu entfernen. Das Leukozytenfreie PRP wurde in 5 ml Portionen in 15 ml PP Röhrchen überführt und 15 Minuten bei 360xg abzentrifugiert, um die Plättchen zu pelletieren. Anschließend wurde das Plasma dekantiert und das Plättchensediment aus 5 ml PRP in 1 ml Tyrode (120 mM NaCl, 2,6 mM KCl, 12 mM NaHCO₃, 0,39 mM NaH₂PO₄ x H₂O, 10 mM HEPES, 0,35% BSA, 5,5 mM Glukose, pH 7,4) resuspendiert und mit Tyrode auf eine Plättchenzahl von 3x10⁵ / Mikroliter (µL) eingestellt. 13 ml dieser Zellsuspension wurde dann mit 866 µL 10 mM CaCl₂-Lösung versetzt und 120 µL davon pro well einer 96-well-Platte pipettiert, in dem 15 µL der zu testenden Substanz vorgelegt waren. Nach 30 Minuten Inkubation bei RT im Dunklen wurden 15 µL einer TRAP-Lösung (70-100 µM) als Agonist zugegeben und in einem SpectraMax 340 bei 650 nm über 20 Minuten bei 37° C unter Schütteln eine Kinetik aufgezeichnet. Die Flächen unter den Kurven von Negativkontrolle (Tyrode/ DMSO) und Positivkontrolle (15 µl Agonist /DMSO) wurden berechnet und die Differenz als 100 % Wert festgelegt. Die zu testenden Substanzen wurden in Doppelbestimmung als Verdünnungsreihen pipettiert, ebenfalls die AUC jeder Substanzkonzentration bestimmt und die % Hemmung der AUC gegen die Kontrolle errechnet. Anhand der % Hemmung wurde mit Hilfe nichtlinearer Regressionsanalyse gemäß der 4 Parameter-Gleichung die IC50 berechnet.

Tabelle 2 zeigt die Ergebnisse.

**Tabelle 2:**

| Verbindung aus Beispiel | Hemmung der Thrombozytenaggregation IC₅₀ [mikro M] | Verbindung aus Beispiel | Hemmung der Thrombozytenaggregation IC₅₀ [mikro M] |
|---|---|---|---|
| 2 | 13 | 12 | 0,02 |
| 3 | 0,85 | 18 | 9,5 |
| 6b | 0,73 | 19 | 0,9 |
| 8 | 0,51 | 20 | 0,07 |

## Patentansprüche

1. Verbindung der Formel I 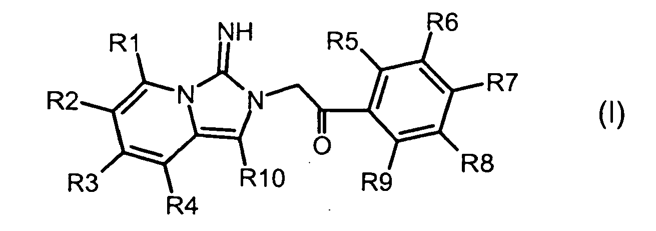 und/oder alle stereoisomeren oder tautomeren Formen der Verbindung der Formel I und/oder Gemische dieser Formen in jedem Verhältnis, und/oder ein physiologisch verträgliches Salz der Verbindung der Formel 1, wobei R1, R2, R3 und R4 gleich oder verschieden sind und unabhängig voneinander für
1) -(C₁-C₆)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist,
2) -O-(C₁-C₈)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist, wobei -(C₆-C₁₄)-Aryl und Het unsubstituiert oder zusätzlich ein-, zwei- oder dreifach durch R15 substituiert ist,
3) -(C₀-C₄)-Alkylen-C(O)-R11, wobei R11 für
3)1) Wasserstoffatom,
3)2) -(C₁-C₆)-Alkyl,
3)3) -(C₀-C₄)-Alkylen-(C₆-C₁₄)-Aryl,
3)4) -(C₀-C₄)-Alkylen-Het oder
3)5) -(C₀-C₄)-Alkylen-(C₃-C₆)-Cycloalkyl, steht,
4) -(C₀-C₄)-Alkylen-C(O)-O-R11, wobei R11 wie oben definiert ist,
5) -(C₀-C₄)-Alkylen-N(R12)-R13, wobei R12 und R13 gleich oder verschieden sind und unabhängig voneinander für
5)1) Wasserstoffatom,
5)2) -(C₁-C₆)-Alkyl,
5)3) -(C₁-C₃)-Fluoralkyl,
5)4) -(C₀-C₄)-Alkylen-(C₆-C₁₄)-Aryl,
5)5) -(C₀-C₄)-Alkylen-Het oder
5)6) -(C₀-C₄)-Alkylen-(C₃-C₆)-Cycloalkyl, stehen,
6) -(C₀-C₄)-Alkylen-C(O)-N(R12)-R13, wobei R12 und R13 gleich oder verschieden sind und unabhängig voneinander wie oben definiert sind,
7) -(C₀-C₄)-Alkylen-N(R12)-C(O)-R13, wobei R12 und R13 gleich oder verschieden sind und unabhängig voneinander wie oben definiert sind,
8) -(C₁-C₃)-Fluoralkyl,
9) -O-(C₁-C₃)-Fluoralkyl,
10) -SO₂-CH₃,
11) -SO₂-CF₃,
12) -NO₂,
13) -CN,
14) -OH,
15) =O,
16) Wasserstoffatom oder
17) Halogen, stehen
R10 und R15 gleich oder verschieden sind und unabhängig voneinander für
1) Wasserstoffatom,
2) -(C₁-C₄)-Alkyl,
3) -O-(C₁-C₄)-Alkyl,
4) -(C₁-C₃)-Fluoralkyl,
5) -O-(C₁-C₃)-Fluoralkyl,
6) -(C₀-C₄)-Alkylen-N(R16)(R17), worin R16 und R17 unabhängig voneinander Wasserstoffatom oder -(C₁-C₆)-Alkyl bedeuten,
7) -(C₀-C₄)-Alkylen-(C₆-C₁₄)-Aryl,
8) -(C₀-C₄)-Alkylen-(C₃-C₆)-Cycloalkyl,
9) -(C₀-C₄)-Alkylen-Het,
10) -OH,
11) =O,
12) -NO₂,
13) -CN,
14) Halogen,
15) -SO₂-(C₁-C₄)-Alkyl oder
16) -SO₂-(C₁-C₃)-Fluoralkyl, stehen,
R5, R6, R7, R8 und R9 gleich oder verschieden sind und unabhängig voneinander für
1) Wasserstoffatom,
2) -(C₀-C₄)-Alkylen-(C₆-C₁₄)-Aryl, wobei Aryl unsubstituiert oder ein-, zwei- oder dreifach unabhängig voneinander durch -(C₁-C₄)-Alkyl,
-(C₆-C₁₄)-Aryl, Het, -(C₃-C₆)-Cycloalkyl, Halogen, -NH₂, -OH oder Methoxy substituiert ist,
3) -(C₀-C₄)-Alkylen-(C₃-C₆)-Cycloalkyl,
4) -(C₀-C₄)-Alkylen-Het, wobei Het unsubstituiert oder ein-, zwei- oder dreifach unabhängig voneinander durch -(C₁-C₄)-Alkyl, -(C₆-C₁₄)-Aryl, Het, -(C₃-C₆)-Cycloalkyl, -C(O)-O-R16, -C(O)-N(R16)(R17), worin R16 und R17 wie oben definiert sind, Halogen, -NH₂, -OH oder Methoxy substituiert ist,
5) -SF₅,
6) -(C₁-C₆)-Alkyl, wobei Alkyl unsubstituiert oder ein-, zwei- oder dreifach unabhängig voneinander durch Halogen, -NH₂, -OH oder Methoxy substituiert ist,
7) -O-(C₁-C₈)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist,
8) -(C₀-C₄)-Alkylen-C(O)-R11, wobei R11 wie oben definiert ist,
9) -(C₀-C₄)-Alkylen-C(O)-O-R11, wobei R11 wie oben definiert ist,
10) -(C₀-C₄)-Alkylen-N(R12)-R13, wobei R12 und R13 gleich oder verschieden sind und unabhängig voneinander wie oben definiert sind,
11) -(C₀-C₄)-Alkylen-C(O)-N(R12)-R13, wobei R12 und R13 gleich oder verschieden sind und unabhängig voneinander wie oben definiert sind,
12) -(C₀-C₄)-Alkylen-N(R12)-C(O)-R13, wobei R12 und R13 gleich oder verschieden sind und unabhängig voneinander wie oben definiert sind,
13) -(C₁-C₃)-Fluoralkyl,
14) -O-(C₁-C₃)-Fluoralkyl,
15) -SO₂-CH₃,
16) -SO₂-CF₃,
17) -NO₂,
18) -CN,
19) -OH oder
20) Halogen, stehen oder
R5 und R6, R6 und R7, R7 und R8 oder R8 und R9 bilden zusammen mit den Ringatomen, an die sie gebunden sind, einen vier- bis achtgliedrigen Heterocyclus, der zusammen mit dem Phenylring, an den der Heterocyclus anneliert ist, ein bicyclisches System bildet, wobei der heterocyclische Teil unsubstituiert oder ein-, zwei- oder dreifach unabhängig voneinander durch - (C₁-C₄)-Alkyl, -(C₆-C₁₄)-Aryl, -(C₃-C₆)-Cycloalkyl, Halogen, -NH₂, -OH oder Methoxy substituiert ist, und
wobei "Het" Ringsysteme sind mit 4 bis 15 Kohlenstoff-atomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten.

2. Verbindung der Formel I gemäß Anspruch 1, wobei
R1, R2, R3 und R4 gleich oder verschieden sind und unabhängig voneinander für
1) -(C₁-C₆)-Alkyl, wobei Alkyl unsubstituiert oder ein-, zwei- oder dreifach unabhängig voneinander durch -(C₃-C₆)-Cycloalkyl, Halogen, -NH₂, -OH, Methoxy, -(C₆-C₁₄)-Aryl, oder Het, substituiert ist,
wobei Aryl ausgewählt ist aus der Gruppe Phenyl, Naphthyl, Anthryl und Fluorenyl und worin Aryl unsubstituiert oder ein-, zwei- oder dreifach durch R15 substituiert ist,
wobei Het ausgewählt ist aus der Gruppe Acridinyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrol, Thienopyridin, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl und Xanthenyl und worin Het unsubstituiert oder zusätzlich ein-, zwei- oder dreifach durch R15 substituiert ist,
2) -O-(C₁-C₆)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist, wobei Het und Aryl wie oben definiert sind,
3) -(C₀-C₄)-Alkylen-C(O)-R11, wobei R11 für
3)1) Wasserstoffatom, 3)2) -(C₁-C₆)-Alkyl, 3)3) -(C₀-C₄)-Alkylen-(C₆-C₁₄)-Aryl, wobei Aryl wie oben definiert ist,
3)4) -(C₀-C₄)-Alkylen-Het, wobei Het wie oben definiert ist, oder
3)5) -(C₀-C₄)-Alkylen-(C₃-C₆)-Cycloalkyl, steht,
4) -(C₀-C₄)-Alkylen-C(O)-O-R11, wobei R11 wie oben definiert ist,
5) -(C₀-C₄)-Alkylen-N(R12)-R13, wobei R12 und R13 gleich oder verschieden sind und unabhängig voneinander für
5)1) Wasserstoffatom,
5)2) -(C₁-C₆)-Alkyl,
5)3) -(C₁-C₃)-Fluoralkyl,
5)4) -(C₀-C₄)-Alkylen-(C₆-C₁₄)-Aryl, wobei Aryl wie oben definiert ist,
5)5) -(C₀-C₄)-Alkylen-Het, wobei Het wie oben definiert ist, oder
5)6) -(C₀-C₄)-Alkylen-(C₃-C₆)-Cycloalkyl, stehen,
6) -(C₀-C₄)-Alkylen-C(O)-N(R12)-R13, wobei R12 und R13 gleich oder verschieden sind und unabhängig voneinander wie oben definiert sind,
7) -(C₀-C₄)-Alkylen-N(R12)-C(O)-R13, wobei R12 und R13 gleich oder verschieden sind und unabhängig voneinander wie oben definiert sind,
8) -(C₁-C₃)-Fluoralkyl,
9) -O-(C₁-C₃)-Fluoralkyl,
10) -SO₂-CH₃,
11) -SO₂-CF₃,
12) -NO₂,
13) -CN,
14) -OH,
15) =O,
16) Wasserstoffatom oder
17) Halogen, stehen
R10 und R15 gleich oder verschieden sind und unabhängig voneinander für
1) Wasserstoffatom,
2) -(C₁-C₄)-Alkyl,
3) -O-(C₁-C₄)-Alkyl,
4) -(C₁-C₃)-Fluoralkyl,
5) -O-(C₁-C₃)-Fluoralkyl,
6) -(C₀-C₄)-Alkylen-N(R16)(R17), worin R16 und R17 unabhängig voneinander Wasserstoffatom oder -(C₁-C₆)-Alkyl bedeuten,
7) -(C₀-C₄)-Alkylen-(C₆-C₁₄)-Aryl, wobei Aryl wie oben definiert ist,
8) -(C₀-C₄)-Alkylen-(C₃-C₆)-Cycloalkyl,
9) -(C₀-C₄)-Alkylen-Het, wobei Het wie oben definiert ist,
10) -OH,
11) =O,
12) -NO₂,
13) -CN,
14) Halogen,
15) -SO₂-(C₁-C₄)-Alkyl oder
16) -SO₂-(C₁-C₃)-Fluoralkyl, stehen,
R5, R6, R7, R8 und R9 gleich oder verschieden sind und unabhängig voneinander für
1) Wasserstoffatom,
2) -(C₀-C₄)-Alkylen-(C₆-C₁₄)-Aryl, wobei Aryl unsubstituiert oder ein-, zwei- oder dreifach unabhängig voneinander durch -(C₁-C₄)-Alkyl, -(C₆-C₁₄)-Aryl, Het, -(C₃-C₆)-Cycloalkyl, Halogen, -NH₂, -OH oder Methoxy substituiert ist, wobei Aryl und Het wie oben definiert sind,
3) -(C₀-C₄)-Alkylen-(C₃-C₆)-Cycloalkyl,
4) -(C₀-C₄)-Alkylen-Het, wobei Het unsubstituiert oder ein-, zwei- oder dreifach unabhängig voneinander durch -(C₁-C₄)-Alkyl, -(C₆-C₁₄)-Aryl, Het, -(C₃-C₆)-Cycloalkyl, -C(O)-O-R16, -C(O)-N(R16)(R17), worin R16 und R17 wie oben definiert sind, Halogen, -NH₂, -OH oder Methoxy substituiert ist, wobei Aryl und Het wie oben definiert sind,
5) -SF₅,
6) -(C₁-C₆)-Alkyl, wobei Alkyl unsubstituiert oder ein-, zwei- oder dreifach unabhängig voneinander durch Halogen, -NH₂, -OH oder Methoxy substituiert ist,
7) -O-(C₁-C₈)-Alkyl, wobei Alkyl unsubstituiert oder ein-, zwei- oder dreifach unabhängig voneinander durch -(C₆-C₁₄)-Aryl, -(C₃-C₆)-Cycloalkyl, Het, Halogen, -NH₂, -OH oder Methoxy substituiert ist, wobei Aryl und Het wie oben definiert sind,
8) -(C₀-C₄)Alkylen-C(O)-R11, wobei R11 wie oben definiert ist,
9) -(C₀-C₄)-Alkylen-C(O)-O-R11, wobei R11 wie oben definiert ist,
10) -(C₀-C₄)-Alkylen-N(R12)-R13, wobei R12 und R13 gleich oder verschieden sind und unabhängig voneinander wie oben definiert sind,
11) -(C₀-C₄)-Alkylen-C(O)-N(R12)-R13, wobei R12 und R13 gleich oder verschieden sind und unabhängig voneinander wie oben definiert sind,
12) -(C₀-C₄)-Alkylen-N(R12)-C(O)-R13, wobei R12 und R13 gleich oder verschieden sind und unabhängig voneinander wie oben definiert sind,
13) -(C₁-C₃)-Fluoralkyl,
14) -O-(C₁-C₃)-Fluoralkyl,
15) -SO₂-CH₃,
16) -SO₂-CF₃,
17) -NO₂,
18) -CN,
19) -OH oder
20) Halogen, stehen oder
R5 und R6, R6 und R7, R7 und R8 oder R8 und R9 bilden zusammen mit den Ringatomen, an die sie gebunden sind, einen vier- bis achtgliedrigen Heterocyclus, der zusammen mit dem Phenylring, an den der Heterocyclus anneliert ist, ein bicyclisches System bildet, ausgewählt aus der Gruppe Benzimidazol, Benzisothiazol, Benzisoxazol, Benzo[1,3]dioxol, Benzofuranyl, Benzothiazol, Benzisoxazol, Benzothiofuran, Benzothiophen, Benzo[1,3]oxathiol, Benzoxazol, Benzthiazol, Benztriazolyl, Chinazolin, Chinazolon, Chinolin, 4H-Chinolizin, Chinoxalin, Chroman, Chromen, Cinnolin, 2,3-Dihydro-benzo[1,4]dioxin, 2,3-Dihydro-benzofuranyl, 1,3-Dihydro-isobenzofuran, 3,4-Dihydro-2H-benzo[1,4]oxazin, 2,3-Dihydro-benzooxazol, 2,3-Dihydro-benzothiazol, 1,3-Dihydro-benzo[c]thiophen, 2,3-Dihydro-benzo[b]thiophen, Indazol, Indol, Indolin, Isobenzofuran, Isochinolin, Isochroman, Isoindazol, Isoindol, Isoindolin, 7-Oxa-bicyclo[4.2.0]octa-1,3,5-trien, Phthalazin, 2,3,4,5-Tetrahydro-1H-benzo[b]azepin, 6,7,8,9-Tetrahydro-5-oxa-9-aza-benzocyclohepten, 3,4,5,6-Tetrahydro-2H-benzo[b][1,4]oxazozin, Tetrahydrochinolin, 1,2,3,4-Tetrahydro-chinoxalin oder Tetrahydroisochinolin,
wobei der heterocyclische Teil unsubstituiert oder ein-, zwei- oder dreifach unabhängig voneinander durch -(C₁-C₄)-Alkyl, -(C₆-C₁₄)-Aryl, -(C₃-C₆)-Cycloalkyl, Halogen, -NH₂, -OH oder Methoxy substituiert ist.

3. Verbindung der Formel I gemäß den Ansprüchen 1 oder 2, wobei R1, R2, R3 und R4 gleich oder verschieden sind und unabhängig voneinander für
1) Wasserstoffatom,
2) -(C₁-C₄)-Alkyl,
3) -O-(C₁-C₄)-Alkyl,
4) -(C₀-C₄)-Alkylen-C(O)-N(R12)-R13, wobei R12 und R13 gleich und verschieden sind und unabhängig voneinander Wasserstoffatom oder -(C₁-C₄)-Alkyl bedeuten,
5) -(C₁-C₃)-Fluoralkyl,
6) -(C₀-C₄)-Alkylen-C(O)-O-(C₁-C₄)-Alkyl oder
7) =O,
8) Halogen stehen,
R10 für 1) Wasserstoffatom,
2) -(C₁-C₄)-Alkyl,
3) -(C₀-C₄)-Alkylen-(C₃-C₆)-Cycloalkyl oder
4) -(C₀-C₄)-Alkylen-Phenyl, steht,
R5, R6, R7, R8 und R9 gleich oder verschieden sind und unabhängig voneinander für
1) Wasserstoffatom,
2) -(C₁-C₃)-Fluoralkyl,
3) Halogen,
4) -O-(C₁-C₄)-Alkyl,
5) -OH,
6) -(C₁-C₄)-Alkyl,
7) -SF₅,
8) -(C₀-C₄)-Alkylen-NH-C(O)-(C₁-C₃)-Fluoralkyl,
9) -(C₀-C₄)-Alkylen-N(R12)-R13, wobei R12 und R13 gleich und verschieden sind und unabhängig voneinander Wasserstoffatom oder -(C₁-C₄)-Alkyl bedeuten, oder
10) -(C₀-C₄)-Alkylen-Het, wobei Het ausgewählt ist aus der Gruppe Morpholinyl oder Pyrrolidinyl und unsubstituiert oder ein- oder zweifach unabhängig voneinander durch -(C₁-C₄)-Alkyl, =O oder -NH₂ substituiert ist, stehen oder
R5 und R6, R6 und R7, R7 und R8 oder R8 und R9 bilden zusammen mit den Ringatomen, an die sie gebunden sind, einen vier- bis achtgliedrigen Heterocyclus, der zusammen mit dem Phenylring, an den der Heterocyclus anneliert ist, ein bicyclisches System bildet, ausgewählt aus der Gruppe 2,3-Dihydro-benzo[1,4]dioxin, Benzo[1,3]dioxol, 3,4-Dihydro-2H-benzo[1,4]oxazin, 2,3,4,5-Tetrahydro-1H-benzo[b]azepin, Tetrahydrochinolin, Tetrahydroisochinolin, 1,2,3,4-Tetrahydro-chinoxalin oder 6,7,8,9-Tetrahydro-5-oxa-9-aza-benzocyclohepten, wobei der heterocyclische Teil unsubstituiert oder ein- oder zweifach durch -(C₁-C₄)-Alkyl oder Halogen substituiert ist.

4. Verbindung der Formel I gemäß einem oder mehreren der Ansprüche 1 bis 3, **dadurch gekennzeichnet, dass** es die Verbindung
1-(3,5-Di-tert-butyl-4-hydroxy-phenyl)-2-(3-imino-imidazo[1,5-a]pyridin-2-yl)-ethanon,
2-(3-Imino-imidazo[1,5-a]pyridin-2-yl)-1-(3-pentafluorsulfanyl-phenyl)-ethanon, 2-(1-Cyclopropyl-3-imino-imidazo[1,5-a]pyridin-2-yl)-1-(3,5-di-tert-butyl-4-hydroxy-phenyl)-ethanon,
1-(3,5-Di-tert-butyl-4-hydroxy-phenyl)-2-(3-imino-1-phenyl-imidazo[1,5-a]pyridin-2-yl)-ethanon,
1-(3,5-Di-tert-butyl-4-hydroxy-phenyl)-2-(3-imino-8-methyl-imidazo[1,5-a]pyridin-2-yl)-ethanon,
1-(3,5-Di-tert-butyl-4-hydroxy-phenyl)-2-(3-imino-8-methyl-imidazo[1,5-a]pyridin-2-yl)-ethanon als Hydrobromidsalz,
1-(3,5-Di-tert-butyl-4-hydroxy-phenyl)-2-(3-imino-7-trifluoromethyl-imidazo[1,5-a]pyridin-2-yl)-ethanon,
1-(3,5-Di-tert-butyl-4-hydroxy-phenyl)-2-(3-imino-7-trifluoromethyl-imidazo[1,5-a]pyridin-2-yl)-ethanon als Hydrobromidsalz,
2-[2-(3,5-Di-tert-butyl-4-hydroxy-phenyl)-2-oxo-ethyl]-3-imino-2,3-dihydro-imidazo[1,5-a]pyridine-6-carbonsäuremethylester,
2-[2-(3-tert-Butyl-4-methoxy-5-morpholin-4-yl-phenyl)-2-oxo-ethyl]-3-imino-2,3-dihydro-imidazo[1,5-a]pyridin-6-carbonsäuremethylester,
1-(3-tert-Butyl-4-methoxy-5-morpholin-4-yl-phenyl)-2-(3-imino-imidazo[1,5-a]pyridin-2-yl)-ethanon,
1-(3-tert-Butyl-4-methoxy-5-morpholin-4-yl-phenyl)-2-(3-imino-8-methyl-imidazo[1,5-a]pyridin-2-yl)-ethanon,
1-(3-Dimethylamino-5-pentafluorsulfanyl-phenyl)-2-(3-imino-imidazo[1,5-a]pyridin-2-yl)-ethanon,
2-[2-(8-tert-Butyl-4-methyl-3,4-dihydro-2H-benzo[1,4]oxazin-6-yl)-2-oxo-ethyl]-7-ethoxy-3-imino-2,3-dihydro-imidazo[1,5-a]pyridin-6-carbonsäuremethylamid,
2-[2-(3-tert-Butyl-4-methoxy-5-morpholin-4-yl-phenyl)-2-oxo-ethyl]-7-chlor-3-imino-2,3-dihydro-imidazo[1,5-a]pyridin-6-carbonsäure-methylamid,2,2,2-Trifluor-N-{3-[2-(3-imino-imidazo[1,5-a]pyridin-2-yl)-acetyl]-5- pentafluorsulfanyl-phenyl}-acetamid als Trifluoressigsäuresalz,
1-(3-Brom-4-methoxy-5-trifluormethyl-phenyl)-2-(3-imino-imidazo[1,5-a]pyridin-2-yl)- ethanon als Trifluoressigsäuresalz,
2-(3-Imino-imidazo[1,5-a]pyridin-2-yl)-1-(4-methoxy-3-morpholin-4-yl-5-trifluormethyl-phenyl)-ethanon als Trifluoressigsäuresalz,
6-Ethoxy-3-imino-2-[2-(3-methylamino-5-pentafluorsulfanyl-phenyl)-2-oxo-ethyl]-2,3-dihydro-1H-imidazo[1,5-a]-pyridin-5-on als Trifluoressigsäuresalz,
2-[2-(3-tert-Butyl-4-methoxy-5-morpholin-4-yl-phenyl)-2-oxo-ethyl]-6-ethoxy-3-imino-2,3-dihydro-1H-imidazo[1,5-a]pyridin-5-on als Trifluoressigsäuresalz,
2-[2-(3-tert-Butyl-4-methoxy-5-morpholin-4-yl-phenyl)-2-oxo-ethyl]-7-ethoxy-3-imino-2,3-dihydro-imidazo[1,5-a]pyridin-6-carbonsäure-methylamid als Trifluoressigsäure-salz oder
2-[2-(3-tert-Butyl-4-methoxy-5-morpholin-4-yl-phenyl)-2-oxo-ethyl]-3-imino-5-methoxy- 2,3-dihydro-imidazo[1,5-a]pyridin-7-carbonsäure-ethylester als Trifluoressigsäuresalz ist..

5. Arzneimittel, **gekennzeichnet durch** einen wirksamen Gehalt an mindestens einer Verbindung der Formel I gemäß einem oder mehreren der Ansprüche 1 bis 4 zusammen mit einem pharmazeutisch geeigneten und physiologisch verträglichen Trägerstoff, Zusatzstoff und/oder anderen Wirk- und Hilfsstoffen.

6. Verwendung der Verbindung der Formel I gemäß einem oder mehreren der Ansprüche 1 bis 4 zur Herstellung eines Arzneimittels zur Prophylaxe, Sekundärprevention und Therapie all solcher Erkrankungen, die die mit Thrombosen, Embolien, Hyperkoagulabilität oder fibrotischen Veränderungen einhergehen.

7. Verwendung gemäß Anspruch 6, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder Eingriffen, die zu einem Kontakt des Blutes mit Fremdoberflächen führen wie bei Dialysepatienten und Patienten mit Verweilkathetern oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung und/oder Behandlung von Narbenbildung.

8. Verfahren zur Herstellung der Verbindung der Formel I gemäß einem oder mehreren der Ansprüche 1 bis 4, **dadurch gekennzeichnet, dass** man
a) eine Verbindung der Formel II 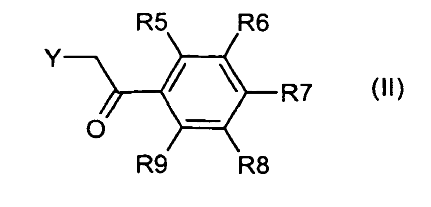 wobei die Reste R5, R6, R7, R8 und R9 wie in Formel I definiert sind und Y für Chlorid, Bromid, Mesylat oder Tosylat steht mit einer Verbindung der Formel III 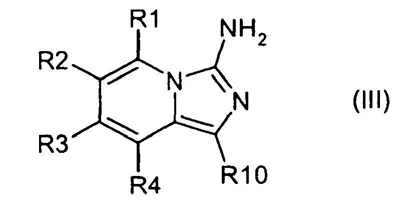 in Gegenwart einer Base und eines Lösungsmittels zu einer Verbindung der Formel I umsetzt, oder
b) eine Verbindung der Formel VII 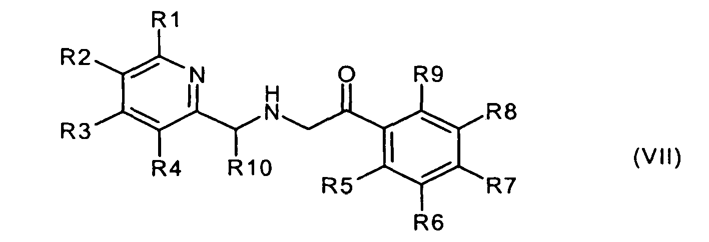 wobei die Reste R1 bis R10 wie in Formel I definiert sind mit einer Verbindung Z-CN, wobei Z Tosylat oder Bromid bedeutet, in Gegenwart einer Base zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

9. Verfahren zur Herstellung der Verbindung der Formel VII gemäß Anspruch 8, **dadurch gekennzeichnet, dass** man eine Verbindung der Formel VIII 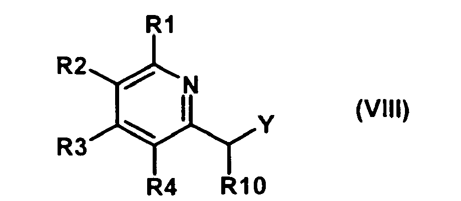 wobei die Reste R1, R2, R3, R4 und R10 wie in Formel I definiert sind und Y für Chlorid, Bromid, Mesylat oder Tosylat steht mit einer Verbindung der Formel IX 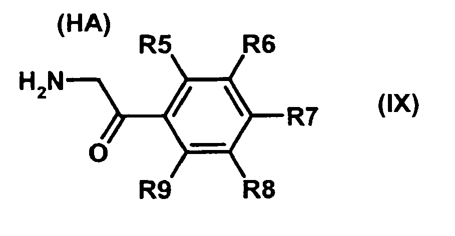 in Gegenwart einer Base und eines Lösungsmittels zu einer Verbindung der Formel I umsetzt.

10. Verfahren zur Herstellung der Verbindung der Formel II gemäß Anspruch 8,
wobei Y Br bedeutet und einer der Reste R5, R6, R7, R8 oder R9 für Pentafluorsulfanyl steht, **dadurch gekennzeichnet, dass** man
a) eine Verbindung der Formel Xa 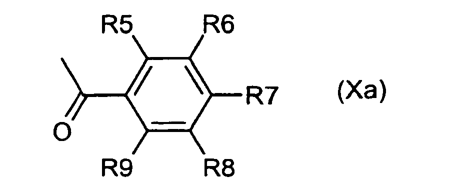 wobei einer der Reste R5, R6, R7, R8 oder R9 für Pentafluorsulfanyl steht und die anderen Reste R5, R6, R7, R8 und R9 wie in Formel I definiert sind mit einem Bromierungsreagent in die Verbindung der Formel II überführt, oder
b) eine Verbindung der Formel Xla 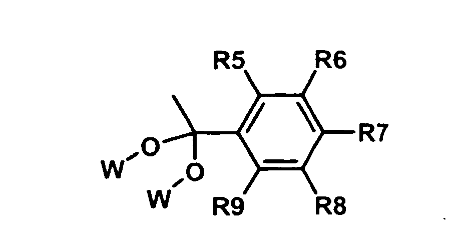
wobei einer der Reste R5, R6, R7, R8 oder R9 für Pentafluorsulfanyl steht und die anderen Reste R5, R6, R7, R8 und R9 wie in Formel I definiert sind, W für Ethylen, Propylen oder Butylen steht oder zusammen mit der Gruppe -O-C-O- einen 1,3- Dioxo-Ring der Ringgröße 5, 6 oder 7 bildet, mit einem Bromierungsreagent behandelt wird und anschließend in Gegenwart einer Säure in die Verbindung der Formel II überführt wird.

11. Verfahren zur Herstellung der Verbindung der Formel Xa gemäß Anspruch 10,
wobei R6 für Pentafluorsulfanyl und R8 für Dimethylamin steht und die Reste R5, R7 und R9 wie in Formel I definiert sind, **dadurch gekennzeichnet, dass** man
a) eine Verbindung der Formel XIVa 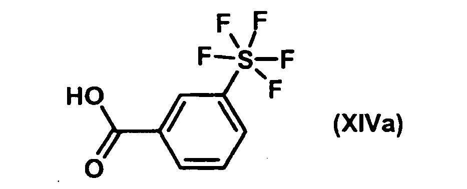 Zunächst nitriert und anschließend mit Wasserstoff zum Amin der Formel XIIIa reduziert, 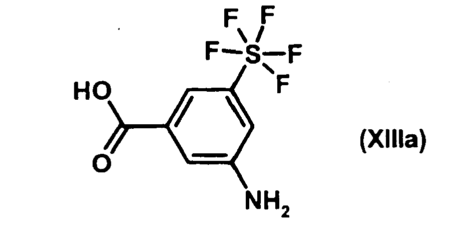
b) die erhaltene Verbindung der Formel XIIIa am Stickstoff dimethyliert und die Carbonsäure mit Thionylchlorid ins Säurechlorid übergeführt und anschließend mit O,N-dimethyl-hydroxylamin zu einer Verbindung der Formel XIIa umgesetzt, und 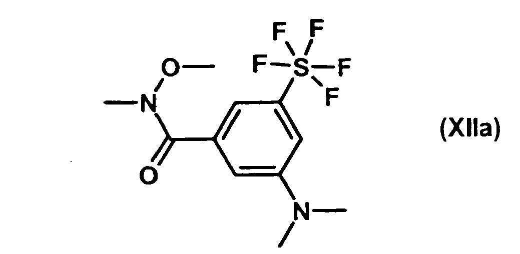
c) die erhaltene Verbindung der Formel Xlla mit Methylmagnesiumbromid in Verbindung der Formel Xa überführt.

## Claims

1. A compound of the formula I 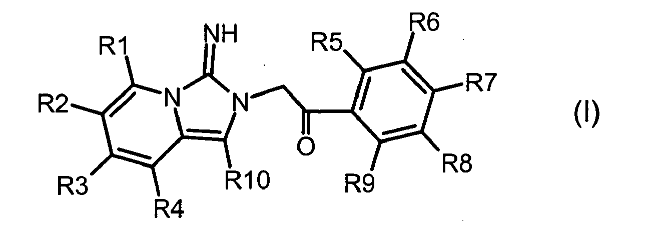 and/or all stereoisomeric or tautomeric forms of the compound of the formula I and/or mixtures of these forms in any ratio, and/or a physiologically tolerable salt of the compound of the formula I, where R1, R2, R3 and R4 are identical or different and independently of one another are
1) -(C₁-C₆)-alkyl, where alkyl is unsubstituted or mono-, di- or trisubstituted independently of one another by -(C₆-C₁₄)-aryl, -(C₃-C₆)-cycloalkyl, Het, halogen, -NH₂, -OH or methoxy,
2) -O-(C₁-C₈)-alkyl, where alkyl is unsubstituted or mono-, di- or trisubstituted independently of one another by -(C₆-C₁₄)-aryl, -(C₃-C₆)-cycloalkyl, Het, halogen, -NH₂, -OH or methoxy, where -(C₆-C₁₄)-aryl and Het are unsubstituted or additionally mono-, di- or trisubstituted by R15,
3) -(C₀-C₄)-alkylene-C(O)-R11, where R11 is
3)1) a hydrogen atom,
3)2) -(C₁-C₆)-alkyl,
3)3) -(C₀-C₄)-alkylene-(C₆-C₁₄)-aryl,
3)4) -(C₀-C₄)-alkylene-Het or
3)5) -(C₀-C₄)-alkylene-(C₃-C₆)-cycloalkyl,
4) -(C₀-C₄)-alkylene-C(O)-O-R11, where R11 is as defined above,
5) -(C₀-C₄)-alkylene-N(R12)-R13, where R12 and R13 are identical or different and independently of one another are
5)1) a hydrogen atom,
5)2) -(C₁-C₆)-alkyl,
5)3) -(C₁-C₃)-fluoroalkyl,
5)4) -(C₀-C₄)-alkylene-(C₆-C₁₄)-aryl,
5)5) -(C₀-C₄)-alkylene-Het or
5)6) -(C₀-C₄)-alkylene-(C₃-C₆)-cycloalkyl,
6) -(C₀-C₄)-alkylene-C(O)-N(R12)-R13, where R12 and R13 are identical or different and independently of one another are as defined above,
7) -(C₀-C₄)-alkylene-N(R12)-C(O)-R13, where R12 and R13 are identical or different and independently of one another are as defined above,
8) -(C₁-C₃)-fluoroalkyl,
9) -O-(C₁-C₃)-fluoroalkyl,
10) -SO₂-CH₃,
11) -SO₂-CF₃,
12) -NO₂,
13) -CN,
14) -OH,
15) a hydrogen atom or
16) halogen,
R10 and R15 are identical or different and independently of one another are
1) a hydrogen atom,
2) -(C₁-C₄)-alkyl,
3) -O-(C₁-C₄)-alkyl,
4) -(C₁-C₃)-fluoroalkyl,
5) -O-(C₁-C₃)-fluoroalkyl,
6) -(C₀-C₄)-alkylene-N(R16) (R17), in which R16 and R17 independently of one another are a hydrogen atom or -(C₁-C₆)-alkyl,
7) -(C₀-C₄)-alkylene-(C₆-C₁₄)-aryl,
8) -(C₀-C₄)-alkylene-(C₃-C₆)-cycloalkyl,
9) -(C₀-C₄)-alkylene-Het,
10) -OH,
11) =O,
12) -NO₂,
13) -CN,
14) halogen,
15) -SO₂-(C₁-C₄)-alkyl or
16) -SO₂-(C₁-C₃)-fluoroalkyl,
R5, R6, R7, R8 and R9 are identical or different and independently of one another are
1) a hydrogen atom,
2) -(C₀-C₄)-alkylene-(C₆-C₁₄)-aryl, where aryl is unsubstituted or mono-, di- or trisubstituted independently of one another by - (C₁-C₄)-alkyl,
-(C₆-C₁₄)-aryl, Het, -(C₃-C₆)-cycloalkyl, halogen, -NH₂, -OH or methoxy,
3) -(C₀-C₄)-alkylene-(C₃-C₆)-cycloalkyl,
4) -(C₀-C₄)-alkylene-Het, where Het is unsubstituted or mono-, di- or trisubstituted independently of one another by -(C₁-C₄)-alkyl, -(C₆-C₁₄)-aryl, Het, -(C₃-C₆)-cycloalkyl, -C(O)-O-R16, - C(O)-N(R16)(R17), in which R16 and R17 are as defined above, halogen, -NH₂, -OH or methoxy,
5) -SF₅,
6) -(C₁-C₆)-alkyl, where alkyl is unsubstituted or mono-, di- or trisubstituted independently of one another by halogen, -NH₂, -OH or methoxy,
7) -O-(C₁-C₈)-alkyl, where alkyl is unsubstituted or mono-, di- or trisubstituted independently of one another by -(C₆-C₁₄)-aryl, -(C₃-C₆)-cycloalkyl, Het, halogen, -NH₂, -OH or methoxy,
8) -(C₀-C₄)-alkylene-C(O)-R11, where R11 is as defined above,
9) -(C₀-C₄)-alkylene-C(O)-O-R11, where R11 is as defined above,
10) -(C₀-C₄)-alkylene-N(R12)-R13, where R12 and R13 are identical or different and independently of one another are as defined above,
11) -(C₀-C₄)-alkylene-C(O)-N(R12)-R13, where R12 and R13 are identical or different and independently of one another are as defined above,
12) -(C₀-C₄)-alkylene-N(R12)-C(O)-R13, where R12 and R13 are identical or different and independently of one another are as defined above,
13) -(C₁-C₃)-fluoroalkyl,
14) -O-(C₁-C₃)-fluoroalkyl,
15) -SO₂-CH₃,
16) -SO₂-CF₃,
17) -NO₂,
18) -CN,
19) -OH or
20) halogen, or
R5 and R6, R6 and R7, R7 and R8 or R8 and R9, together with the ring atoms to which they are bonded, form a four- to eight-membered heterocycle which, together with the phenyl ring to which the heterocycle is fused, forms a bicyclic system, where the heterocyclic moiety is unsubstituted or mono-, di- or trisubstituted independently of one another by -(C₁-C₄)-alkyl, -(C₆-C₁₄)-aryl, - (C₃-C₆)-cycloalkyl, halogen, -NH₂, -OH or methoxy, and where "Het" is a ring system having 4 to 15 carbon atoms, which are present in one, two and three ring systems bonded to one another and which each, depending on ring size, contain one, two, three or four identical or different heteroatoms from the group consisting of oxygen, nitrogen or sulfur.

2. A compound of the formula I as claimed in claim 1, where
R1, R2, R3 and R4 are identical or different and independently of one another are
1) -(C₁-C₆)-alkyl, where alkyl is unsubstituted or mono-, di- or trisubstituted independently of one another by -(C₃-C₆)-cycloalkyl, halogen, -NH₂, -OH, methoxy, -(C₆-C₁₄)-aryl, or Het,
where aryl is selected from the group consisting of phenyl, naphthyl, anthryl and fluorenyl and in which aryl is unsubstituted or mono-, di- or trisubstituted by R15,
where Het is selected from the group consisting of acridinyl, azepinyl, azetidinyl, aziridinyl, benzimidazolyl, benzofuranyl, benzothiofuranyl, benzothiophenyl, benzoxazolyl, benzothiazolyl, benzotriazolyl, benzisoxazolyl, benzisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, decahydroquinolinyl, dibenzofuranyl, dibenzothiophenyl, dihydrofuran[2,3-b]tetrahydrofuranyl, dihydrofuranyl, dioxolyl, dioxanyl, 2H, 6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolidinyl, 2-isothiazolinyl, isothiazolyl, isoxazolyl, isoxazolidinyl, 2-isoxazolinyl, morpholinyl, naphthyridinyl, octahydroisoquinolinyl, 1,2,3-oxadiazolyl, 1,2,4-oxadiazolyl, 1,2,5-oxadiazolyl, 1,3,4-oxadiazolyl, oxazolidinyl, oxazolyl, oxothiolanyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyroazolidinyl, pyrazolinyl, pyrazolyl, pyridazinyl, pyridooxazolyl, pyridoimidazolyl, pyridothiazolyl, pyridothiophenyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolidinyl, thiazolinyl, thiazolyl, thienyl, thienoimidazolyl, thienooxazolyl, thienopyrrolyl, thienopyridinyl, thienothiazolyl, thienothiophenyl, thiomorpholinyl, triazinyl, 1,2,3-triazolyl, 1,2,4-triazolyl, 1,2,5-triazolyl, 1,3,4-triazolyl and xanthenyl and in which Het is unsubstituted or additionally mono-, di- or trisubstituted by R15,
2) -O-(C₁-C₆)-alkyl, where alkyl is unsubstituted or mono-, di- or trisubstituted independently of one another by -(C₆-C₁₄)-aryl, -(C₃-C₆)-cycloalkyl, Het, halogen, -NH₂, -OH or methoxy, where Het and aryl are as defined above,
3) -(C₀-C₄)-alkylene-C(O)-R11, where R11 is
3)1) a hydrogen atom,
3)2) -(C₁-C₆)-alkyl,
3)3) -(C₀-C₄)-alkylene-(C₆-C₁₄)-aryl, where aryl is as defined above,
3)4) -(C₀-C₄)-alkylene-Het, where Het is as defined above, or
3)5) -(C₀-C₄)-alkylene-(C₃-C₆)-cycloalkyl,
4) -(C₀-C₄)-alkylene-C(O)-O-R11, where R11 is as defined above,
5) -(C₀-C₄)-alkylene-N(R12)-R13, where R12 and R13 are identical or different and independently of one another are
5)1) a hydrogen atom,
5)2) -(C₁-C₆)-alkyl,
5)3) -(C₁-C₃)-fluoroalkyl,
5)4) -(C₀-C₄)-alkylene-(C₆-C₁₄)-aryl, where aryl is as defined above,
5)5) -(C₀-C₄)-alkylene-Het, where Het is as defined above,or
5)6) -(C₀-C₄)-alkylene-(C₃-C₆)-cycloalkyl,
6) -(C₀-C₄)-alkylene-C(O)-N(R12)-R13, where R12 and R13 are identical or different and independently of one another are as defined above,
7) -(C₀-C₄)-alkylene-N(R12)-C(O)-R13, where R12 and R13 are identical or different and independently of one another are as defined above,
8) -(C₁-C₃)-fluoroalkyl,
9) -O-(C₁-C₃)-fluoroalkyl,
10) -SO₂-CH₃,
11) -SO₂-CF₃,
12) -NO₂,
13) -CN,
14) -OH,
15) a hydrogen atom or
16) halogen,
R10 and R15 are identical or different and independently of one another are
1) a hydrogen atom,
2) -(C₁-C₄)-alkyl,
3) -O-(C₁-C₄)-alkyl,
4) -(C₁-C₃)-fluoroalkyl,
5) -O-(C₁-C₃)-fluoroalkyl,
6) -(C₀-C₄)-alkylene-N(R16) (R17), in which R16 and R17 independently of one another are a hydrogen atom or -(C₁-C₆)-alkyl,
7) -(C₀-C₄)-alkylene-(C₆-C₁₄)-aryl, where aryl is as defined above,
8) -(C₀-C₄)-alkylene-(C₃-C₆)-cycloalkyl,
9) -(C₀-C₄)-alkylene-Het, where Het is as defined above,
10) -OH,
11) =O,
12) -N02,
13) -CN,
14) halogen,
15) -SO₂-(C₁-C₄)-alkyl or
16) -SO₂-(C₁-C₃)-fluoroalkyl,
R5, R6, R7, R8 and R9 are identical or different and independently of one another are
1) a hydrogen atom,
2) -(C₀-C₄)-alkylene-(C₆-C₁₄)-aryl, where aryl is unsubstituted or mono-, di- or trisubstituted independently of one another by - (C₁-C₄)-alkyl,
-(C₆-C₁₄)-aryl, Het, -(C₃-C₆)-cycloalkyl, halogen, -NH₂, -OH or methoxy, where aryl and Het are as defined above,
3) -(C₀-C₄)-alkylene-(C₃-C₆)-cycloalkyl,
4) -(C₀-C₄)-alkylene-Het, where Het is unsubstituted or mono-, di-or trisubstituted independently of one another by -(C₁-C₄)-alkyl, -(C₆-C₁₄)-aryl, Het, -(C₃-C₆)-cycloalkyl, -C(O)-O-R16, - C(O)-N(R16)(R17), in which R16 and R17 are as defined above, halogen, -NH₂, -OH or methoxy, where aryl and Het are as defined above,
5) -SF₅,
6) -(C₁-C₆)-alkyl, where alkyl is unsubstituted or mono-, di- or trisubstituted independently of one another by halogen, -NH₂, -OH or methoxy,
7) -O-(C₁-C₈)-alkyl, where alkyl is unsubstituted or mono-, di- or trisubstituted independently of one another by -(C₆-C₁₄)-aryl, -(C₃-C₆)-cycloalkyl, Het, halogen, -NH₂, -OH or methoxy, where aryl and Het are as defined above,
8) -(C₀-C₄)-alkylene-C(O)-R11, where R11 is as defined above,
9) -(C₀-C₄)-alkylene-C(O)-O-R11, where R11 is as defined above,
10) -(C₀-C₄)-alkylene-N(R12)-R13, where R12 and R13 are identical or different and independently of one another are as defined above,
11) -(C₀-C₄)-alkylene-C(O)-N(R12)-R13, where R12 and R13 are identical or different and independently of one another are as defined above,
12) -(C₀-C₄)-alkylene-N(R12)-C(O)-R13, where R12 and R13 are identical or different and independently of one another are as defined above,
13) -(C₁-C₃)-fluoroalkyl,
14) -O-(C₁-C₃)-fluoroalkyl,
15) -SO₂-CH₃,
16) -SO₂-CF₃,
17) -NO₂,
18) -CN,
19) -OH or
20) halogen, or
R5 and R6, R6 and R7, R7 and R8 or R8 and R9, together with the ring atoms to which they are bonded, form a four- to eight-membered heterocycle which, together with the phenyl ring to which the heterocycle is fused, forms a bicyclic system selected from the group consisting of benzimidazole, benzisothiazole, benzisoxazole, benzo[1,3]dioxole, benzofuranyl, benzothiazole, benzisoxazole, benzothiofuran, benzothiophene, benzo[1,3]oxathiole, benzoxazole, benzothiazole, benzotriazolyl, quinazoline, quinazolone, quinoline, 4H-quinolizine, quinoxaline, chroman, chromene, cinnoline, 2,3-dihydrobenzo[1,4]dioxin, 2,3-dihydrobenzofuranyl, 1,3-dihydro-isobenzofuran, 3,4-dihydro-2H-benzo[1,4]oxazine, 2,3-dihydrobenzooxazole, 2,3-dihydrobenzothiazole, 1,3-dihydrobenzo[c]thiophene, 2,3-dihydro-benzo[b]thiophene, indazole, indole, indoline, isobenzofuran, isoquinoline, isochroman, isoindazole, isoindole, isoindoline, 7-oxabicyclo[4.2.0]octa-1,3,5-triene, phthalazine, 2,3,4,5-tetrahydro-1H-benzo[b]azepine, 6,7,8,9-tetrahydro-5-oxa-9-azabenzocycloheptene, 3,4,5,6-tetrahydro-2H-benzo[b][1,4]oxazozine, tetrahydroquinoline, 1,2,3,4-tetrahydroquinoxaline or tetrahydroisoquinoline, where the heterocyclic moiety is unsubstituted or mono-, di- or trisubstituted independently of one another by -(C₁-C₄)-alkyl, -(C₆-C₁₄)-aryl, -(C₃-C₆)-cycloalkyl, halogen, -NH₂, -OH or methoxy.

3. A compound of the formula I as claimed in claims 1 or 2, where
R1, R2, R3 and R4 are identical or different and independently of one another are
1) a hydrogen atom,
2) -(C₁-C₄)-alkyl,
3) -O-(C₁-C₄)-alkyl,
4) -(C₀-C₄)-alkylene-C(O)-N(R12)-R13, where R12 and R13 are identical or different and independently of one another are a hydrogen atom or
-(C₁-C₄)-alkyl,
5) -(C₁-C₃)-fluoroalkyl,
6) -(C₀-C₄)-alkylene-C(O)-O-(C₁-C₄)-alkyl or
7) halogen,
R10 is 1) a hydrogen atom,
2) -(C₁-C₄)-alkyl,
3) -(C₀-C₄)-alkylene-(C₃-C₆)-cycloalkyl or
4) -(C₀-C₄)-alkylenephenyl,
R5, R6, R7, R8 and R9 are identical or different and independently of one another are
1) a hydrogen atom,
2) -(C₁-C₃)-fluoroalkyl,
3) halogen,
4) -O-(C₁-C₄)-alkyl,
5) -OH,
6) -(C₁-C₄)-alkyl,
7) -SF₅,
8) -(C₀-C₄)-alkylene-NH-C(O)-(C₁-C₃)-fluoroalkyl,
9) -(C₀-C₄)-alkylene-N(R12)-R13, where R12 and R13 are identical or different and independently of one another are a hydrogen atom or
-(C₁-C₄)-alkyl, or
10) -(C₀-C₄)-alkylene-Het, where Het is selected from the group consisting of morpholinyl or pyrrolidinyl and is unsubstituted or mono- or disubstituted independently of one another by - (C₁-C₄)-alkyl, =O or
-NH₂, or
R5 and R6, R6 and R7, R7 and R8 or R8 and R9, together with the ring atoms to which they are bonded, form a four- to eight-membered heterocycle which, together with the phenyl ring to which the heterocycle is fused, forms a bicyclic system selected from the group consisting of 2,3-dihydro-benzo[1,4]dioxin, benzo[1,3]dioxole, 3,4-dihydro-2H-benzo[1,4]oxazine, 2,3,4,5-tetrahydro-1H-benzo[b]azepine, tetrahydroquinoline, tetrahydroisoquinoline, 1,2,3,4-tetrahydroquinoxaline or 6,7,8,9-tetrahydro-5-oxa-9-azabenzocycloheptene, where the heterocyclic moiety is unsubstituted or mono- or disubstituted by -(C₁-C₄)-alkyl or halogen.

4. A compound of the formula I as claimed in one or more of claims 1 to 3, which is the compound
1-(3,5-di-tert-butyl-4-hydroxyphenyl)-2-(3-iminoimidazo[1,5-a]pyridin-2-yl)ethanone,
2-(3-iminoimidazo[1,5-a]pyridin-2-yl)-1-(3-pentafluorosulfanylphenyl)ethanone,
2-(1-cyclopropyl-3-iminoimidazo[1,5-a]pyridin-2-yl)-1-(3,5-di-tert-butyl-4-hydroxyphenyl)ethanone,
1-(3,5-di-tert-butyl-4-hydroxyphenyl)-2-(3-imino-1-phenylimidazo[1,5-a]pyridin-2-yl)ethanone,
1-(3,5-di-tert-butyl-4-hydroxyphenyl)-2-(3-imino-8-methylimidazo[1,5-a]pyridin-2-yl)ethanone,
1-(3,5-di-tert-butyl-4-hydroxyphenyl)-2-(3-imino-8-methylimidazo[1,5-a]pyridin-2-yl)ethanone as the hydrobromide salt,
1-(3,5-di-tert-butyl-4-hydroxyphenyl)-2-(3-imino-7-trifluoromethylimidazo[1,5-a]pyridin-2-yl)ethanone,
1-(3,5-di-tert-butyl-4-hydroxyphenyl)-2-(3-imino-7-trifluoromethylimidazo[1,5-a]pyridin-2-yl)ethanone as the hydrobromide salt,
methyl 2-[2-(3,5-di-tert-butyl-4-hydroxyphenyl)-2-oxoethyl]-3-imino-2,3-dihydroimidazo[1,5-a]pyridine-6-carboxylate,
methyl 2-[2-(3-tert-butyl-4-methoxy-5-morpholin-4-ylphenyl)-2-oxoethyl]-3-imino-2,3-dihydroimidazo[1,5-a]pyridine-6-carboxylate,
1-(3-tert-butyl-4-methoxy-5-morpholin-4-ylphenyl)-2-(3-iminoimidazo[1,5-a]pyridin-2-yl)ethanone,
1-(3-tert-butyl-4-methoxy-5-morpholin-4-ylphenyl)-2-(3-imino-8-methylimidazo[1,5-a]pyridin-2-yl)ethanone,
1-(3-dimethylamino-5-pentafluorosulfanylphenyl)-2-(3-iminoimidazo[1,5-a]pyridin-2-yl)ethanone,
2-[2-(8-tert-butyl-4-methyl-3,4-dihydro-2H-benzo[1,4]oxazin-6-yl)-2-oxoethyl]-7-ethoxy-3-imino-2,3-dihydroimidazo[1,5-a]pyridine-6-carboxylic acid methylamide or
2-[2-(3-tert-butyl-4-methoxy-5-morpholin-4-ylphenyl)-2-oxoethyl]-7-chloro-3-imino-2,3-dihydroimidazo[1,5-a]pyridine-6-carboxylic acid methylamide.

5. A medicament which comprises an efficacious amount of at least one compound of the formula I as claimed in one or more of claims 1 to 4 together with a pharmaceutically suitable and physiologically tolerable vehicle, additive and/or other active ingredients and excipients.

6. The use of the compound of the formula I as claimed in one or more of claims 1 to 4 for the production of a medicament for the prophylaxis, secondary prevention and therapy of all diseases of the type which accompany thromboses, embolisms, hypercoagulability or fibrotic changes.

7. The use as claimed in claim 6, which comprises myocardial infarct, angina pectoris and other forms of acute coronary syndrome, stroke, peripheral vascular disorders, deep vein thrombosis, pulmonary embolism, embolic or thrombotic events caused by cardiac arrhythmias, cardiovascular events such as restenosis after revascularization and angioplasty and similar interventions such as stent implantations and bypass operations, or the reduction of the risk of thrombosis after surgical interventions such as in knee and hip joint operations, or interventions which lead to contact of the blood with foreign surfaces such as in dialysis patients and patients having indwelling catheters or disseminated intravascular coagulation, sepsis and other intravascular events which are accompanied by inflammation, atherosclerosis, diabetes and the metabolic syndrome and their sequelae, tumor growth and tumor metastasis, inflammatory and degenerative joint disorders such as rheumatoid arthritis and arthrosis, disorders of the hemostatic system such as fibrin deposits, fibrotic changes of the lung, such as chronic obstructive pulmonary disease, adult respiratory distress syndrome or fibrin deposits of the eye after eye operations or prevention and/or treatment of scar formation.

8. A process for the preparation of the compound of the formula I as claimed in one or more of claims 1 to 4, which comprises
a) reacting a compound of the formula II 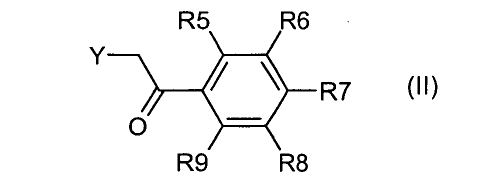 where the radicals R5, R6, R7, R8 and R9 are as defined in formula I and Y is chloride, bromide, mesylate or tosylate, with a compound of the formula III 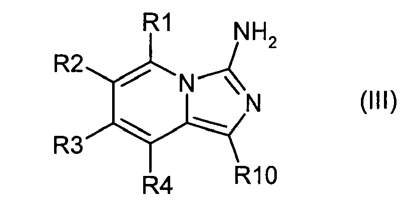 in the presence of a base and of a solvent to give a compound of the formula I, or
b) reacting a compound of the formula VII 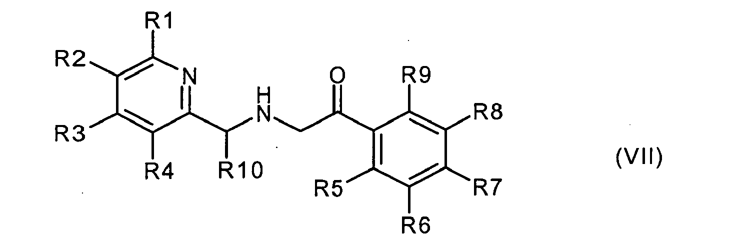 where the radicals R1 to R10 are as defined in formula I, with a compound Z-CN, where Z is tosylate or bromide, in the presence of a base to give a compound of the formula I, or
c) either isolating the compound of the formula I prepared according to process a) or b) in free form or releasing it from physiologically intolerable salts or, in the case of the presence of acidic or basic groups, converting it to physiologically tolerable salts, or
d) separating a compound of the formula I prepared according to process a) or b), or a suitable precursor of the formula I, which on account of its chemical structure occurs in enantiomeric or diastereomeric forms, by salt formation with enantiomerically pure acids or bases, chromatography on chiral stationary phases or derivatization by means of chiral enantiomerically pure compounds such as amino acids, separation of the diastereomers thus obtained, and cleavage of the chiral auxiliary groups into the pure enantiomers or diastereomers.

9. The process for the preparation of the compound of the formula VII as claimed in claim 8, wherein a compound of the formula VIII 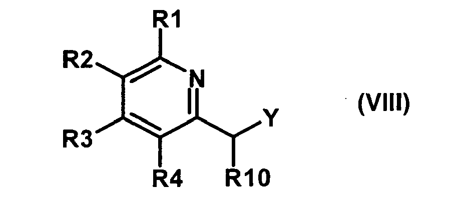 where the radicals R1, R2, R3, R4 and R10 are as defined in formula I and Y is chloride, bromide, mesylate or tosylate, is reacted with a compound of the formula IX 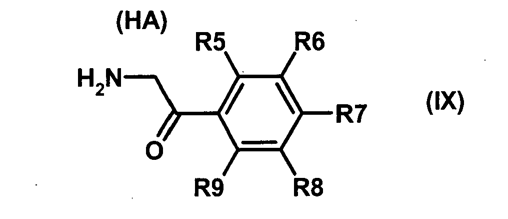 in the presence of a base and of a solvent to give a compound of the formula I.

10. The process for the preparation of the compound of the formula II as claimed in claim 8, where Y is Br and one of the radicals R5, R6, R7, R8 or R9 is pentafluorosulfanyl, wherein
a) a compound of the formula Xa 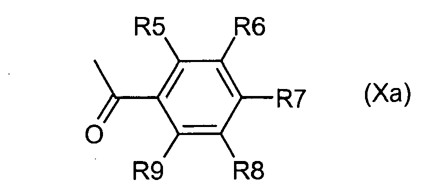 where one of the radicals R5, R6, R7, R8 or R9 is pentafluorosulfanyl and the other radicals R5, R6, R7, R8 and R9 are as defined in formula I, is converted with a brominating reagent to the compound of the formula II, or
b) a compound of the formula XIa 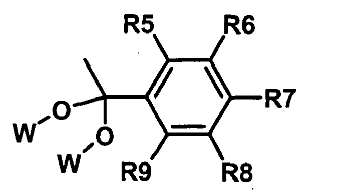
where one of the radicals R5, R6, R7, R8 or R9 is pentafluorosulfanyl and the other radicals R5, R6, R7, R8 and R9 are as defined in formula I, W is ethylene, propylene or butylene or, together with the group
-O-C-O-, forms a 1,3-dioxo ring of ring size 5, 6 or 7, is treated with a brominating reagent and subsequently converted in the presence of an acid to the compound of the formula II.

11. The process for the preparation of the compound of the formula Xa as claimed in claim 10, where R6 is pentafluorosulfanyl and R8 is dimethylamine and the radicals R5, R7 and R9 are as defined in formula I, wherein
a) a compound of the formula XIVa 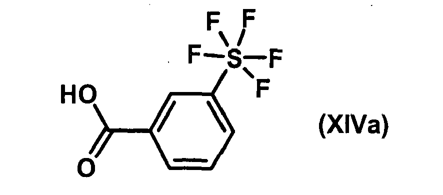 is first nitrated and subsequently reduced with hydrogen to give the amine of the formula XIIIa, 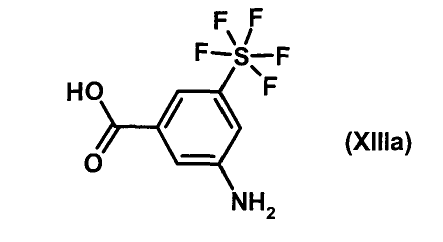
b) the compound of the formula XIIIa obtained is dimethylated on the nitrogen and the carboxylic acid is converted with thionyl chloride to the acid chloride and subsequently reacted with O,N-dimethylhydroxylamine to give a compound of the formula XIIa, and 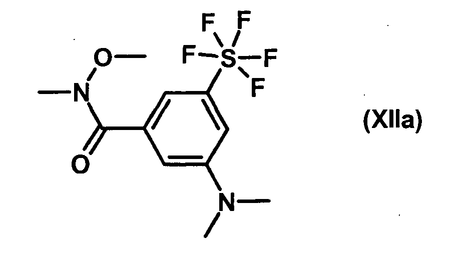
c) the compound of the formula Xlla obtained is converted with methylmagnesium bromide to the compound of the formula Xa.

## Revendications

1. Composé de formule I 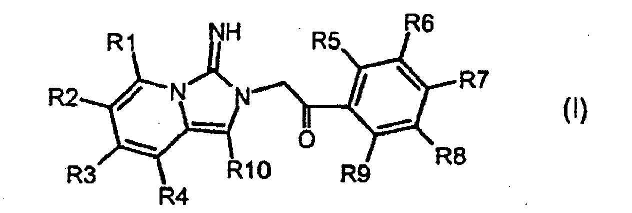 et/ou toutes les formes stéréo-isomères ou tautomères du composé de formule I et/ou les mélanges de ces formes, dans tous les rapports, et/ou un sel physiologiquement acceptable du composé de formule (I), où
R1, R2, R3 et R4 sont identiques ou différents et représentent, indépendamment l'un de l'autre
1) -(C₁-C₆)-alkyle, où alkyle est non substitué ou monosubstitué, disubstitué ou trisubstitué, indépendamment l'un de l'autre, par -(C₆-C₁₄)-aryle, -(C₃-C₆)-cycloalkyle, Het, halogène, -NH₂, -OH ou méthoxy,
2) -O-(C₁-C₈)-alkyle, où alkyle est non substitué ou monosubstitué, disubstitué ou trisubstitué, indépendamment l'un de l'autre, par -(C₆-C₁₄) aryle, -(C₃-C₆)-cycloalkyle, Het, halogène, -NH₂, -OH ou méthoxy, où -(C₆-C₁₄)-aryle et Het sont non substitués ou en outre monosubstitués, disubstitués ou trisubstitués par R15,
3) -(C₀-C₄)-alkylène-C(O)-R11, où R11 représente
3)1) un atome d'hydrogène,
3)2) (C₁-C₆)-alkyle,
3)3) -(C₀-C₄)-alkylène-(C₆-C₁₄)-aryle,
3)4) -(C₀-C₄)-alkylène-Het ou
3)5) -(C₀-C₄)-alkylène-(C₃-C₆)-cycloalkyle,
4) -(C₀-C₄)-alkylène-C(O)-O-R11, où R11 est défini comme ci-dessus,
5) -(C₀-C₄)-alkylène-N(R12)-R13, où R12 et R13 sont identiques ou différents et représentent, indépendamment l'un de l'autre
5)1) un atome d'hydrogène,
5)2) -(C₁-C₆)-alkyle,
5)3) -(C₁-C₃)-fluoroalkyle,
5)4) -(C₀-C₄)-alkylène-(C₆-C₁₄)-aryle,
5)5) -(C₀-C₄)-alkylène-Het ou
5)6) -(C₀-C₄)-alkylène-(C₃-C₆)-cycloalkyle,
6) -(C₀-C₄)-alkylène-C(O)-N(R12)-R13, où R12 et R13 sont identiques ou différents et sont définis, indépendamment l'un de l'autre, comme ci-dessus,
7) -(C₀-C₄)-alkylène-N(R12)-C(O)-R13, où R12 et R13 sont identiques ou différents et sont définis, indépendamment l'un de l'autre, comme ci-dessus,
8) -(C₁-C₃)-fluoroalkyle,
9) -O-(C₁-C₃)-fluoroalkyle,
10) -SO₂-CH₃,
11) -SO₂-CF₃,
12) -NO₂,
13) -CN
14) -OH,
15) =O,
16) un atome d'hydrogène ou
17) halogène,
R10 et R15 sont identiques ou différents et représentent, indépendamment l'un de l'autre
1) un atome d'hydrogène,
2) -(C₁-C₄)-alkyle,
3) -O-(C₁-C₄)-alkyle,
4) -(C₁-C₃)-fluoroalkyle,
5) -O-(C₁-C₃)-fluoroalkyle,
6) -(C₀-C₄)-alkylène-N(R16)(R17), où R16 et R17 signifient, indépendamment l'un de l'autre, un atome d'hydrogène ou -(C₁-C₆)-alkyle, ou
7) -(C₀-C₄)-alkylène-(C₆-C₁₄)-aryle,
8) -(C₀-C₄)-alkylène-(C₃-C₆)-cycloalkyle,
9) -(C₀-C₄)-alkylène-Het,
10) -OH,
11) =O,
12) -NO₂,
13) -CN
14) halogène,
15) -SO₂-(C₁-C₄)-alkyle ou
16) -SO₂-(C₁-C₃)-fluoroalkyle,
R5, R6, R7, R8 et R9 sont identiques ou différents et représentent, indépendamment l'un de l'autre
1) un atome d'hydrogène,
2) -(C₀-C₄)-alkylène-(C₆-C₁₄)-aryle, où aryle est non substitué ou monosubstitué, disubstitué ou trisubstitué, indépendamment l'un de l'autre, par
-(C₁-C₄)-alkyle, -(C₆-C₁₄)-aryle, Het, -(C₃-C₆)-cycloalkyle, halogène, -NH₂, -OH ou méthoxy,
3) -(C₀-C₄)-alkylène-(C₃-C₆)-cycloalkyle,
4) -(C₀-C₄)-alkylène-Het, où Het est non substitué ou monosubstitué, disubstitué ou trisubstitué, indépendamment l'un de l'autre, par -(C₁-C₄)-alkyle, - (C₆-C₁₄) -aryle, Het, - (C₃-C₆) -cycloalkyle,
-C (O) -O-R16, -C (O) -N (R16) (R17), où R16 et R17 sont définis comme ci-dessus, halogène, -NH₂, -OH ou méthoxy,
5) -SF₅,
6) -(C₁-C₆)-alkyle, où alkyle est non substitué ou monosubstitué, disubstitué ou trisubstitué, indépendamment l'un de l'autre, par halogène, -NH₂, -OH ou méthoxy,
7) -O-(C₁-C₈)-alkyle, où alkyle est non substitué ou monosubstitué, disubstitué ou trisubstitué, indépendamment l'un de l'autre, par -(C₆-C₁₄)-aryle, -(C₃-C₆)-cycloalkyle, Het, halogène, -NH₂, -OH ou méthoxy,
8) -(C₀-C₄)-alkylène-C(O)-R11, où R11 est défini comme ci-dessus,
9) -(C₀-C₄)-alkylène-C(O)-O-R11, où R11 est défini comme ci-dessus,
10) -(C₀-C₄)-alkylène-N(R12)-R13, où R12 et R13 sont identiques ou différents et sont définis, indépendamment l'un de l'autre, comme ci-dessus,
11) -(C₀-C₄)-alkylène-C(O)-N(R12)-R13, où R12 et R13 sont identiques ou différents et sont définis, indépendamment l'un de l'autre, comme ci-dessus,
12) -(C₀-C₄)-alkylène-N(R12)-C(O)-R13, où R12 et R13 sont identiques ou différents et sont définis, indépendamment l'un de l'autre, comme ci-dessus,
13) -(C₁-C₃)-fluoroalkyle,
14) -O-(C₁-C₃)-fluoroalkyle,
15) -SO₂-CH₃,
16) -SO₂-CF₃,
17) -NO₂,
18) -CN
19) -OH ou
20) halogène, ou
R5 et R6, R6 et R7, R7 et R8 ou R8 et R9 forment ensemble avec les atomes de cycle auxquels ils sont liés, un hétérocycle de quatre à huit chaînons, qui forme, ensemble avec le cycle phényle, sur lequel l'hétérocycle est annelé, un système bicyclique, où la partie hétérocyclique est non substituée ou monosubstituée, disubstituée ou trisubstituée, indépendamment l'un de l'autre, par -(C₁-C₄)-alkyle, -(C₆-C₁₄)-aryle, -(C₃-C₆)-cycloalkyle, halogène, -NH₂, -OH ou méthoxy, et où "Het" sont des systèmes cycliques comprenant 4 à 15 atomes de carbone, qui se trouvent dans un, deux ou trois systèmes cycliques reliés ensemble et qui, en fonction de la taille du cycle, contiennent un, deux, trois ou quatre hétéroatomes identiques ou différents de la série oxygène, azote ou soufre.

2. Composé de formule I selon la revendication 1, où R1, R2, R3 et R4 sont identiques ou différents et représentent, indépendamment l'un de l'autre
1) -(C₁-C₆)-alkyle, où alkyle est non substitué ou monosubstitué, disubstitué ou trisubstitué, indépendamment l'un de l'autre, par -(C₃-C₆)-cycloalkyle, halogène, -NH₂, -OH, méthoxy, -(C₆-C₁₄)-aryle, ou Het,
- où aryle est choisi dans le groupe constitué par phényle, naphtyle, anthryle et fluorényle et où aryle est non substitué ou monosubstitué, disubstitué ou trisubstitué par R15,
- où Het est choisi dans le groupe acridinyle, azépinyle, azétidinyle, aziridinyle, benzimidazolyle, benzofurannyle, benzothiofurannyle, benzothiophényle, benzoxazolyle, benzothiazolyle, benzotriazolyle, benzo-isoxazolyle, benzo-isothiazolyle, carbazolyle, 4aH-carbazolyle, carbolinyle, quinazolinyle, quinoléinyle, 4H-quinoléizinyle, quinoxalinyle, quinuclidinyle, chromanyle, chroményle, cinnolinyle, décahydroquinoléinyle, dibenzofurannyle, dibenzothiophényle, dihydrofuranne[2,3-b]-tétrahydrofurannyle, dihydrofurannyle, dioxolyle, dioxanyle, 2H,6H-1,5,2-dithiazinyle, furannyle, furazanyle, imidazolidinyle, imidazolinyle, imidazolyle, 1H-indazolyle, indolinyle, indolizinyle, indolyle, 3H-indolyle, isobenzofurannyle, isochromanyle, iso-indazolyle, iso-indolinyle, iso-indolyle, isoquinoléinyle, isothiazolidinyle, 2-isothiazolinyle, isothiazolyle, isoxazolyle, isoxazolidinyle, 2-isoxazolinyle, morpholinyle, naphtyridinyle, octahydro-isoquinoléinyle, 1,2,3-oxadiazolyle, 1,2,4-oxadiazolyle, 1,2,5-oxadiazolyle, 1,3,4-oxadiazolyle, oxazolidinyle, oxazolyle, oxothiolanyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purinyle, pyrannyle, pyrazinyle, pyroazolidinyle, pyrazolinyle, pyrazolyle, pyridazinyle, pyridooxazolyle, pyrido-imidazolyle, pyridothiazolyle, pyridothiophényle, pyridyle, pyrimidinyle, pyrrolidinyle, pyrrolinyle, 2H-pyrrolyle, pyrrolyle, tétrahydrofurannyle, tétrahydro-isoquinoléinyle, tétrahydroquinoléinyle, tétrahydropyridinyle, 6H-1,2,5-thiadiazinyle, 1,2,3-thiadiazolyle, 1,2,4-thiadiazolyle, 1,2,5-thiadiazolyle, 1,3,4-thiadiazolyle, thianthrényle, thiazolidinyle, thiazolinyle, thiazolyle, thiényle, thiéno-imidazolyle, thiéno-oxazolyle, thiénopyrrolyle, thiénopyridinyle, thiénothiazolyle, thiénothiophényle, thiomorpholinyle, triazinyle, 1,2,3-triazolyle, 1,2,4-triazolyle, 1,2,5-triazolyle, 1,3,4-triazolyle et xanthényle et où Het est non substitué ou en outre monosubstitué, disubstitué ou trisubstitué par R15,
2) -O-(C₁-C₆)-alkyle, où alkyle est non substitué ou monosubstitué, disubstitué ou trisubstitué, indépendamment l'un de l'autre, par - (C₆-C₁₄) - aryle, -(C₃-C₆)-cycloalkyle, Het, halogène, -NH₂, -OH ou méthoxy, où Het et aryle sont définis comme ci-dessus,
3) -(C₀-C₄)-alkylène-C(O)-R11, où R11 représente
3)1) un atome d'hydrogène,
3)2) -(C₁-C₆)-alkyle,
3)3) -(C₀-C₄)-alkylène-(C₆-C₁₄)-aryle, où aryle est défini comme ci-dessus,
3)4) -(C₀-C₄)-alkylène-Het, où Het est défini comme ci-dessus, ou
3)5) -(C₀-C₄)-alkylène-(C₃-C₆)-cycloalkyle,
4) -(C₀-C₄)-alkylène-C(O)-O-R11, où R11 est défini comme ci-dessus,
5) -(C₀-C₄)-alkylène-N(R12)-R13, où R12 et R13 sont identiques ou différents et représentent, indépendamment l'un de l'autre
5)1) un atome d'hydrogène,
5)2) -(C₁-C₆)-alkyle,
5)3) -(C₁-C₃)-fluoroalkyle,
5)4) -(C₀-C₄)-alkylène-(C₆-C₁₄)-aryle, où aryle est défini comme ci-dessus,
5)5) -(C₀-C₄)-alkylène-Het, où Het est défini comme ci-dessus, ou
5)6) -(C₀-C₄)-alkylène-(C₃-C₆)-cycloalkyle,
6) -(C₀-C₄)-alkylène-C(O)-N(R12)-R13, où R12 et R13 sont identiques ou différents et sont définis, indépendamment l'un de l'autre, comme ci-dessus,
7) -(C₀-C₄)-alkylène-N(R12)-C(O)-R13, où R12 et R13 sont identiques ou différents et sont définis, indépendamment l'un de l'autre, comme ci-dessus,
8) -(C₁-C₃)-fluoroalkyle,
9) -O-(C₁-C₃)-fluoroalkyle,
10) -SO₂-CH₃
11) -SO₂-CF₃
12) -NO₂,
13) -CN
14) -OH,
15) =O,
16) un atome d'hydrogène ou
17) halogène,
R10 et R15 sont identiques ou différents et représentent, indépendamment l'un de l'autre
1) un atome d'hydrogène,
2) -(C₁-C₄)-alkyle,
3) -O-(C₁-C₄)-alkyle,
4) -(C₁-C₃)-fluoroalkyle,
5) -O-(C₁-C₃)-fluoroalkyle,
6) -(C₀-C₄)-alkylène-N(R16) (R17), où R16 et R17 signifient, indépendamment l'un de l'autre, un atome d'hydrogène ou -(C₁-C₆)-alkyle, ou
7) -(C₀-C₄)-alkylène-(C₆-C₁₄)-aryle, où aryle est défini comme ci-dessus,
8) -(C₀-C₄)-alkylène-(C₃-C₆)-cycloalkyle,
9) -(C₀-C₄)-alkylène-Het, où Het est défini comme ci-dessus,
10) -OH,
11) =O,
12) -NO₂,
13) -CN
14) halogène,
15) -SO₂-(C₁-C₄)-alkyle ou
16) -SO₂-(C₁-C₃)-fluoroalkyle,
R5, R6, R7, R8 et R9 sont identiques ou différents et représentent, indépendamment l'un de l'autre
1) un atome d'hydrogène,
2) -(C₀-C₄)-alkylène-(C₆-C₁₄)-aryle, où aryle est non substitué ou monosubstitué, disubstitué ou trisubstitué, indépendamment l'un de l'autre, par -(C₁-C₄)-alkyle, -(C₆-C₁₄)-aryle, Het, -(C₃-C₆)-cycloalkyle, halogène, -NH₂, -OH ou méthoxy, ou aryle et Het sont définis comme ci-dessus,
3) -(C₀-C₄)-alkylène-(C₃-C₆)-cycloalkyle,
4) -(C₀-C₄)-alkylène-Het, où Het est non substitué ou monosubstitué, disubstitué ou trisubstitué, indépendamment l'un de l'autre, par -(C₁-C₄)-alkyle, -(C₆-C₁₄)-aryle, Het, -(C₃-C₆)-cycloalkyle, -C(O)-O-R16, -C(O)-N(R16) (R17), où R16 et R17 sont définis comme ci-dessus, halogène, -NH₂, -OH ou méthoxy, où aryle et Het sont définis comme ci-dessus,
5) -SF₅,
6) -(C₁-C₆)-alkyle, où alkyle est non substitué ou monosubstitué, disubstitué ou trisubstitué, indépendamment l'un de l'autre, par halogène, -NH₂, -OH ou méthoxy,
7) -O-(C₁-C₈)-alkyle, où alkyle est non substitué ou monosubstitué, disubstitué ou trisubstitué, indépendamment l'un de l'autre, par -(C₆-C₁₄)-aryle, -(C₃-C₆)-cycloalkyle, Het, halogène, -NH₂, -OH ou méthoxy, où Het et aryle sont définis comme ci-dessus,
8) -(C₀-C₄)-alkylène-C(O)-R11, où R11 est défini comme ci-dessus,
9) -(C₀-C₄)-alkylène-C(O)-O-R11, où R11 est défini comme ci-dessus,
10) -(C₀-C₄)-alkylène-N(R12)-R13, où R12 et R13 sont identiques ou différents et sont définis, indépendamment l'un de l'autre, comme ci-dessus,
11) -(C₀-C₄)-alkylène-C(O)-N(R12)-R13, où R12 et R13 sont identiques ou différents et sont définis, indépendamment l'un de l'autre, comme ci-dessus,
12) -(C₀-C₄)-alkylène-N(R12)-C(O)-R13, où R12 et R13 sont identiques ou différents et sont définis, indépendamment l'un de l'autre, comme ci-dessus,
13) -(C₁-C₃)-fluoroalkyle,
14) -O-(C₁-C₃)-fluoroalkyle,
15) -SO₂-CH₃,
16) -SO₂-CF₃,
17) -NO₂,
18) -CN
19) -OH ou
20) halogène, ou
R5 et R6, R6 et R7, R7 et R8 ou R8 et R9 forment ensemble avec les atomes de cycle auxquels ils sont liés, un hétérocycle de quatre à huit chaînons, qui forme, ensemble avec le cycle phényle, sur lequel l'hétérocycle est annelé, un système bicyclique, choisi dans le groupe benzo-imidazole, benzo-isothiazole, benzo-isoxazole, benzo[1,3]dioxole, benzofurannyle, benzothiazole, benzo-isoxazole, benzothiofuranne, benzothiophène, benzo[1,3]oxathiole, benzoxazole, benzothiazole, benzotriazolyle, quinazoline, quinazolone, quinoléine, 4H-quinoléizine, quinoxaline, chromane, chromène, cinnoline, 2,3-dihydrobenzo[1,4]dioxine, 2,3-dihydrobenzofurannyle, 1,3-dihydro-isobenzofuranne, 3,4-dihydro-2H-benzo[1,4]oxazine, 2,3-dihydrobenzooxazole, 2,3-dihydrobenzothiazole, 1,3-dihydrobenzo[c]thiophène, 2,3-dihydrobenzo[b]thiophène, indazole, indole, indoline, isobenzofuranne, isoquinoléine, isochromane, iso-indazole, iso-indole, iso-indoline, 7-oxabicyclo[4,2,0]octa-1,3,5-triène, phtalazine, 2,3,4,5-tétrahydro-1H-benzo[b]azépine, 6,7,8,9-tétrahydro-5-oxa-9-azabenzocycloheptène, 3,4,5,6-tétrahydro-2H-benzo[1,4]oxazozine, tétrahydroquinoléine, 1,2,3,4-tétrahydroquinoxaline ou tétrahydro-isoquinoléine, où la partie hétérocyclique est non substituée ou monosubstituée, disubstituée ou trisubstituée, indépendamment l'un de l'autre, par -(C₁-C₄)-alkyle, -(C₆-C₁₄)-aryle, -(C₃-C₆)-cycloalkyle, halogène, -NH₂, -OH ou méthoxy.

3. Composé de formule I selon les revendications 1 ou 2, où
R1, R2, R3 et R4 sont identiques ou différents et représentent, indépendamment l'un de l'autre
1) un atome d'hydrogène,
2) -(C₁-C₄)-alkyle,
3) -O-(C₁-C₄)-alkyle,
4) -(C₀-C₄)-alkylène-C(O)-N(R12)-R13, où R12 et R13 sont identiques ou différents et signifient, indépendamment l'un de l'autre, un atome d'hydrogène ou -(C₁-C₄)-alkyle,
5) -(C₁-C₃)-fluoroalkyle,
6) -(C₀-C₄)-alkylène-C(O)-O-(C₁-C₄)-alkyle ou
7) =O,
8) halogène,
R10 représente
1) un atome d'hydrogène,
2) -(C₁-C₄)-alkyle,
3) -(C₀-C₄)-alkylène-(C₃-C₆)-cycloalkyle ou
4) -(C₀-C₄)-alkylène-phényle,
R5, R6, R7, R8 et R9 sont identiques ou différents et représentent, indépendamment l'un de l'autre
1) un atome d'hydrogène,
2) -(C₁-C₃)-fluoroalkyle,
3) halogène,
4) -O-(C₁-C₄)-alkyle,
5) -OH,
6) -(C₁-C₄)-alkyle,
7) -SF₅,
8) -(C₀-C₄)-alkylène-NH-C(O)-(C₁-C₃)-fluoroalkyle,
9) -(C₀-C₄)-alkylène-N(R12) -R13, où R12 et R13 sont identiques ou différents et signifient, indépendamment l'un de l'autre, un atome d'hydrogène ou -(C₁-C₄)-alkyle, ou
10) -(C₀-C₄)-alkylène-Het, où Het est choisi dans le groupe formé par morpholinyle ou pyrrolidinyle et est non substitué ou monosubstitué ou disubstitué, indépendamment l'un de l'autre, par -(C₁-C₄)-alkyle, =O ou -NH₂, ou
R5 et R6, R6 et R7, R7 et R8 ou R8 et R9 forment ensemble avec les atomes de cycle, auxquels ils sont liés, un hétérocycle de quatre à huit chaînons, qui forme ensemble avec le cycle phényle, sur lequel l'hétérocycle est annelé, un système bicyclique, choisi dans le groupe formé par 2,3-dihydrobenzo[1,4]dioxine, benzo[1,3]dioxole, 3,4-dihydro-2H-benzo[1,4]oxazine, 2,3,4,5-tétrahydro-1H-benzo[b]azépine,
tétrahydroquinoléine, tétrahydro-isoquinoléine, 1,2,3,4-tétrahydroquinoxaline ou 6,7,8,9-tétrahydro-5-oxa-9-azabenzocycloheptène, où la partie hétérocyclique est non substituée ou monosubstituée ou disubstituée par -(C₁-C₄)-alkyle ou halogène.

4. Composé de formule I selon l'une ou plusieurs des revendications 1 à 3, **caractérisé en ce qu'**il s'agit du composé
1-(3,5-di-tert-butyl-4-hydroxyphényl)-2-(3-imino-imidazo[1,5-a]pyridin-2-yl)-éthanone,
2-(3-imino-imidazo[1,5-a]pyridin-2-yl)-1-(3-pentafluorosulfanylphényl)-éthanone,
2-(1-cyclopropyl-3-imino-imidazo[1,5-a]pyridin-2-yl)-1-(3,5-di-tert-butyl-4-hydroxyphényl)-éthanone,
1-(3,5-di-tert-butyl-4-hydroxyphényl)-2-(3-imino-1-phénylimidazo[1,5-a]pyridin-2-yl)-éthanone,
1-(3,5-di-tert-butyl-4-hydroxyphényl)-2-(3-imino-8-méthylimidazo[1,5-a]pyridin-2-yl)-éthanone,
1-(3,5-di-tert-butyl-4-hydroxyphényl)-2-(3-imino-8-méthylimidazo[1,5-a]pyridin-2-yl)-éthanone sous forme de sel bromhydrate,
1-(3,5-di-tert-butyl-4-hydroxyphényl)-2-(3-imino-7-trifluorométhylimidazo[1,5-a]pyridin-2-yl)-éthanone,
1-(3,5-di-tert-butyl-4-hydroxyphényl)-2-(3-imino-7-trifluorométhylimidazo[1,5-a]pyridin-2-yl)-éthanone sous forme de sel bromhydrate,
ester méthylique de l'acide 2-[2-(3,5-di-tert-butyl-4-hydroxyphényl)-2-oxoéthyl]-3-imino-2,3-dihydro-imidazo[1,5-a]pyridine-6-carboxylique,
ester méthylique de l'acide 2-[2-(3-tert-butyl-4-méthoxy-5-morpholin-4-ylphényl)-2-oxoéthyl]-3-imino-2,3-dihydro-imidazo[1,5-a]pyridin-6-carboxylique,
1-(3-tert-butyl-4-méthoxy-5-morpholin-4-ylphényl)-2-(3-imino-imidazo[1,5-a]pyridine-2-yl)-éthanone,
1-(3-tert-butyl-4-méthoxy-5-morpholin-4-ylphényl)-2-(3-imino-8-méthylimidazo[1,5-a]pyridin-2-yl)-éthanone,
1-(3-diméthylamino-5-pentafluorosulfanylphényl)-2-(3-imino-imidazo[1,5-a]pyridin-2-yl)-éthanone, méthylamide de l'acide 2-[2-(8-tert-butyl-4-méthyl-3,4-dihydro-2H-benzo[1,4]oxazin-6-yl)-2-oxoéthyl]-7-éthoxy-3-imino-2,3-dihydro-imidazo[1,5-a]pyridine-6-carboxylique,
méthylamide de l'acide 2-2-[(3-tert-butyl-4-méthoxy-5-morpholin-4-ylphényl)-2-oxoéthyl]-7-chloro-3-imino-2,3-dihydro-imidazo[1,5-a]pyridine-6-carboxylique,
2,2,2-trifluoro-N-{3-[2-(3-imino-imidazo[1,5-a]pyridin-2-yl)-acétyl]-5-pentafluorosulfanylphényl}-acétamide sous forme de sel trifluoroacétique,
1-(3-bromo-4-méthoxy-5-trifluorométhylphényl)-2-(3-imino-imidazo[1,5-a]pyridin-2-yl)-éthanone sous forme de sel trifluoroacétique,
2-(3-imino-imidazo[1,5-a]pyridin-2-yl)-1-(4-méthoxy-3-morpholin-4-yl-5-trifluorométhylphényl)-éthanone sous forme de sel trifluoroacétique,
6-éthoxy-3-imino-2-[2-(3-méthylamino-5-pentafluorosulfanylphényl)-2-oxoéthyl]-2,3-dihydro-1H-imidazo[1,5-a]-pyridin-5-one sous forme de sel trifluoroacétique,
2-[2-(3-tert-butyl-4-méthoxy-5-morpholin-4-ylphényl)-2-oxoéthyl]-6-éthoxy-3-imino-2,3-dihydro-1H-imidazo[1,5-a]pyridin-5-one sous forme de sel trifluoroacétique,
méthylamide de l'acide 2-[2-(3-tert-butyl-4-méthoxy-5-morpholin-4-ylphényl)-2-oxoéthyl]-7-éthoxy-3-imino-2,3-dihydro-imidazo[1,5-a]pyridine-6-carboxylique sous forme de sel trifluoroacétique ou
ester éthylique de l'acide 2-[2-(3-tert-butyl-4-méthoxy-5-morpholin-4-ylphényl)-2-oxoéthyl]-3-imino-5-méthoxy-2,3-dihydro-imidazo[1,5-a]pyridine-7-carboxylique sous forme de sel trifluoroacétique.

5. Médicament, **caractérisé par** une teneur active en au moins un composé de formule I selon l'une ou plusieurs des revendications 1 à 4 avec un support, un additif et/ou d'autres substances actives et adjuvants pharmaceutiquement approprié(s) et physiologiquement compatible(s).

6. Utilisation du composé de formule I selon l'une ou plusieurs des revendications 1 à 4 pour la préparation d'un médicament destiné à la prophylaxie, la prévention secondaire et la thérapie de toutes les maladies qui vont de pair avec les thromboses, les embolies, l'hypercoagulabilité ou les modifications fibrotiques.

7. Utilisation selon la revendication 6, **caractérisée en ce qu'**il s'agit de l'infarctus du myocarde, de l'angine de poitrine et d'autres formes du syndrome coronaire aigu, de l'attaque, de maladies vasculaires périphériques, de la thrombose veineuse profonde, de l'embolie pulmonaire, d'événements emboliques ou thrombotiques provoqués par les arythmies cardiaques, d'événements cardiovasculaires tels que la resténose après une revascularisation et une angioplastie et des interventions analogues telles que les implantations d'endoprothèses et les opérations de dérivation, ou de la réduction du risque de thrombose après des interventions chirurgicales, comme lors d'opérations des articulations du genou ou de la hanche ou des interventions qui conduisent à un contact du sang avec des surfaces étrangères comme chez les patients en dialyse et les patients avec des cathéters permanents ou de la coagulation intravasculaire disséminée, de la septicémie et d'autres événements intravasculaires, qui vont de pair avec une inflammation, de l'athérosclérose, du diabète et du syndrome métabolique et ses conséquences, de la croissance tumorale et des métastases tumorales, de maladies articulaires inflammatoires et de dégénérescence, telles que l'arthrite rhumatoïde et l'arthrose, de troubles du système hémostatique, tels que les dépôts de fibrine, de modifications fibrotiques des poumons, telles que la maladie pulmonaire obstructive chronique, le syndrome de détresse respiratoire adulte ou de dépôts de fibrine des yeux après des opérations des yeux ou de la prévention et/ou du traitement de la formation de cicatrices.

8. Procédé pour la préparation du composé de formule I selon l'une ou plusieurs des revendications 1 à 4, **caractérisé en ce qu'**on transforme
a) un composé de formule II 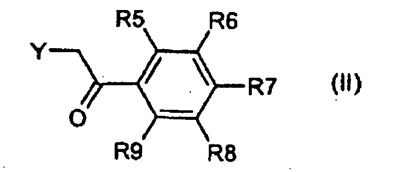 où les radicaux R5, R6, R7, R8 et R9 sont définis comme dans la formule I et Y représente chlorure, bromure, mésylate ou tosylate, avec un composé de formule III 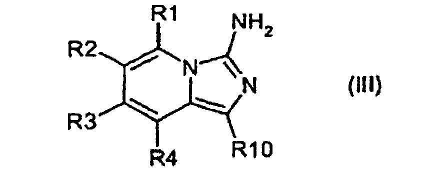 en présence d'une base et d'un solvant en un composé de formule I, ou
b) un composé de formule VII 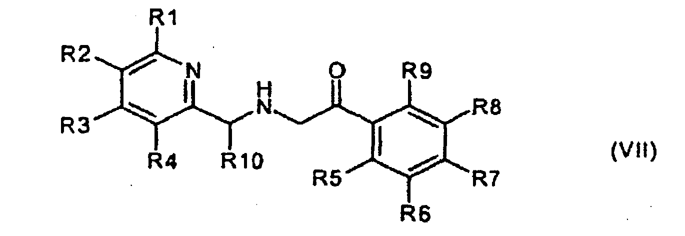 où les radicaux R1 à R10 sont définis comme dans la formule I avec un composé Z-CN, où Z signifie tosylate ou bromure, en présence d'une base en un composé de formule I, ou
c) on isole le composé de formule I, préparé selon les procédés a) ou b) sous forme libre ou on le libère à partir de sels physiologiquement non acceptables, ou, dans le cas de la présence de groupes acides ou basiques, on le convertit en sels physiologiquement acceptables, ou
d) on sépare un composé de formule I, préparé selon les procédés a) ou b), ou un précurseur approprié de formule I, qui, en raison de sa structure chimique, apparaît sous des formes énantiomères ou diastéréo-isomères, par salification avec des acides ou des bases énantiomères pur(e)s, chromatographie sur des phases stationnaires chirales ou transformation en dérivés au moyen de composés chiraux énantiomères purs, tels que des aminoacides, séparation des diastéréo-isomères ainsi obtenus et élimination des groupes auxiliaires chiraux, en énantiomères purs ou diastéréo-isomères.

9. Procédé pour la préparation du composé de formule VII selon la revendication 8, **caractérisé en ce qu'**on transforme un composé de formule VIII 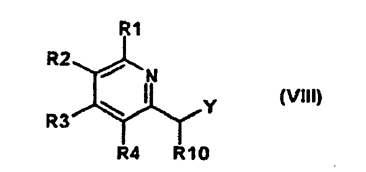 où les radicaux R1, R2, R3, R4 et R10 sont définis comme dans la formule I et Y représente chlorure, bromure, mésylate ou tosylate, avec un composé de formule IX 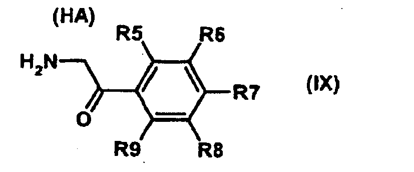 en présence d'une base et d'un solvant en un composé de formule I.

10. Procédé pour la préparation du composé de formule II selon la revendication 8, où Y signifie Br et un des radicaux R5, R6, R7, R8 ou R9 représente pentafluorosulfanyle, **caractérisé en ce qu'**on
a) transforme un composé de formule Xa 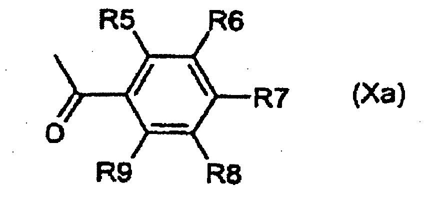 où un des radicaux R5, R6, R7, R8 ou R9 représente pentafluorosulfanyle et les autres radicaux R5, R6, R7, R8 et R9 sont définis comme dans la formule I avec un réactif de bromuration en composé de formule II, ou
b) traite un composé de formule XIa 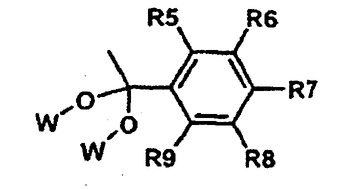
où un des radicaux R5, R6, R7, R8 ou R9 représente pentafluorosulfanyle et les autres radicaux R5, R6, R7, R8 et R9 sont définis comme dans la formule I, W représente éthylène, propylène ou butylène ou forme ensemble avec le groupe -O-C-O-un cycle 1,3-dioxo présentant une taille de cycle 5, 6 ou 7, avec un réactif de bromuration puis on le transforme en présence d'un acide en composé de formule II.

11. Procédé pour la préparation du composé de formule Xa selon la revendication 10, où R6 représente pentafluorosulfanyle et R8 représente diméthylamine et les radicaux R5, R7 et R9 sont définis comme dans la formule I, **caractérisé en ce qu'**on
a) nitre d'abord un composé de formule XIVa 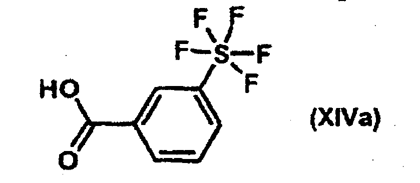 puis on le réduit avec le l'hydrogène en amine de formule XIIIa, 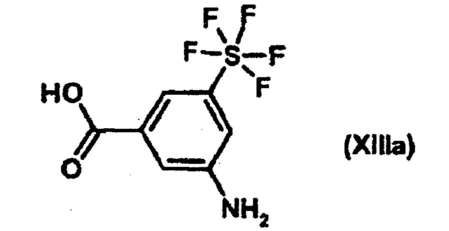
b) déméthyle le composé obtenu de formule XIIIa sur l'azote et on transforme l'acide carboxylique avec du chlorure de thionyle en chlorure d'acide, puis on le transforme avec de l'O,N-diméthylhydroxylamine en un composé de formule XIIa, et 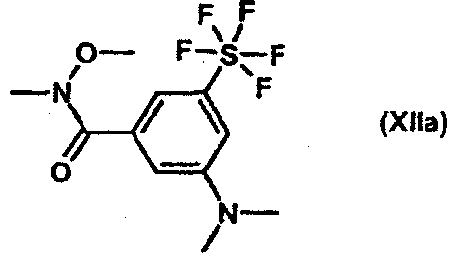
c) transforme le composé obtenu de formule XIIa avec du bromure de méthylmagnésium en composé de formule Xa.
